(19)

**Europäisches Patentamt**
**European Patent Office**
**Office européen des brevets**

(11)  **EP 3 919 126 A1**

(12)  **EUROPEAN PATENT APPLICATION**

(43) Date of publication:
**08.12.2021  Bulletin 2021/49**

(51) Int Cl.:
*A61P 31/14* (2006.01)     *C07K 16/10* (2006.01)

(21) Application number: **20210803.1**

(22) Date of filing: **30.11.2020**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **04.06.2020  EP 20178386**
**04.06.2020  EP 20178327**
**10.06.2020  EP 20179323**
**18.06.2020  EP 20180942**
**03.07.2020  EP 20184057**
**27.11.2020  EP 20210462**

(71) Applicant: **Corat Therapeutics GmbH**
**38124 Braunschweig (DE)**

(72) Inventors:
• **Frenzel, André**
**38104 Braunschweig (DE)**
• **Kuhn, Philipp**
**28355 Bremen (DE)**
• **Kügler, Jonas**
**38126 Braunschweig (DE)**
• **Hust, Michael**
**38118 Braunschweig (DE)**
• **Dübel, Stefan**
**38108 Braunschweig (DE)**
• **Meier, Doris**
**37444 St. Andreasberg (DE)**
• **Bertoglio, Federico**
**38106 Braunschweig (DE)**

• **Schubert, Maren**
**38104 Braunschweig (DE)**
• **Steinke, Stephan**
**38114 Braunschweig (DE)**
• **Becker, Marlies**
**38104 Braunschweig (DE)**
• **Führer, Viola**
**38104 Braunschweig (DE)**
• **Ruschig, Maximilian**
**38102 Braunschweig (DE)**
• **Heine, Philip Alexander**
**38118 Braunschweig (DE)**
• **Schneider, Kai-Thomas**
**38100 Braunschweig (DE)**
• **Ballmann, Rico**
**38102 Braunschweig (DE)**
• **Zock-Emmenthal, Susanne**
**38444 Wolfsburg (DE)**
• **Roth, Kristian Daniel Ralph**
**38112 Braunschweig (DE)**
• **Langreder, Nora**
**38114 Braunschweig (DE)**

(74) Representative: **Fuchs Patentanwälte**
**Partnerschaft mbB**
**Westhafenplatz 1**
**60327 Frankfurt am Main (DE)**

Remarks:
The complete document including Reference Tables
and the Sequence Listing can be downloaded from
the EPO website

(54)  **THERAPEUTIC ANTIBODIES WITH NEUTRALIZING ACTIVITY AGAINST SARS-COV-2**
**GLYCOPROTEIN S**

(57)  The present invention is related to antibodies
and antigen-binding fragments of antibodies that specifically bind the SARS-CoV-2 glycoprotein S (spike) as well
as diagnostic, preventive and therapeutic methods of us-
ing those antibodies.

**(Cont. next page)**

EP 3 919 126 A1

Figure 2:

## Description

Field of the Invention

The present invention is related to antibodies and antigen-binding fragments of antibodies that specifically bind the SARS-CoV-2 glycoprotein S (spike) as well as diagnostic and therapeutic methods of using those antibodies.

Background of the Invention

Coronavirus disease 2019 (COVID-19) is a highly infectious disease caused by severe acute respiratory syndrome coronavirus 2 (SARS-CoV-2).

Common symptoms of COVID-19 include fever, cough, fatigue, shortness of breath, and loss of smell and taste. While the majority of cases result in mild symptoms, some progress to acute respiratory distress syndrome (ARDS) characterized by a cytokine storm, multi-organ failure, septic shock, and blood clots. The time from exposure to onset of symptoms is typically around five days, but may range from two to fourteen days.

SARS-CoV-2 was previously referred to by its provisional name,"2019 *novel coronavirus"* (2019-nCoV). As described by the National Institutes of Health, it is the successor to "SARS-CoV-1" (or just "SARS-CoV"). It is believed to have zoonotic origins and has close genetic similarity to bat coronaviruses, suggesting it emerged from a bat-borne virus.

Taxonomically, SARS-CoV-2 belongs to the *coronaviridae,* positive-sense single-stranded RNA viruses. Coronaviruses are large, roughly spherical, particles with bulbous surface projections. The average diameter of the virus particles is around 125 nm (0.125 $\mu$m). The diameter of the envelope is 85 nm and the spikes are 20 nm long.

The viral envelope consists of a lipid bilayer, in which the membrane (M), envelope (E) and spike (S) structural proteins are anchored. The ratio of E:S:M in the lipid bilayer is approximately 1:20:300 (wt/wt). On average a coronavirus particle has 74 surface spikes.

The coronavirus surface spikes are homotrimers of the glycoprotein protein S, which is composed of an S1 and S2 subunit.

It is believed that, similar to SARS-CoV and MERS, the virus mainly enters human cells by binding specifically to the receptor angiotensin converting enzyme 2 (ACE2). It is believed that the S1-domain of the spike glycoprotein binds host's ACE2-receptor and the S2 domain mediates the viral fusion with the host cell membrane.

Although some similarities between SARS-CoV and SARS-CoV-2 exist, it has been found that SARS-CoV-2 S protein binds specifically ACE2 with significant higher affinity than SARS-CoV (10- to 20-fold). Furthermore, it was shown that antibody cross-reactivity is limited between the two virus S proteins, suggesting its recognition to ACE2 is different as compared to SARS-CoV.

Several published SARS-CoV Abs do not have appreciable binding specifically to SARS-CoV-2 S protein. A recent study (Tian et al., 2020) shows a SARS-CoV antibody, CR3022, which binds specifically to SARS-CoV-2 RBD (receptor binding domain), but to a highly conserved region outside of the ACE2-RBD-interaction site and with weak or absent neutralization capability.

As of today, globally more than 5.600.000 people are reported to be infected with SARS-CoV-2, and about 350.000 people died because of COVID-19.

Neutralizing antibodies of therapeutic quality, which prevent the virus from entering the host cell would be of vital importance as primary treatment, especially in the case of COVID-19 where no medication or vaccination has been identified.

Thus, a great need exists to develop neutralizing antibodies, which prevent the infection with SARS-CoV-2 in a quality suitable for therapeutic use.

Furthermore, it has been reported that SARS-CoV-2 shows "escape"-mutations. These are mutations especially in the RBD-region, which still enable the virus to bind to cellular ACE-2 protein via its spike-protein, but seem to allow the evasion of host's immune response.

This finding is of great importance for estimating the chances of a re-infection with SARS-CoV-2 after having successfully overcome a first SARS-CoV-2-infection, as well as having an impact on the development of vaccines and the treatment with antiviral agents, such as monoclonal antibodies.

Prior art attempts to overcome the danger of the virus building a resistance against certain treatments include the use of polyclonal antibodies or cocktails of two or more monoclonal antibodies, which bind to different epitopes of the target protein.

However, these strategies comprise other negative consequences. For example, polyclonal antibodies do show a wide range of specificity and, consequently, are difficult to predict in their efficacy. Therapeutics based on polyclonal antibodies are therefore difficult to dose and to be produced with constant quality.

Cocktails of two or more monoclonal antibodies immediately double the costs of production and, since the efficacy and pharmacokinetic properties of each antibody differ, are difficult to formulate in order to maintain reliable administration schemes.

The present invention, however, provides monoclonal antibodies which show an advantageous binding profile to the S1-protein of SARS-CoV-2 allowing the binding and neutralization of SARS-CoV-2-activity of seven different "escape"-mutants reported up to date, as well as also the binding specifically to the "7-PM-mutant", which comprises all seven known "escape"-mutations in one molecule.

Detailed Description

In a first embodiment, the present invention pertains to a therapeutic antibody or antigen-binding fragment thereof that binds specifically to SARS-CoV-2 glycoprotein S with the amino acid sequence of SEQ ID NO: 02 and neutralizes the infectious activity of SARS-CoV-2.

In a second embodiment, the present invention pertains to a therapeutic antibody or antigen-binding fragment thereof, wherein said antibody or antigen-binding fragment thereof competes for binding to the SARS-CoV-2 glycoprotein S with an antibody comprising a heavy chain variable region of the amino acid sequence in SEQ ID NO: 406; and a light chain variable region of the amino acid sequence in SEQ ID NO: 408.

In another embodiment, the present invention pertains to a therapeutic antibody or antigen-binding fragment thereof, wherein said antibody or antigen-binding fragment thereof competes for binding to the SARS-CoV-2 glycoprotein S with an antibody comprising at least one, two, three or all of the amino acid sequences of SEQ ID NO: 909 - 928 (YU505-A02); SEQ ID NO: 349 - 368 (YU536-D04); SEQ ID NOs: 389 - 408 (YU537-H11) and/or SEQ ID NO: 1305 - 1312 (STE90-C11).

In a fourth embodiment, the present invention pertains to a therapeutic antibody or antigen-binding fragment thereof, comprising a variable heavy chain with an amino acid sequence selected from the group consisting of SEQ ID NO: 26, 46, 66, 86, 106, 126, 146, 166, 186, 206, 226, 246, 266, 286, 306, 326, 346, 366, 386, 406, 426, 446, 466, 486, 506, 526, 546, 566, 586, 606, 626, 646, 666, 686, 706, 726, 746, 766, 786, 806, 826, 846, 866, 886, 906, 926, 946, 966, 986, 1006, 1026, 1046, 1066, 1086, 1106, 1126, 1146, 1166, 1186, 1206, 1226, 1246, 1266, and 1286; and a light chain with an amino acid sequence selected from the group consisting of SEQ ID NO: 28, 48, 68, 88, 108, 128, 148, 168, 188, 208, 228, 248, 268, 288, 308, 328, 348, 368, 388, 408, 428, 448, 468, 488, 508, 528, 548, 568, 588, 608, 628, 648, 668, 688, 708, 728, 748, 768, 788, 808, 828, 848, 868, 888, 908, 928, 948, 968, 988, 1008, 1028, 1048, 1068, 1088, 1108, 1128, 1148, 1168, 1188, 1208, 1228, 1248, 1268, and 288.

In another embodiment, the present invention pertains to a therapeutic antibody or antigen-binding fragment thereof, comprising a variable heavy chain with an amino acid sequence selected from the group consisting of SEQ ID NO: 1289, 1297, 1305, 1313, 1321, 1329, 1337, 1345, 1353, 1361, 1369, 1377, 1385, 1393, 1401, 1409, 1417, 1425, 1433, 1441, 1449, 1457, 1465, 1473, 1481, 1489, 1497, 1505, 1513, 1521, 1529, 1537, 1545, 1553, 1561, 1569, 1577, 1585, 1593, 1601, 1609, 1617, 1625, 1633, 1641, 1649, 1657, 1665, 1673, 1681, 1689, 1697, 1705, 1713, and 1721; and a light chain with an amino acid sequence selected from the group consisting of SEQ ID NO: 1293, 1301, 1309, 1317, 1325, 1333, 1341, 1349, 1357, 1365, 1373, 1381, 1389, 1397, 1405, 1413, 1421, 1429, 1437, 1445, 1453, 1461, 1469, 1477, 1485, 1493, 1501, 1509, 1517, 1525, 1533, 1541, 1549, 1557, 1565, 1573, 1581, 1589, 1597, 1605, 1613, 1621, 1629, 1637, 1645, 1653, 1661, 1669, 1677, 1685, 1693, 1701, 1709, 1717, and 1725.

Another aspect of the invention relates to a monoclonal antibody or an antigen-binding fragment thereof characterized by a VH region and optionally a VL region each comprising 3 CDRs designated as H-CDR1, H-CDR2, H-CDR3, L-CDR1, L-CDR2 and L-CDR3 defining the binding specificity of the antibody or the antigen-binding antibody fragment.

The position of CDRs within a VH or VL region can be defined according to Kabat, et al., Sequences of Proteins of Immunological Interest, 5th Ed. Public Health Service, National Institutes of Health, Bethesda, MD (1991). In another embodiment, the method IMGT may be used to define the CDRs (Brochet, X. et al., Nucl. Acids Res. 36, W503-508 (2008). PMID: 18503082. Giu-dicelli, V., Brochet, X., Lefranc, M.-P., Cold Spring Harb Protoc. 2011 Jun 1; 2011 (6). pii: pdb.prot5633. doi: 10.1101/pdb.prot5633).

The CDR-sequences defined according to Kabat et al. are depicted in SEQ ID Nos: 10, 30, 50, 70, 90, 110, 130, 150, 170, 190, 210, 230, 250, 270, 290, 310, 330, 350, 370, 390, 410, 430, 450, 470, 490, 510, 530, 550, 570, 590, 610, 630, 650, 670, 690, 710, 730, 750, 770, 790, 810, 830, 850, 870, 890, 910, 930, 950, 970, 990, 1110, 1130, 1150, 1170, 1190, 1210, 1230, 1250, 1270; 12, 32, 52, 72, 92, 112, 132, 152, 172, 192, 212, 232, 252, 272, 292, 312, 332, 352, 372, 392, 412, 432, 452, 472, 492, 512, 532, 552, 572, 592, 612, 632, 652, 672, 692, 712, 732, 752, 772, 792, 812, 832, 852, 872, 892, 912, 932, 952, 972, 992, 1112, 1132, 1152, 1172, 1192, 1212, 1232, 1252, 1272; 14, 34, 54, 74, 94, 114, 134, 154, 174, 194, 214, 234, 254, 274, 294, 314, 334, 354, 374, 394, 414, 434, 454, 474, 494, 514, 534, 554, 574, 594, 614, 634, 654, 674, 694, 714, 734, 754, 774, 794, 814, 834, 854, 874, 894, 914, 934, 954, 974, 994, 1114, 1134, 1154, 1174, 1194, 1214, 1234, 1254, 1274;

16, 36, 56, 76, 96, 116, 136, 156, 176, 196, 216, 236, 256, 276, 296, 316, 336, 356, 376, 396, 416, 436, 456, 476, 496, 516, 536, 556, 576, 596, 616, 636, 656, 676, 696, 716, 736, 756, 776, 796, 816, 836, 856, 876, 896, 916, 936, 956, 976, 996, 1116, 1136, 1156, 1176, 1196, 1216, 1236, 1256, 1276;,

18, 38, 58, 78, 98, 118, 138, 158, 178, 198, 218, 238, 258, 278, 298, 318, 338, 358, 378, 398, 418, 438, 458, 478, 498, 518, 538, 558, 578, 598, 618, 638, 658, 678, 698, 718, 738, 758, 778, 798, 818, 838, 858, 878, 898, 918, 938, 958, 978, 998, 1118, 1138, 1158, 1178, 1198, 1218, 1238, 1258, 1278;

20, 40, 60, 80, 100, 120, 140, 160, 180, 200, 220, 240, 260, 280, 300, 320, 340, 360, 380, 400, 420, 440, 460, 480, 500, 520, 540, 560, 580, 600, 620, 640, 660, 680, 700, 720, 740, 760, 780, 800, 820, 840, 860, 880, 900, 920, 940, 960, 980, 1000, 1120, 1140, 1160, 1180, 1200, 1220, 1240, 1260, and 1280.

The CDR-sequences defined according to IMGT are depicted in SEQ ID Nos: 1290, 1298, 1306, 1314, 1322, 1330, 1338, 1346, 1354, 1362, 1370, 1378, 1386, 1394, 1402, 1410, 1418, 1426, 1434, 1442, 1450, 1458, 1466, 1474, 1482, 1490, 1498, 1506, 1514, 1522, 1530, 1538, 1546, 1554, 1562, 1570, 1578, 1586, 1594, 1602, 1610, 1618, 1626, 1634, 1642, 1650, 1658, 1666, 1674, 1682, 1690, 1698, 1706, 1714, 1722;

1291, 1299, 1307, 1315, 1323, 1331, 1339, 1347, 1355, 1363, 1371, 1379, 1387, 1395, 1403, 1411, 1419, 1427, 1435, 1443, 1451, 1459, 1467, 1475, 1483, 1491, 1499, 1507, 1515, 1523, 1531, 1539, 1547, 1555, 1563, 1571, 1579, 1587, 1595, 1603, 1611, 1619, 1627, 1635, 1643, 1651, 1659, 1667, 1675, 1683, 1691, 1699, 1707, 1715, 1723;

1292, 1300, 1308, 1316, 1324, 1332, 1340, 1348, 1356, 1364, 1372, 1380, 1388, 1396, 1404, 1412, 1420, 1428, 1436, 1444, 1452, 1460, 1468, 1476, 1484, 1492, 1500, 1508, 1516, 1524, 1532, 1540, 1548, 1556, 1564, 1572, 1580, 1588, 1596, 1604, 1612, 1620, 1628, 1636, 1644, 1652, 1660, 1668, 1676, 1684, 1692, 1700, 1708, 1716, 1724;

1294, 1302, 1310, 1318, 1326, 1334, 1342, 1350, 1358, 1366, 1374, 1382, 1390, 1398, 1406, 1414, 1422, 1430, 1438, 1446, 1454, 1462, 1470, 1478, 1486, 1494, 1502, 1510, 1518, 1526, 1534, 1542, 1550, 1558, 1566, 1574, 1582, 1590, 1598, 1606, 1614, 1622, 1630, 1638, 1646, 1654, 1662, 1670, 1678, 1686, 1694, 1702, 1710, 1718, 1726;

1295, 1303, 1311, 1319, 1327, 1335, 1343, 1351, 1359, 1367, 1375, 1383, 1391, 1399, 1407, 1415, 1423, 1431, 1439, 1447, 1455, 1463, 1471, 1479, 1487, 1495, 1503, 1511, 1519, 1527, 1535, 1543, 1551, 1559, 1567, 1575, 1583, 1591, 1599, 1607, 1615, 1623, 1631, 1639, 1647, 1655, 1663, 1671, 1679, 1687, 1695, 1703, 1711, 1719, 1727;

1296, 1304, 1312, 1320, 1328, 1336, 1344, 1352, 1360, 1368, 1376, 1384, 1392, 1400, 1408, 1416, 1424, 1432, 1440, 1448, 1456, 1464, 1472, 1480, 1488, 1496, 1504, 1512, 1520, 1528, 1536, 1544, 1552, 1560, 1568, 1576, 1584, 1592, 1600, 1608, 1616, 1624, 1632, 1640, 1648, 1656, 1664, 1672, 1680, 1688, 1696, 1704, 1712, 1720, and 1728.

According to the present invention, a VH region or the CDRs thereof alone may constitute a complete antigen-binding site. In certain embodiments, the antibody comprises a VH region or the CDRs thereof as defined herein alone. In certain embodiments, the antibody comprises a VH region or the CDRs thereof as defined herein together with a VL region or the CDRs thereof, particularly with a VL region or the CDRs thereof as defined herein. Thus, the present invention pertains in some embodiments to an antibody or antigen-binding fragment thereof, comprising:

a heavy chain CDR1 (H-CDR1) domain with an amino acid sequence selected from the group consisting of SEQ ID NO: 10, 30, 50, 70, 90, 110, 130, 150, 170, 190, 210, 230, 250, 270, 290, 310, 330, 350, 370, 390, 410, 430, 450, 470, 490, 510, 530, 550, 570, 590, 610, 630, 650, 670, 690, 710, 730, 750, 770, 790, 810, 830, 850, 870, 890, 910, 930, 950, 970, 990, 1110, 1130, 1150, 1170, 1190, 1210, 1230, 1250, and 1270;

a heavy chain CDR2 (H-CDR2) domain with an amino acid sequence selected from the group consisting of SEQ ID NO: 12, 32, 52, 72, 92, 112, 132, 152, 172, 192, 212, 232, 252, 272, 292, 312, 332, 352, 372, 392, 412, 432, 452, 472, 492, 512, 532, 552, 572, 592, 612, 632, 652, 672, 692, 712, 732, 752, 772, 792, 812, 832, 852, 872, 892, 912, 932, 952, 972, 992, 1112, 1132, 1152, 1172, 1192, 1212, 1232, 1252, and 1272;

a heavy chain CDR3 (H-CDR3) domain with an amino acid sequence selected from the group consisting of SEQ ID NO: 14, 34, 54, 74, 94, 114, 134, 154, 174, 194, 214, 234, 254, 274, 294, 314, 334, 354, 374, 394, 414, 434, 454, 474, 494, 514, 534, 554, 574, 594, 614, 634, 654, 674, 694, 714, 734, 754, 774, 794, 814, 834, 854, 874, 894, 914, 934, 954, 974, 994, 1114, 1134, 1154, 1174, 1194, 1214, 1234, 1254, and 1274; and

a light chain CDR1 (L-CDR1) domain with an amino acid sequence selected from the group consisting of SEQ ID NO: 16, 36, 56, 76, 96, 116, 136, 156, 176, 196, 216, 236, 256, 276, 296, 316, 336, 356, 376, 396, 416, 436, 456, 476, 496, 516, 536, 556, 576, 596, 616, 636, 656, 676, 696, 716, 736, 756, 776, 796, 816, 836, 856, 876, 896, 916, 936, 956, 976, 996, 1116, 1136, 1156, 1176, 1196, 1216, 1236, 1256, and 1276;

a light chain CDR2 (L-CDR2) domain with an amino acid sequence selected from the group consisting of SEQ ID NO: 18, 38, 58, 78, 98, 118, 138, 158, 178, 198, 218, 238, 258, 278, 298, 318, 338, 358, 378, 398, 418, 438, 458, 478, 498, 518, 538, 558, 578, 598, 618, 638, 658, 678, 698, 718, 738, 758, 778, 798, 818, 838, 858, 878, 898, 918, 938, 958, 978, 998, 1118, 1138, 1158, 1178, 1198, 1218, 1238, 1258, and 1278;

a light chain CDR3 (L-CDR3) domain with an amino acid sequence selected from the group consisting of SEQ ID NO: 20, 40, 60, 80, 100, 120, 140, 160, 180, 200, 220, 240, 260, 280, 300, 320, 340, 360, 380, 400, 420, 440, 460, 480, 500, 520, 540, 560, 580, 600, 620, 640, 660, 680, 700, 720, 740, 760, 780, 800, 820, 840, 860, 880, 900, 920, 940, 960, 980, 1000, 1120, 1140, 1160, 1180, 1200, 1220, 1240, 1260, and 1280.

In another embodiment, the present invention pertains to an antibody or antigen-binding fragment thereof, comprising:

a heavy chain CDR1 (H-CDR1) domain with an amino acid sequence selected from the group consisting of SEQ ID NO: 1290, 1298, 1306, 1314, 1322, 1330, 1338, 1346, 1354, 1362, 1370, 1378, 1386, 1394, 1402, 1410, 1418, 1426, 1434, 1442, 1450, 1458, 1466, 1474, 1482, 1490, 1498, 1506, 1514, 1522, 1530, 1538, 1546, 1554, 1562, 1570, 1578, 1586, 1594, 1602, 1610, 1618, 1626, 1634, 1642, 1650, 1658, 1666, 1674, 1682, 1690, 1698, 1706, 1714, and 1722;

a heavy chain CDR2 (H-CDR2) domain with an amino acid sequence selected from the group consisting of SEQ ID NO: 1291, 1299, 1307, 1315, 1323, 1331, 1339, 1347, 1355, 1363, 1371, 1379, 1387, 1395, 1403, 1411, 1419, 1427, 1435, 1443, 1451, 1459, 1467, 1475, 1483, 1491, 1499, 1507, 1515, 1523, 1531, 1539, 1547, 1555, 1563, 1571, 1579, 1587, 1595, 1603, 1611, 1619, 1627, 1635, 1643, 1651, 1659, 1667, 1675, 1683, 1691, 1699, 1707, 1715, and 1723;

a heavy chain CDR3 (H-CDR3) domain with an amino acid sequence selected from the group consisting of SEQ ID NO: 1292, 1300, 1308, 1316, 1324, 1332, 1340, 1348, 1356, 1364, 1372, 1380, 1388, 1396, 1404, 1412, 1420, 1428, 1436, 1444, 1452, 1460, 1468, 1476, 1484, 1492, 1500, 1508, 1516, 1524, 1532, 1540, 1548, 1556, 1564, 1572, 1580, 1588, 1596, 1604, 1612, 1620, 1628, 1636, 1644, 1652, 1660, 1668, 1676, 1684, 1692, 1700, 1708, 1716, and 1724;
and

a light chain CDR1 (L-CDR1) domain with an amino acid sequence selected from the group consisting of SEQ ID NO: 1294, 1302, 1310, 1318, 1326, 1334, 1342, 1350, 1358, 1366, 1374, 1382, 1390, 1398, 1406, 1414, 1422, 1430, 1438, 1446, 1454, 1462, 1470, 1478, 1486, 1494, 1502, 1510, 1518, 1526, 1534, 1542, 1550, 1558, 1566, 1574, 1582, 1590, 1598, 1606, 1614, 1622, 1630, 1638, 1646, 1654, 1662, 1670, 1678, 1686, 1694, 1702, 1710, 1718, and 1726;

a light chain CDR2 (L-CDR2) domain with an amino acid sequence selected from the group consisting of SEQ ID NO: 1295, 1303, 1311, 1319, 1327, 1335, 1343, 1351, 1359, 1367, 1375, 1383, 1391, 1399, 1407, 1415, 1423, 1431, 1439, 1447, 1455, 1463, 1471, 1479, 1487, 1495, 1503, 1511, 1519, 1527, 1535, 1543, 1551, 1559, 1567, 1575, 1583, 1591, 1599, 1607, 1615, 1623, 1631, 1639, 1647, 1655, 1663, 1671, 1679, 1687, 1695, 1703, 1711, 1719, and 1727;

a light chain CDR3 (L-CDR3) domain with an amino acid sequence selected from the group consisting of SEQ ID NO: 1296, 1304, 1312, 1320, 1328, 1336, 1344, 1352, 1360, 1368, 1376, 1384, 1392, 1400, 1408, 1416, 1424, 1432, 1440, 1448, 1456, 1464, 1472, 1480, 1488, 1496, 1504, 1512, 1520, 1528, 1536, 1544, 1552, 1560, 1568, 1576, 1584, 1592, 1600, 1608, 1616, 1624, 1632, 1640, 1648, 1656, 1664, 1672, 1680, 1688, 1696, 1704, 1712, 1720, and 1728.

In certain embodiments, the antibody or antigen-binding fragment thereof comprises a VH region comprising the H-CDR1 of SEQ ID NO:10, the H-CDR2 of SEQ ID NO:12, and the H-CDR3 of SEQ ID NO:14, and optionally a VL region comprising the L-CDR1 of SEQ ID NO:16, the L-CDR2 of the SEQ ID NO:18 and the L-CDR3 of the SEQ ID NO:20; and so forth as depicted in table 1:

Table 1: Composition of certain antibody or antigen-binding fragments thereof

| VH-region | | | and, optionally, a VL-region | | |
|---|---|---|---|---|---|
| H-CDR1, SEQ ID NO.: | H-CDR2, SEQ ID NO.: | H-CDR3, SEQ ID NO.: | L-CDR1, SEQ ID NO.: | L-CDR2, SEQ ID NO.: | L-CDR3, SEQ ID NO.: |
| 10 | 12 | 14 | 16 | 18 | 20 |
| 30 | 32 | 34 | 36 | 38 | 40 |
| 50 | 52 | 54 | 56 | 58 | 60 |
| 70 | 72 | 74 | 76 | 78 | 80 |
| 90 | 92 | 94 | 96 | 98 | 100 |
| 110 | 112 | 114 | 116 | 118 | 120 |
| 130 | 132 | 134 | 136 | 138 | 140 |
| 150 | 152 | 154 | 156 | 158 | 160 |
| 170 | 172 | 174 | 176 | 178 | 180 |
| 190 | 192 | 194 | 196 | 198 | 200 |
| 210 | 212 | 214 | 216 | 218 | 220 |
| 230 | 232 | 234 | 236 | 238 | 240 |
| 250 | 252 | 254 | 256 | 258 | 260 |
| 270 | 272 | 274 | 276 | 278 | 280 |
| 290 | 292 | 294 | 296 | 298 | 300 |
| 310 | 312 | 314 | 316 | 318 | 320 |
| 330 | 332 | 334 | 336 | 338 | 340 |
| 350 | 352 | 354 | 356 | 358 | 360 |
| 370 | 372 | 374 | 376 | 378 | 380 |
| 390 | 392 | 394 | 396 | 398 | 400 |
| 410 | 412 | 414 | 416 | 418 | 420 |
| 430 | 432 | 434 | 436 | 438 | 440 |
| 450 | 452 | 454 | 456 | 458 | 460 |
| 470 | 472 | 474 | 476 | 478 | 480 |
| 490 | 492 | 494 | 496 | 498 | 500 |
| 510 | 512 | 514 | 516 | 518 | 520 |
| 530 | 532 | 534 | 536 | 538 | 540 |
| 550 | 552 | 554 | 556 | 558 | 560 |
| 570 | 572 | 574 | 576 | 578 | 580 |
| 590 | 592 | 594 | 596 | 598 | 600 |
| 610 | 612 | 614 | 616 | 618 | 620 |
| 630 | 632 | 634 | 636 | 638 | 640 |
| 650 | 652 | 654 | 656 | 658 | 660 |
| 670 | 672 | 674 | 676 | 678 | 680 |
| 690 | 692 | 694 | 696 | 698 | 700 |
| 710 | 712 | 714 | 716 | 718 | 720 |

(continued)

| VH-region | | | and, optionally, a VL-region | | |
|---|---|---|---|---|---|
| H-CDR1, SEQ ID NO.: | H-CDR2, SEQ ID NO.: | H-CDR3, SEQ ID NO.: | L-CDR1, SEQ ID NO.: | L-CDR2, SEQ ID NO.: | L-CDR3, SEQ ID NO.: |
| 730 | 732 | 734 | 736 | 738 | 740 |
| 750 | 752 | 754 | 756 | 758 | 760 |
| 770 | 772 | 774 | 776 | 778 | 780 |
| 790 | 792 | 794 | 796 | 798 | 800 |
| 810 | 812 | 814 | 816 | 818 | 820 |
| 830 | 832 | 834 | 836 | 838 | 840 |
| 850 | 852 | 854 | 856 | 858 | 860 |
| 870 | 872 | 874 | 876 | 878 | 880 |
| 890 | 892 | 894 | 896 | 898 | 900 |
| 910 | 912 | 914 | 916 | 918 | 920 |
| 930 | 932 | 934 | 936 | 938 | 940 |
| 950 | 952 | 954 | 956 | 958 | 960 |
| 970 | 972 | 974 | 976 | 978 | 980 |
| 990 | 992 | 994 | 996 | 998 | 1000 |
| 1010 | 1012 | 1014 | 1016 | 1018 | 1020 |
| 1030 | 1032 | 1034 | 1036 | 1038 | 1040 |
| 1050 | 1052 | 1054 | 1056 | 1058 | 1060 |
| 1070 | 1072 | 1074 | 1076 | 1078 | 1080 |
| 1090 | 1092 | 1094 | 1096 | 1098 | 1100 |
| 1110 | 1112 | 1114 | 1116 | 1118 | 1120 |
| 1130 | 1132 | 1134 | 1136 | 1138 | 1140 |
| 1150 | 1152 | 1154 | 1156 | 1158 | 1160 |
| 1170 | 1172 | 1174 | 1176 | 1178 | 1180 |
| 1190 | 1192 | 1194 | 1196 | 1198 | 1200 |
| 1210 | 1212 | 1214 | 1216 | 1218 | 1220 |
| 1230 | 1232 | 1234 | 1236 | 1238 | 1240 |
| 1250 | 1252 | 1254 | 1256 | 1258 | 1260 |
| 1270 | 1272 | 1274 | 1276 | 1278 | 1280 |
| 1290 | 1291 | 1292 | 1294 | 1295 | 1296 |
| 1298 | 1299 | 1300 | 1302 | 1303 | 1304 |
| 1306 | 1307 | 1308 | 1310 | 1311 | 1312 |
| 1314 | 1315 | 1316 | 1318 | 1319 | 1320 |
| 1322 | 1323 | 1324 | 1326 | 1327 | 1328 |
| 1330 | 1331 | 1332 | 1334 | 1335 | 1336 |
| 1338 | 1339 | 1340 | 1342 | 1343 | 1344 |
| 1346 | 1347 | 1348 | 1350 | 1351 | 1352 |

(continued)

| VH-region | | | and, optionally, a VL-region | | |
|---|---|---|---|---|---|
| H-CDR1, SEQ ID NO.: | H-CDR2, SEQ ID NO.: | H-CDR3, SEQ ID NO.: | L-CDR1, SEQ ID NO.: | L-CDR2, SEQ ID NO.: | L-CDR3, SEQ ID NO.: |
| 1354 | 1355 | 1356 | 1358 | 1359 | 1360 |
| 1362 | 1363 | 1364 | 1366 | 1367 | 1368 |
| 1370 | 1371 | 1372 | 1374 | 1375 | 1376 |
| 1378 | 1379 | 1380 | 1382 | 1383 | 1384 |
| 1386 | 1387 | 1388 | 1390 | 1391 | 1392 |
| 1394 | 1395 | 1396 | 1398 | 1399 | 1400 |
| 1402 | 1403 | 1404 | 1406 | 1407 | 1408 |
| 1410 | 1411 | 1412 | 1414 | 1415 | 1416 |
| 1418 | 1419 | 1420 | 1422 | 1423 | 1424 |
| 1426 | 1427 | 1428 | 1430 | 1431 | 1432 |
| 1434 | 1435 | 1436 | 1438 | 1439 | 1440 |
| 1442 | 1443 | 1444 | 1446 | 1447 | 1448 |
| 1450 | 1451 | 1452 | 1454 | 1455 | 1456 |
| 1458 | 1459 | 1460 | 1462 | 1463 | 1464 |
| 1466 | 1467 | 1468 | 1470 | 1471 | 1472 |
| 1474 | 1475 | 1476 | 1478 | 1479 | 1480 |
| 1482 | 1483 | 1484 | 1486 | 1487 | 1488 |
| 1490 | 1491 | 1492 | 1494 | 1495 | 1496 |
| 1498 | 1499 | 1500 | 1502 | 1503 | 1504 |
| 1506 | 1507 | 1508 | 1510 | 1511 | 1512 |
| 1514 | 1515 | 1516 | 1518 | 1519 | 1520 |
| 1522 | 1523 | 1524 | 1526 | 1527 | 1528 |
| 1530 | 1531 | 1532 | 1534 | 1535 | 1536 |
| 1538 | 1539 | 1540 | 1542 | 1543 | 1544 |
| 1546 | 1547 | 1548 | 1550 | 1551 | 1552 |
| 1554 | 1555 | 1556 | 1558 | 1559 | 1560 |
| 1562 | 1563 | 1564 | 1566 | 1567 | 1568 |
| 1570 | 1571 | 1572 | 1574 | 1575 | 1576 |
| 1578 | 1579 | 1580 | 1582 | 1583 | 1584 |
| 1586 | 1587 | 1588 | 1590 | 1591 | 1592 |
| 1594 | 1595 | 1596 | 1598 | 1599 | 1600 |
| 1602 | 1603 | 1604 | 1606 | 1607 | 1608 |
| 1610 | 1611 | 1612 | 1614 | 1615 | 1616 |
| 1618 | 1619 | 1620 | 1622 | 1623 | 1624 |
| 1626 | 1627 | 1628 | 1630 | 1631 | 1632 |
| 1634 | 1635 | 1636 | 1638 | 1639 | 1640 |

(continued)

| VH-region | | | and, optionally, a VL-region | | |
|---|---|---|---|---|---|
| H-CDR1, SEQ ID NO.: | H-CDR2, SEQ ID NO.: | H-CDR3, SEQ ID NO.: | L-CDR1, SEQ ID NO.: | L-CDR2, SEQ ID NO.: | L-CDR3, SEQ ID NO.: |
| 1642 | 1643 | 1644 | 1646 | 1647 | 1648 |
| 1650 | 1651 | 1652 | 1654 | 1655 | 1656 |
| 1658 | 1659 | 1660 | 1662 | 1663 | 1664 |
| 1666 | 1667 | 1668 | 1670 | 1671 | 1672 |
| 1674 | 1675 | 1676 | 1678 | 1679 | 1680 |
| 1682 | 1683 | 1684 | 1686 | 1687 | 1688 |
| 1690 | 1691 | 1692 | 1694 | 1695 | 1696 |
| 1698 | 1699 | 1700 | 1702 | 1703 | 1704 |
| 1706 | 1707 | 1708 | 1710 | 1711 | 1712 |
| 1714 | 1715 | 1716 | 1718 | 1719 | 1720 |
| 1722 | 1723 | 1724 | 1726 | 1727 | 1728 |

The terms "SARS-CoV-2 glycoprotein S", "S protein", "homotrimeric S protein", "spike protein" or "SARS-CoV-2 spike glycoprotein" and "SARS-CoV-2 S protein", as used herein, all refer to the same protein having the nucleic acid sequence shown in SEQ ID NO: 01 and the amino acid sequence of SEQ ID NO: 02, or a substantially similar variant thereof, or a biologically active fragment thereof.

SARS-CoV-2 can be divided into different strains. According to their genotype and distribution the strains may be divided into the following subgroups: The "L-Lineage" comprises WH 2019/12/30.h, Thailand 2020/01/13.a, Japan 2020/01/25.a, TW 2020/02/05.a, WH 2020/01/02.a, WW2020/01/02.b, USA-2020/01/27.a, FS 2020/01/22.c, FS-2020/01/22.b, USA 2020/01/31.a, GZ_2020/01/22.a, Japan_2020/01/29.b, WH 2020/01/01.c, GO-2020/01/17.a, GD-2020/01/22.a, GD-020/01/18.a, USA_2020/01/29.a, WH 2019/12/30.i, Germany_2020/01/28.a, WH 2020/01/07.a, Nepal 2020/01/13.a, Thailand 2020/01/08.a, HZ 2020701/20.a, Sydney 2020/01/22.a, ZJ 202CY/01/16.a, WH 2019/12/30.1, Singapore 2020/01/25.a, Singapore 2020/01/23.a, GD 2020/01/23.a, France 2020/01/29.a, WH 2019/12/30.f, HZ 2020/01/19.a, WH 2019/12/30.g, WH 2019/12/30.k, USA 2020/01/29.b, FS 2020/01/22.a, USA 2020/01/29.c, USA-2020/01/29.d, SZ 2020/01/16.a, USA_2020/01/21.a, GD 2020/0115.b, SZ_2020/01/16, WH 2019/12/30.n, WH 2019/12/30.c, JS 2020/01TI9.a, WH_2019/12/26.a, ZJ 2020/01/17.a, WI, 2020/01/01.b, JX 2020/01/11.a, WH 2020/01/01.e, WH 2019/12/30.m, WH 2019/12/30.b, WH 2019/12/30.e, CQ 2020/01/23.a, WH_2020/01/01.f, WH 2020/01/01.a, WW2020/01/01.d, WH 2019/12730.a, WH 2019/12/30.d, WH 2019/12/24.a, WH 2019/12/30.j, France 2020/01/23.a, France 2020/01/23.b, TW 2020/01/23.a, Sydney 2020/01/25.a, USA 2020/01/22.a, Australia_2020/01/25.a, Skorea_2020/01.a, WH_2019/12/31.a, France 2020/01/29.b, Singapore 2020/02/01.a, CQ_2020/01/18.a, and SD_2020/01/19.a.

The "S-Lineage" comprises SZ 2020/01/13.a, GD_2020/01/15.c, SZ_2020/01/13.a, GD-2020/01/15.a, GD 2020/01/14.a, SZ 2020/01/10.a, Japan 2020/01/31.b, Japan 2020/01/31.a, Japan 2020/01/29.a, SZ 2020/01/13.b, SZ_2020/01/11.a, USA 2020/01/28.a, SC 2020/01/15.a, USA 2020/01/23.a, Vietnam_2020/01/24.a, Korea 2020/01/25.a, WH 2020/01/05.a, Australia 2020/01/24.a, Belgium 2020/02/03.a, TW 2020/01731.a, Australia 2020/01/28.a, Australia 2020/01 /30.a, England 2020/01/29.a, England 2020/01/29.b, USA 2020/01/25.a, USA-2020/01/19.a, USA ~020/01 /25.b, CQ 2020/01/21.a, USA 2020/01/22.b, YN_2020/01/17.b, and YN_2020/01/17.a.

In one embodiment the therapeutic antibody or antigen-binding fragment thereof is preferred which binds specifically to at least one of the SARS-CoV-2 glycoprotein S from the above mentioned strains. In yet another embodiment it binds specifically more preferably to the SARS-CoV-2 glycoprotein S of one of the most abundant strains. In yet another embodiment the therapeutic antibody or antigen-binding fragment thereof binds specifically to the SARS-CoV-2 glycoproteins S of at least two, at least five, at least 10, at least 20, at least 30 strains. In a most preferred embodiment it binds specifically to the SARS-CoV-2 glycoproteins S of all SARS-CoV-2-strains listed above.

In another embodiment the therapeutic antibody or antigen-binding fragment thereof binds preferably to the SARS-CoV-2 glycoproteins S of at least one strain of the "L-lineage" as listed above. In yet another embodiment the therapeutic antibody or antigen-binding fragment thereof binds preferably to the SARS-CoV-2 glycoproteins S of at least one strain of the "P-strains" as listed above.

The homotrimeric S protein is a class I fusion protein which mediates the receptor binding and membrane fusion between the virus and host cell. The S1 subunit forms the head of the spike and has the receptor binding domain (RBD). The S2 subunit forms the stem which anchors the spike in the viral envelope and on protease activation enables fusion. In addition, the membrane bound host protease TMPRSS2 is responsible for S protein priming by cleavage of the furin site between S1 and S2 as well as the S2' site for proteolytic activation, conformational change and viral entry.

The "S1 subunit", as used herein, has the nucleic acid sequence shown in SEQ ID NO: 03 and the amino acid sequence of SEQ ID NO: 04, or a substantially similar variant thereof, or a biologically active fragment thereof.

The "receptor binding domain" (RBD) or "RBD" or "S1-RBD", as used herein, has the nucleic acid sequence shown in SEQ ID NO: 05 and the amino acid sequence of SEQ ID NO: 06, or a substantially similar variant thereof, or a biologically active fragment thereof.

SARS-CoV-2 is believed to bind via the RBD to ACE2, a surface protein of the host cell.

The term "Angiotensin-converting enzyme 2" or "ACE2", as used herein, refers to the human version of Angiotensin-converting enzyme 2 having the nucleic acid sequence shown in SEQ ID NO: 07 and the amino acid sequence of SEQ ID NO: 08, or a biologically active fragment thereof.

For reference the following SEQ ID NOs pertain to the following molecules:

SEQ ID NO: 01 - DNA-sequence of full-length SARS-CoV-2 glycoprotein S (Spike)

SEQ ID NO: 02 - Protein-sequence of full-length SARS-CoV-2 glycoprotein S (Spike)

SEQ ID NO: 03 - DNA-sequence of full-length of S1-subunit of the SARS-CoV-2 glycoprotein S

SEQ ID NO: 04 - Protein-sequence of full-length of S1-subunit of the SARS-CoV-2 glycoprotein S

SEQ ID NO: 05 - DNA-sequence of full-length of receptor binding domain (RBD) of the S1-subunit of SARS-CoV-2 glycoprotein S

SEQ ID NO: 06 - Protein-sequence of full-length of receptor binding domain (RBD) of the S1-subunit of SARS-CoV-2 glycoprotein S

SEQ ID NO: 07 - DNA-sequence of full-length human ACE2

SEQ ID NO: 08 - Protein-sequence of full-length von human ACE2

In another embodiment the therapeutic antibody or antigen-binding fragment thereof specifically binds preferably to the version of the SARS-CoV-2 glycoproteins S as depicted in SEQ ID NO: 02 as well as at least one, two, three, four, five, six, or preferably all of the "escape"-mutants mentioned herein: S1-V367F, S1-N439K, S1-G476S, S1-V483A, S1-E484K, S1-G485R, S1-F486V and S1-7PM (SEQ ID NO: 1730). (The numbering of the residues correspond to the numbering used in the SWISS-MODEL workspace (YP_009724390.1);(UniProt ID PODTC2).

The use of the disclosed point mutations, preferably to the S1-7PM-mutant, for screening potential therapeutic antibodies, has the technical effect that antibodies are selected which bind not only to the "wild-type" virus, but also to other viral strains which have already developed or may develop mutations in near future. Thus, the therapeutic antibody or antigen-binding fragment thereof of the present invention is able to recognize both "wild-type" as well as mutated versions of the SARS-CoV-2-virus. Consequentially, patients with "wild-type" virus populations only, with variable virus populations, or viral populations only comprising mutated viruses, may still be treated with the inventive antibodies.

Additionally, a second effect is that it is more difficult for the virus to develop resistance to said antibodies, since the virus needs to mutate further in order to "escape" the binding of the therapeutic antibody or antigen-binding fragment thereof. As such no other strategies, like for example antibody-cocktails comprising two or more different antibodies need to be applied.

Thus, the antibodies of the present invention provide a "one fits all" solution, which is more cost-effective and results in a simplified formulation scheme and market approval process.

The sequence alignment between the "wild-type" RBD-sequence and the S1-7PM mutation is depicted below, the respective point mutations are highlighted in bold:

319  RVQPTESIVRFPNITNLCPFGEVFNATRFASVYAWNRKRISNCVADYS**F**L  368

319  RVQPTESIVRFPNITNLCPFGEVFNATRFASVYAWNRKRISNCVADYS**V**L  368

369 YNSASFSTFKCYGVSPTKLNDLCFTNVYADSFVIRGDEVRQIAPGQTGKI 418

369 YNSASFSTFKCYGVSPTKLNDLCFTNVYADSFVIRGDEVRQIAPGQTGKI 418

419  ADYNYKLPDDFTGCVIAWNS**K**NLDSKVGGNYNYLYRLFRKSNLKPFERDI  468

419  ADYNYKLPDDFTGCVIAWNS**N**NLDSKVGGNYNYLYRLFRKSNLKPFERDI  468

469  STEIYQA**S**STPCNG**AKRV**NCYFPLQSYGFQPTNGVGYQPYRVVVLSFELL  518

469  STEIYQA**G**STPCNG**VEGF**NCYFPLQSYGFQPTNGVGYQPYRVVVLSFELL  518

519 HAPATVCGPKKSTNLVKNKCVNFN   542

519 HAPATVCGPKKSTNLVKNKCVNFN   542

The term "antibody", as used herein, is intended to refer any antigen binding molecule, in some embodiments to immunoglobulin molecules comprised of four polypeptide chains, two heavy (H) chains and two light (L) chains interconnected by disulfide bonds. Each heavy chain is comprised of a heavy chain variable region ("HCVR" or "VH") and a heavy chain constant region (comprised of domains CH1, CH2 and CH3). Each light chain is comprised of a light chain variable region ("LCVR or "VL") and a light chain constant region (CL). The VH and VL regions can be further subdivided into regions of hypervariability, termed "complementarity determining regions" (CDR), interspersed with regions that are more conserved, termed "framework regions" (FR). Each VH and VL is composed of three CDRs and four FRs, arranged from amino-terminus to carboxy-terminus in the following order: FR1, CDR1, FR2, CDR2, FR3, CDR3, FR4.

Substitution of up to one, up to two or up to three amino acid residues per CDR, of up to two, up to three or up to four amino acid residues within all three CDRs of one light or heavy chain or omission of one or more CDRs is also possible.

In one embodiment up to one, up to two or up to three amino acid residues are substituted in H-CDR1. In another embodiment up to one, up to two or up to three amino acid residues are substituted in H-CDR2. In yet another embodiment up to one, up to two or up to three amino acid residues are substituted in H-CDR3. In one embodiment up to one, up to two or up to three amino acid residues are substituted in L-CDR1. In another embodiment up to one, up to two or up to three amino acid residues are substituted in L-CDR2. In yet another embodiment up to one, up to two or up to three amino acid residues are substituted in L-CDR3.

Antibodies have been described in the scientific literature in which one or two CDRs can be dispensed with for binding.

If a CDR or amino acid residue(s) thereof is omitted, it is usually substituted with an amino acid occupying the corresponding position in another human antibody sequence or a consensus of such sequences.

In one embodiment up to one, up to two or up to three amino acid residues are omitted in H-CDR1. In another embodiment up to one, up to two or up to three amino acid residues are omitted in H-CDR2. In yet another embodiment up to one, up to two or up to three amino acid residues are omitted in H-CDR3. In one embodiment up to one, up to two or up to three amino acid residues are omitted in L-CDR1. In another embodiment up to one, up to two or up to three amino acid residues are omitted in L-CDR2. In yet another embodiment up to one, up to two or up to three amino acid residues are omitted in L-CDR3.

Positions for substitution within CDRs and amino acids to substitute can also be selected empirically. Empirical substitutions can be conservative or non-conservative substitutions.

In some embodiments also in other parts of the antibody or antigen-fragment thereof amino acids may be exchanged by conservative amino acid substitution. Such a sequence variant, which differs from another sequence only by conservative amino acid substitution(s), is called herein "substantially similar variant".

A "conservative amino acid substitution" is one in which an amino acid residue is substituted by another amino acid residue having a side chain (R group) with similar chemical properties (e.g., similar charge or hydrophobicity). In general, a conservative amino acid substitution will not substantially change the functional properties of a protein. In cases where two or more amino acid sequences differ from each other by conservative substitutions, the percent or degree of similarity may be adjusted upwards to correct for the conservative nature of the substitution. Means for making this adjustment are well known to those of skill in the art.

Examples of groups of amino acids that have side chains with similar chemical properties, and whose substitution for each other constitutes conservative substitutions, include 1) aliphatic side chains: glycine, alanine, valine, leucine and isoleucine; 2) aliphatic hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartate and glutamate, and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine.

Alternatively, a conservative replacement is any change having a positive value in the PAM250 log-likelihood matrix disclosed in Gonnet et al. (1992) Science 256: 1443 45. A "moderately conservative" replacement is any change having a nonnegative value in the PAM250 log-likelihood matrix.

Sequence similarity for polypeptides is typically measured using sequence analysis software.

Protein analysis software matches similar sequences using measures of similarity assigned to various substitutions, deletions and other modifications, including conservative amino acid substitutions.

For instance, GCG software contains programs such as GAP and BESTFIT which can be used with default parameters to determine sequence homology or sequence identity between closely related polypeptides, such as homologous polypeptides from different species of organisms or between a wild type protein and a mutein thereof.

Polypeptide sequences also can be compared using FASTA with default or recommended parameters; a program in GCG Version 6.1. FASTA (e.g., FASTA2 and FASTA3) provides alignments and percent sequence identity of the regions of the best overlap between the query and search sequences (Pearson (2000) supra).

Another preferred algorithm when comparing a sequence of the invention to a database containing a large number of sequences from different organisms is the computer program BLAST, especially BLASTP or TBLASTN, using default parameters. See, e.g., Altschul et al. (1990) J. Mol. Biol. 215: 403 410 and (1997) Nucleic Acids Res. 25:3389 402.

The term "sequence identity" when referring to a nucleic acid or antigen-binding fragment thereof, indicates that, when optimally aligned with appropriate nucleotide insertions or deletions with another nucleic acid (or its complementary strand), there is nucleotide sequence identity in at least about 90%, and more preferably at least about 95%, at least about 96%, at least about 97%, at least about 98% or at least about 99% of the nucleotide bases, as measured by any well-known algorithm of sequence identity, such as FASTA, BLAST or GAP, as discussed.

As applied to polypeptides, the term "substantial similarity" or "substantially similar" means that two peptide sequences, when optimally aligned, such as by the programs GAP or BESTFIT using default gap weights, share at least 90% sequence identity, even more preferably at least 95%, at least 98% or at least 99% sequence identity. Preferably, residue positions which are not identical differ by conservative amino acid substitutions.

In some embodiments, the invention is a monoclonal antibody, a polyclonal antibody, a recombinant antibody, a human antibody, a humanized antibody, a chimeric antibody, a multispecific antibody, or an antigen-binding fragment thereof. In some embodiments, the isolated antigen binding protein is a Fab fragment, a Fab' fragment, a F(ab')2 fragment, a Fv fragment, a diabody, a nanobody or a single chain antibody molecule. In some embodiments, the isolated antigen binding protein is of the IgG1-, IgG2- IgG3- or IgG4-type. In some embodiments the antibody may be of IgA-, IgD-, IgE- or IgM-type.

In one embodiment the invention is a monoclonal antibody of IgG1-Type. In another embodiment the invention is a scFV-fragment.

The term "human antibody", as used herein, is intended to include antibodies having variable and constant regions derived from human germline immunoglobulin sequences. The human mAbs of the invention may include amino acid residues not encoded by human germline immunoglobulin sequences (e.g., mutations introduced by random or site-specific mutagenesis in vitro or by somatic mutation in vivo), for example in the CDRs.

The term "therapeutic" antibody or antigen-binding fragment thereof, as used herein, is intended to differentiate the antibodies of this invention from "regular" antibodies or fragments thereof. Regular antibodies may also bind to the target, but usually they show low affinity, or low specificity, or neutralizing effect, or a combination thereof, to the target, i.e. SARS-CoV-2 glycoprotein S. Regular antibodies may also show other undesired features, such as dimerization or multimerization. Such regular antibodies may be suitable for certain scientific applications, such as assays. However, such regular antibodies must not be used for therapeutic purposes, which necessitate therapeutic antibodies with high affinity, high specificity and/or high neutralizing effects; and which are free of undesired features, such as agglutination, such as dimerization or multimerization, which may lead to an undesired host-response, for example immune-response, after treatment with such antibodies and, therefore, such antibodies are normally not admitted for therapeutic use by the regulatory health authorities.

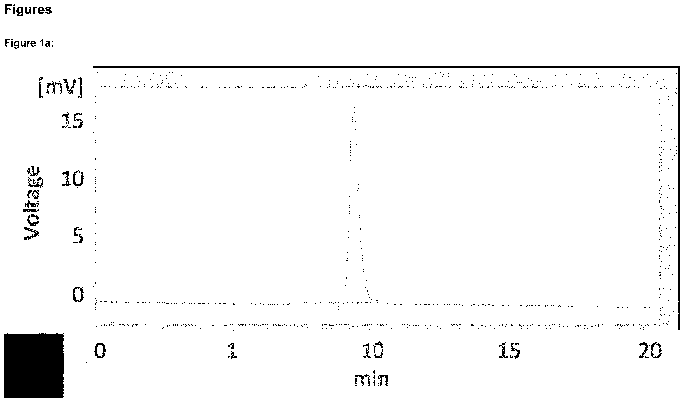

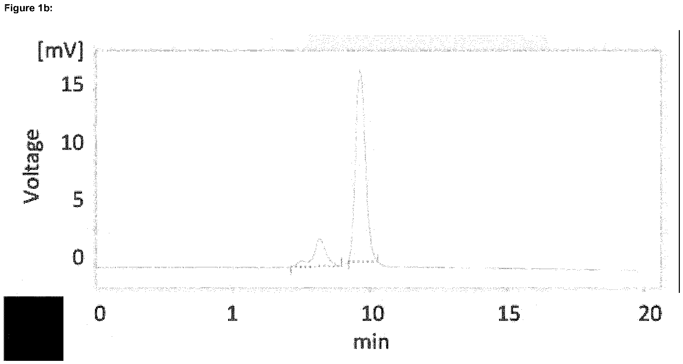

The difference in appearance of a therapeutic antibody and a regular antibody as measured by SEC-HLPC is shown in Figures 1a and 1b. Figure 1a depicts a therapeutic antibody which is nearly 100% monomeric free of agglutination, whereas Figure 1b depicts a regular antibody with dimeric and multimeric peaks. The integral of the curves depicts the amount of monomeric, dimeric and multimeric antibodies. Please note that agglutinations elute quicker from SEC-HPLC than monomers.

In some embodiments, the therapeutic antibodies or antigen binding fragment thereof should be monomeric antibodies with a contamination with agglutinations of less than 10 mol-%, preferably less than 5 mol-%, more preferably less than 2 mol-%, most preferred less than 1 mol-% of agglutinations such as dimeric and/or multimeric antibodies as measured with size-exclusion chromatography (SEC)-HPLC with PBS at 20°C according to standard protocol.

In some embodiments the contamination with agglutinations is "absent", that is, not measureable with SEC-HPLC (i.e. is between 0 - 0.25 mol-%).

In another embodiment the therapeutic antibodies or antigen binding fragment thereof are monomeric antibodies are defined by a "contamination ratio" which is the ratio of the integrals of the HPLC-peak as measured with size-exclusion chromatography (SEC)-HPLC denoting the monomeric antibody (int-mon) and the integrals of the agglutination HPLC-peaks as measured with size-exclusion chromatography (SEC)-HPLC (int-mult) as depicted in Formula I:

Formula I:

$$\frac{int - mult}{int - mon} = contamination\ ratio$$

In another embodiment the therapeutic antibodies or antigen binding fragment thereof possesses a "contamination ratio" less than 0.2, more preferred of less than 0.1, even more preferred of less than 0.05, even more preferred of less than 0.033, even more preferred of less than 0.02.

In some embodiments the therapeutic antibodies or antigen binding fragment thereof is monomeric antibodies free of any contamination with dimeric and/or multimeric antibodies as measured with size-exclusion chromatography (SEC)-HPLC with PBS at 20°C according to standard protocol..

Dimeric and multimeric antibodies, also summarized with the term "agglutination" may provoke unwanted side-effects during therapy and must be reduced or, preferably, completely avoided.

Without being bound to theory, the formation of antibody agglutinations during manufacturing, storage and distribution, the exposure of the antibody to different stress conditions such as mechanical stress, light exposure, temperature differences, pH variations and/or contact to different surface materials, may be the result of mal-designed antibodies (e.g. unfavorable charge distribution, unpaired cysteine-residues, unfavorable hydrophobic patches and the like).

In some embodiments of the present invention the therapeutic antibodies or antigen binding fragment thereof should possess a low "stickiness". The term "stickiness" means the ability of the antibody to aggregate unspecifically with other antibodies. This "stickiness" can be measured by a "specificity ELISA" as outlined in more detail in the examples-section.

Preferred antibodies show a mean stickiness against a control-antibody (for example against Avelumab) of less than 0.9, preferably of less than 0.8, more preferably of less than 0.7, even more preferably of less than 0.6; most preferably of less than 0.5 when measured in a specificity ELISA.

In some embodiments the therapeutic antibodies and antigen-binding fragments thereof possess a stickiness of between 0.4 and 0.75 when measured in a specificity ELISA in comparison to Avelumab.

Fc mediated effector functions support anti-viral function of antibodies, but they can also cause antibody dependent enhancement (ADE) and other severe adverse effect such as cytokine storms and hyperinflammation.

Potential adverse effects of active Fc portion of IgG1 are:

- ADE: FcγR binding on phagocytes or other cells may re-target infection (It is possible that non-neutralizing anti-virus antibodies and/or suboptimal concentrations may actually enhance the infection).

- Hyperinflammation and/or cytokine storm: FcγR binding and activation on immune cells may lead to enhancement of inflammatory events associated with the viral infection or underlying diseases.

- Complement activation may lead to endotheliopathy, hypercoagulability, hyperinflammation and cytokine storm.

COVID-19 is associated with large number of risk factor, mostly underlying diseases (ca. 90% of severe cases in the US). Therefore, antibody drugs with an active Fc portion may cause adverse effects in these cases and safety measures need to be included into the drug and therapy design.Thus, in some embodiments of the present invention, the constant region (Fc-region) of the therapeutic antibody and/or antigen-binding fragment thereof was designed or further amended in order prevent an antibody-dependent enhancement (ADE) and/or cytokine storm syndrome (CSS).

The term "antibody-dependent enhancement" (ADE), sometimes less precisely called immune enhancement or disease enhancement, is a phenomenon in which binding of a virus to antibodies enhances its entry into host cells, and sometimes also its replication.

It is believed that some host cells lack the usual receptors on their surfaces that the virus uses to gain entry, but they have Fc receptors that bind to one end of antibodies. The virus binds specifically to the antigen-binding site at the other end, and in this way gains entry to and infects the host cell, potentially increasing the severity of the disease.

The "cytokine storm", also called cytokine storm syndrome (CSS), is a bodily reaction in human immune systems in which the innate immune system releases a large number of cytokines, potentially overwhelming the body and possibly leading to acute respiratory distress syndrome (ARDS) and even fatality. It is believed that many severe episodes of COVID-19 are related at least partially to the cytokine storm syndrome.

The respective modifications which were introduced in the constant region in order to produce "silent Fc"-regions are depicted in table 2. In some embodiments already one, two or three of the depicted modifications may be introduced in order to achieve sufficient silencing.

**Table 2: Modifications in Fc-region in order to produce silenced Fc.**

| Fc mutation | Effect on FcγRs Ratio to wt mean (SD) | | | | Effect on complement | FcRn | References and remarks |
|---|---|---|---|---|---|---|---|
| Numbering [3] | FcγR1 | FcγR 2A | FcγR 2B | FcγR 3A | | | |
| E233P | 0.12 (0.06) | 0.08 (0.06) | 0.12 (0.01) | 0.04 (0.02) | ? | 0.54 (0.20) | [1] from IgG2 oth er mutations at this position reduce FcγR binding (e.g. LALA) |
| L234V | | | | | ? | | |
| L235A | | | | | ? | | |
| ΔG236 | | | | | ? | | |
| D265G | 0.16 (0.05) | 0.07 (0.01) | 0.13 (0.05) | 0.09 (0.06) | Reduced C 1q binding? | 1.23 (0.14) | [1] D265A: 44.7% 1-3 terminal Gal and highe r sialation vs wt: 33.9% |
| A327Q | 0.6 (0.12) | 0.13 (0.03) | 0.14 (0.03) | 0.06 (0.01) | ? | 0.97 | [1] similar A327G [2] |
| A330S | ? | ? | ? | ? | Decreased C1q | ? | [2] from IgG4. co mplement binding |
| Total | 0 | 0 | 0 | 0 | 0 | | |

SD =Standard deviation; wt =wildtype IgG1-Fc
[1] Shields et al.
[2] Armour, KL, Clark, MR, Hadley, AG, and Williamson, LM. Recombinant human IgG molecules lacking Fcγ receptor I binding and monocyte triggering activities Eur. J. Immunol. 1999. 29: 2613-2624.
[3] Kabat, EA, Wu, TT, Perry, HM, Gottesman, KS and Foeller, C, Sequences of proteins of immunological interest. US Department of Health and Human Services, NIH, Bethesda 1991. (EU Numbering system).

It is obvious to the person skilled in the art, that, whereas regular antibodies may not be used for therapeutic use i.e. *in vivo* application and/or treatment of patients because of the above-mentioned reasons, therapeutic antibodies are suitable also for non-therapeutic use i.e. *in vitro* assays, and the like.

The term "antibody fragment", as used herein, refers to one or more fragments of an antibody that retains the ability to specifically bind to S1-protein and/or RBD.

An "antibody fragment" may include a Fab fragment, a F(ab')2 fragment, a Fv fragment (including scFv-fragments), a dAb fragment, a fragment containing a CDR, or an isolated CDR.

The present invention generally relates to antibodies or antigen-binding fragments thereof capable of specifically blocking and/or inhibiting the interaction between the SARS-CoV-2 spike protein and the ACE receptor, particularly the human ACE receptor, as well as combinations comprising two or more different antibodies or antigen-binding fragments. Specific amino acid sequences of relevant antibody regions are described in the enclosed sequence listing.

The term "specifically blocking and/or inhibiting the interaction between the SARS-CoV-2 spike protein and the ACE receptor" as used herein, refers to the inhibition of the binding between the S1 domain of the spike protein and an ACE positive cell, particularly when determined as described in the present examples. In particular embodiments, the antibody binds specifically to the S1 domain of the spike protein.

More particularly, the present invention relates to an antibody or antigen-binding fragment thereof capable of specifically blocking and/or inhibiting the interaction between the SARS-CoV-2 spike protein and the human ACE receptor defined by one or more complementarity-determining regions (CDRs) and/or by variable heavy chain (VH) and optionally variable light chain (VL) regions comprising said CDRs or related sequences.

In some embodiments of the present invention, the antibodies or antigen-binding fragments of the invention are neutralizing.

A "neutralizing antibody", as used herein (or an "antibody that neutralizes the infectious activity of SARS-CoV-2"), is intended to refer to an antibody whose binding specifically to the SARS-CoV-2 glycoprotein S, more preferably the S1-subunit and/or RBD results in inhibition of virus infectivity.

The term "virus infectivity" or "viral infection" as used herein means any viral activity in the host. Viral infection occurs when a host's body is invaded by SARS-CoV-2, and infectious virus particles (virions) attach to and enter susceptible host cells. Thus, the term "virus infectivity" includes viral activities, such as the entry of virus particles into host cells, the replication of the virus within the host cell, the lysis of the host cell and spreading of the virus to further cells. However, "inhibition of virus infectivity" can also mean the prevention, reduction or healing of any disease or condition resulting from a SARS-CoV-2 infection, especially symptoms associated with COVID-19. Such a disease or condition includes one or more symptoms selected from the list comprising fever, cough, fatigue, shortness of breath, loss of smell and taste, pleurisy, pericarditis, lung consolidation, pulmonary oedema, pneumonia, serous exudation, fibrin exudation, pulmonary oedema, pneumocyte hyperplasia, large atypical pneumocytes, interstitial inflammation with lymphocytic infiltration and multinucleated giant cell formation, diffuse alveolar damage (DAD) with diffuse alveolar exudates, acute respiratory distress syndrome (ARDS), severe hypoxemia, exudates in alveolar cavities and pulmonary interstitial fibrosis plasmocytosis in bronchoalveolar lavage (BAL), disseminated intravascular coagulation (DIC), leukoerythroblastic reaction and microvesicular steatosis in the liver and any combination thereof.

The terms "neutralizing" and "blocking" or "inhibition" are not to be confused with each other. Antibodies exist, which bind to the S1-subunit and/or RBD in such a way that they block/inhibit the binding of the spike-protein to the ACE2-receptor, however, they still may not neutralize the infectious activity of SARS-CoV-2, and, thus, possess no neutralizing activity.

Only if also at least one infectious activity of the SARS-CoV-2-virus is inhibited, the term "neutralizing" is used herein. This inhibition of the infectious activity can be assessed by measuring one or more indicators of viral activity by one or more of several standard *in vitro* or *in vivo* assays known in the art, such as for example the SARS-CoV-2 VERO E6 neutralizing assay, wherein VERO E6 cells are infected with live SARS-CoV-2 virus and the neutralization of the antibody or antigen-binding fragment thereof is measured by the retention of confluence after certain time points.

VERO E6 cells are commonly used for in vitro studies of the severe acute respiratory syndrome-associated coronavirus (SARS-CoV) and for antiviral evaluation purposes. In these tests the SARS-CoV growth kinetics in VERO E6 cells is used in order to elucidate the mechanism of antiviral activity of selective antiviral agents. The growth kinetics of SARS-CoV in VERO E6 cells can be measured qualitatively (or half-quantitatively) by cell-confluence measurement as outlined above and/or quantitatively by intra- and extracellular viral RNA load as well as extracellular virus yield at different time points post-infection.

Without virus-neutralizing agents, at 12 h post-infection, the intracellular viral RNA load is about $3 \times 10^2$-fold higher than at the time of infection, and the extracellular viral RNA load is increased by a factor of about $2 \times 10^3$. Intracellular viral RNA levels started to rise at 6 h post-infection. One hour later (at 7 h post-infection), the levels of extracellular SARS-CoV RNA also begins to rise. This is corroborated by the fact that infectious progeny SARS-CoV also first appeared in the supernatant between 6 and 7 h post-infection. At 12 h post-infection, SARS-CoV reaches titers in the supernatant of $5.2 \times 10^3$ $CCID_{50}$/ml.

Thus, a "neutralizing antibody or antigen-binding fragment thereof" is any antibody or antigen-binding fragment thereof where confluence in a SARS-CoV-2 VERO E6 neutralizing assay in % at 4 dpi is at least 80%, more preferred at least 85%, even more preferred at least 90%, even more preferred at least 95%, most preferred at least 98% after incubation with a mix comprising at least medium, active SARS-CoV-2 and the neutralizing antibody or antigen-binding fragment thereof. In some embodiments, e.g. see example 9, the viral strain SARS-CoV-2/Münster/FI 110320/1/2020 was used. The skilled person is aware that it is possible to use also other SARS-CoV-2 viral strains with similar infectivity to VERO E6 cells.

In case of some embodiments the confluence in SARS-CoV-2 VERO E6 neutralizing assay in % at 4 dpi is between 98% and up to 100%, e.g. the same confluence as visible for non-infected VERO E6 - cells at the same time-point.

In some embodiments the time-point is chosen about 3.5 days after the first incubation with SARS-CoV-2, in other embodiments 3 days and 18 hours; in further embodiments 4 days, 5 days and up to 6 days, or 7 days.

In another embodiment, a "neutralizing antibody or antigen-binding fragment thereof' is any antibody or antigen-binding fragment thereof where extracellular viral RNA load in a SARS-CoV-2 VERO E6 neutralizing assay is reduced by at least 50% as compared to infected cells without treatment with a "neutralizing antibody or antigen-binding fragment thereof", more preferred at least 75%, even more preferred at least 80%, even more preferred at least 90%, even more preferred at least 95%, even more preferred at least 99%, most preferred 99.9%.

In another embodiment, a "neutralizing antibody or antigen-binding fragment thereof' is any antibody or antigen-binding fragment thereof where intracellular viral RNA levels in a SARS-CoV-2 VERO E6 neutralizing assay is reduced by at least 50% as compared to infected cells without treatment with a "neutralizing antibody or antigen-binding fragment thereof", more preferred at least 75%, even more preferred at least 80%, even more preferred at least 90%, even more preferred at least 95%, even more preferred at least 99%, most preferred 99.9%.

In yet another embodiment, a "neutralizing antibody or antigen-binding fragment thereof" is any antibody or antigen-binding fragment thereof where at 12h post infection SARS-CoV-2 titers in the supernatant in a SARS-CoV-2 VERO E6 neutralizing assay is reduced by at least 50% as compared to infected cells without treatment with a "neutralizing antibody or antigen-binding fragment thereof', more preferred at least 75%, even more preferred at least 80%, even more preferred at least 90%, even more preferred at least 95%, even more preferred at least 99%, most preferred 99.9% and/or the virus titration by cell culture infectious dose 50% assay ($CCID_{50}$/ml) is less than $5 \times 10^2$ $CCID_{50}$/ml, more preferred less than 1 x $10^2$ $CCID_{50}$/ml, $5 \times 10^1$ $CCID_{50}$/ml, or 1 x $10^1$ $CCID_{50}$/ml.

Thus, in some embodiments the "antibody that neutralizes S1-subunit and/or RBD activity" inhibits or reduces the interaction, in some embodiments the binding of the S1-subunit and/or RBD to the ACE2-protein on the host cell surface and results in prevention or reduction of entry of virus particles into the host cell.

In some embodiments, the neutralizing ability in vitro is described in terms of an $IC_{50}$, value. In case of in vivo neutralizing ability the $EC_{50}$ is preferred. Thus, the $IC_{50}$ value indicates the concentration of an inhibitor which is necessary to block 50% of an enzyme, a cell, a cell receptor or a microorganism (in general: "targets") in vitro. The $EC_{50}$ value indicates this required concentration in vivo, i.e. for the binding ability to S1/RBD.

Thus, in yet another embodiment, a "neutralizing antibody or antigen-binding fragment thereof" is any antibody or antigen-binding fragment thereof where the $IC_{50}$ as measured in the VERO E6 cell assay is less than 0.2 $\mu$g/ml, less than 0.1 $\mu$g/ml, more preferred less than 0.05 $\mu$g/ml even more preferred less than 0.04 $\mu$g/ml, even more preferred less than 0.036 $\mu$g/ml, even more preferred less than 0.030 $\mu$g/ml, most preferred less than 0.026 $\mu$g/ml as measured in an infectious VERO E6 cell assay.

In another embodiment the neutralizing effect of the therapeutic antibody ($IC_{50}$) is about 0.03672 $\mu$g/ml, in yet another embodiment is about 0.03599 $\mu$g/ml as measured in an infectious VERO E6 cell assay, in yet another embodiment is about 0.02539 $\mu$g/ml as measured in an infectious VERO E6 cell assay.

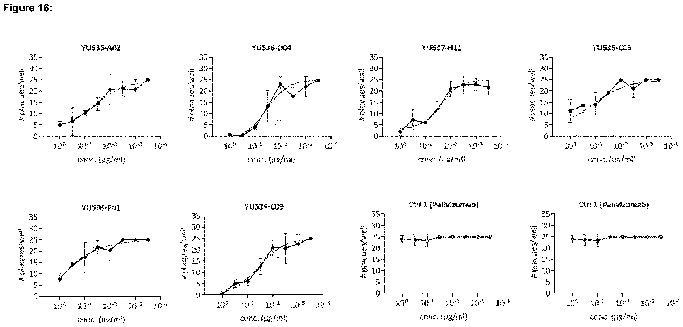

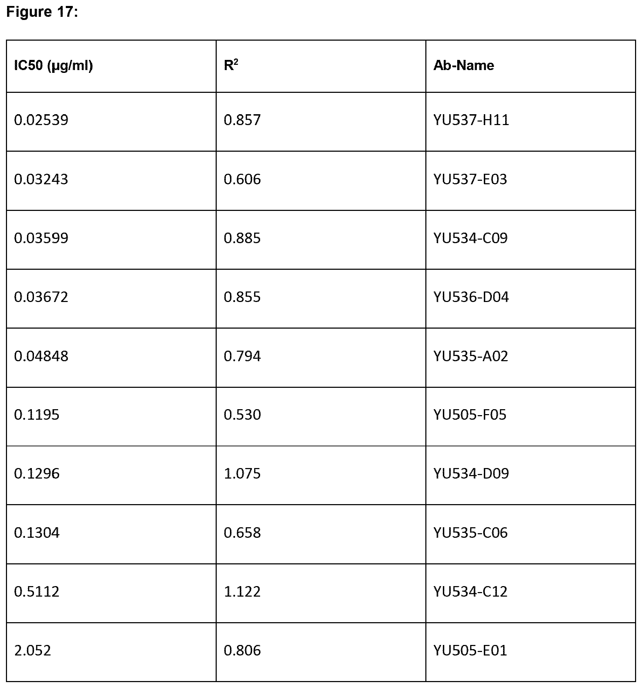

Further examples for $IC_{50}$-values of therapeutic antibodies of the present invention are depicted in figures 16 and 17.

In one embodiment only antibodies with said excellent low $IC_{50}$-values of less than 0.06 $\mu$g/ml even more preferred less than 0.049 $\mu$g/ml, even more preferred less than 0.036 $\mu$g/ml, most preferred less than 0.026 $\mu$g/ml as measured in an infectious VERO E6 cell assay, can be considered "therapeutic antibodies", since only these antibodies facilitate sufficient neutralizing activity within a patient.

In certain embodiments, the antibody or antigen-binding fragment has an $EC_{50}$ value of about 20 nM or less on RBD, of about 10 nM or less, or of about 5 nM or less for the S1 domain of the spike domain, particularly when determined as described in the present example 9.

A low $IC_{50}$ and/or $EC_{50}$ may result in the beneficial effect that smaller antibody dosages can be administered. This has an effect on the frequency, viscosity and/or volume of the pharmaceutical composition which needs to be administered.

For example an antibody with a less preferred higher $IC_{50}$ and/or $EC_{50}$-value may necessitate a higher frequency of treatment, e.g. instead of once or twice during the whole course of the disease, maybe each day or several times per day.

It may also result in the effect that the volume per treatment needs to be so high that not a normal syringe or auto-injector, but a large volume device, i.e. pump-device or perfusion needs to be used.

It may also result in the effect that the administration device needs to be composed of less-breakable material, since due to the high viscosity of the high concentrated antibody composition needs to be applied with elevated pressure.

Finally, it may also result in the effect that the antibody-composition is not commercially feasible.

In certain embodiments, the antibody or antigen-binding fragment inhibits the binding specifically to the spike protein with a molar ratio of about 70:1 or less, particularly when determined as described in the present example 3.

In one embodiment YU536-D04 (SEQ ID NOs: 349 - 368) is considered such a "therapeutic antibody", in yet another embodiment YU537-H11 (SEQ ID NOs: 389 - 408) is considered such a "therapeutic antibody", in yet another embodiment STE90-C11 (SEQ ID NO: 1305 - 1312) is considered such a "therapeutic antibody", in yet another embodiment YU505-A02 (SEQ ID NO: 909 - 928) is considered such a "therapeutic antibody".

Thus, in one embodiment the therapeutic antibody or antigen-binding fragment thereof that binds specifically to SARS-CoV-2 glycoprotein S with the amino acid sequence of SEQ ID NO: 02 and neutralizes the infectious activity of SARS-CoV-2 is selected from the list consisting of: YU505-A01 (SEQ ID NOs: 889 - 908), YU505-A02 (SEQ ID NOs: 909 - 928), YU505-F05 (SEQ ID NOs: 1149 - 1168), YU534-C12 (SEQ ID NOs: 169 - 188), YU534-D09 (SEQ ID NOs: 509 - 528), YU535-A02 (SEQ ID NOs: 189 - 208), YU537-H08 (SEQ ID NOs: 769 - 788), YU537-H11 (SEQ ID NOs: 389 - 408), YU534-C09 (SEQ ID Nos: 149 - 168), YU536-D04 (SEQ ID NOs: 349 - 368), YU535-A02 (SEQ ID NOs: 189 - 208), YU535-C06 (SEQ ID NOs: 529 - 548) and YU505-E01 (SEQ ID NOs: 1049 - 1068).

In some embodiments, the antibodies in Table 1 above are strong neutralizers.

In some embodiments of the present invention, the antibodies or antigen-binding fragments of the invention bind specifically to the target.

The term "specifically binds" or "binds specifically" or the like, means that an antibody or antigen-binding fragment thereof forms a complex with an antigen that is relatively stable under physiologic conditions. Specific binding can be characterized by an equilibrium dissociation constant of less than $1\times10^{-6}$ M, preferably less than $1\times10^{-7}$ M, more preferably less than $1\times10^{-8}$ M, even more preferably less than $1\times10^{-9}$ M; most preferably less than $1\times10^{-10}$ M or less (e.g., a smaller $K_D$ denotes a tighter binding) as measured by affinity chromatography using protein A or surface plasmon resonance, e.g. BIACORE™, biolayer interferometer (BLI), SPRi, solution-affinity ELISA. An isolated antibody or antigen-binding fragment thereof that specifically binds the S1-subunit and/or RBD may, however, still have cross-reactivity to other related antigens, such as the S1-subunit and/or RBD molecules from other species.

Methods for determining whether two molecules specifically bind are well known in the art and include, for example, equilibrium dialysis, surface plasmon resonance, and the like. An isolated antibody that specifically binds the S1-subunit and/or RBD may, however, exhibit cross-reactivity to other antigens such as S1-subunit and/or RBD molecules from other viral species.

In some embodiments of the present invention, the antibodies or antigen-binding fragments of the invention bind with high affinity to the target.

The term "$K_D$", as used herein, is intended to refer to the equilibrium dissociation constant of a particular antibody-antigen interaction.

The term "high affinity" antibody refers to those mAbs having a binding affinity $K_D$ to S1-protein and/or RBD of less than $1\times10^{-8}$ M, preferably of less than $5 \times 10^{-9}$ M, preferably of less than $1\times10^{-9}$ M, preferably of less than $1\times10^{-10}$ M; more preferably of less than $10^{-11}$ M; even more preferably $10^{-12}$ M or less, as measured by affinity chromatography using protein A or by surface plasmon resonance (BIACORE™), or by Bio-Layer Interferometry (BLI).

The terms "fast on rate" or "$k_{on}$" or "ka" refer to an antibody or antigen-binding fragment thereof that dissociates from S1-subunit and/or RBD with a rate constant of $1 \times 10^5$ Ms$^{-1}$ or more, preferably $1 \times 10^6$ Ms$^{-1}$ or more, as determined by surface plasmon resonance, e.g., BIACORE™.

The terms "slow off rate" or "$k_{off}$" or "kd" refer to an antibody or antigen-binding fragment thereof that dissociates from S1-subunit and/or RBD with a rate constant of $1 \times 10^{-3}$ s$^{-1}$ or less, preferably $1 \times 10^{-4}$ s$^{-1}$ or less, as determined by Bio-Layer Interferometry (BLI).

The term "Bio-Layer Interferometry (BLI)", as used herein, refers a label-free technology for measuring biomolecular interactions. It is an optical analytical technique that analyzes the interference pattern of white light reflected from two surfaces: a layer of immobilized protein on the biosensor tip, and an internal reference layer. Any change in the number of molecules bound to the biosensor tip causes a shift in the interference pattern that can be measured in real-time.

The binding between a ligand immobilized on the biosensor tip surface and an analyte in solution produces an increase in optical thickness at the biosensor tip, which results in a wavelength shift, $\Delta\lambda$, which is a direct measure of the change in thickness of the biological layer. Interactions are measured in real time, providing the ability to monitor binding specificity, rates of association and dissociation, or concentration, with high precision and accuracy.

Only molecules binding specifically to or dissociating from the biosensor can shift the interference pattern and generate a response profile. Unbound molecules, changes in the refractive index of the surrounding medium, or changes in flow rate do not affect the interference pattern. This is a unique characteristic of bio-layer interferometry and extends its capability to perform in crude samples used in applications for protein-protein interactions, quantitation, affinity, and kinetics.

BLI-systems are commercially available such as for example Octet® QKe (Fortebio/Sartorius GmbH, Göttingen, Germany).

The term "surface plasmon resonance", as used herein, refers to an optical phenomenon that allows for the analysis of real-time biospecific interactions by detection of alterations in protein concentrations within a biosensor matrix, for example using the BIACORE™ system.

An "isolated antibody", as used herein, is intended to refer to an antibody or antigen-binding fragment thereof that is substantially free of other antibody or antigen-binding fragment thereof having different antigenic specificities (e.g., an isolated antibody that specifically binds the S1-subunit and/or RBD is substantially free of mAbs that specifically bind antigens other than the S1-subunit and/or RBD).

The term "compete" when used in the context of the antibody or antigen-binding fragment thereof means competition between the reference antibody or antigen-binding fragment thereof in binding specifically to the target protein (also called "antigen", in the specific case of the invention this is the RBD and/or the S1-subunit of SARS-CoV-2 S protein) with other antigen-binding molecule.

Such antigen-binding molecule may be selected from the list of: natural ligands to the RBD and/or S1-subunit of SARS-CoV-2 S protein; including ACE2 and fragments thereof; other antibodies or antigen-binding fragments thereof; diabodies, nanobodies, anticalins, scab-proteins, single-chain antibody variable regions, single-chain fragments variables (scFv), etc.

This competition may be determined by an assay in which the reference antibody or antigen-binding fragment thereof (i.e. one of the reference antibodies mentioned hereinunder) prevents or inhibits (e.g., reduces) specific binding of another antigen-binding molecule, such as for example a test-antibody or antigen-binding fragment thereof and/or ACE2 or fragments thereof, from binding specifically to the RBD of the S1-subunit of the SARS-CoV-2 S protein.

Numerous types of competitive binding assays can be used to determine if one antigen binding protein competes with another, for example:

- solid phase direct or indirect radioimmunoassay (RIA), solid phase direct or indirect enzyme immunoassay (EIA), sandwich;

- competition assay (see, e.g., Stahli et al., 1983, Methods in Enzymology 9:242-253);

- solid phase direct biotin-avidin EIA (see, e.g., Kirkland et al., 1986, J. Immunol. 137:3614-3619)

- solid phase direct labeled assay;

- solid phase direct labeled sandwich assay (see, e.g., Harlow and Lane, 1988, Antibodies, A Laboratory Manual, Cold Spring Harbor Press);

- solid phase direct label RIA using I-125 label (see, e.g., Morel et al., 1988, Molec. Immunol. 25:7-15);

- solid phase direct biotin-avidin EIA (see, e.g., Cheung, et al., 1990, Virology 176:546-552); and direct labeled RIA (Moldenhauer et al., 1990, Scand. J. Immunol. 32:77-82).

Typically, such an assay involves the use of purified RBD and/or S1-subunit of SARS-CoV-2 S protein as "antigen", bound to a solid surface or cells bearing either of these, an unlabeled test antigen-binding molecule and a labeled reference antigen-binding protein, for example a reference antibody.

In one embodiment the labeled reference antigen-binding protein may be selected from a group of amino acids depicted in SEQ IDs NO: 906, 908, 926, 928, 1066, 1068, 1166, 1168, or any combination and/or antigen-binding fragment thereof.

In one embodiment the labeled "reference antibody" is selected from YU505-A02 (SEQ ID NOs: 909 - 928), YU536-D04 (SEQ ID NOs: 349 - 368), YU537-H11 (SEQ ID NOs: 389 - 408) and/or STE90-C11 (SEQ ID NOs: 1305 -1312).

In another embodiment the labeled reference antigen-binding protein may be ACE2 of human or mammalian (e.g. mouse, dog, monkey, bat, pangolin) origin, or any fragment thereof which is still able to bind to the S1-subunit of the SARS-CoV-2 glycoprotein S domain.

In yet another embodiment the labeled reference antigen-binding protein may be any molecule, protein or any fragment thereof which is able to bind to the S1-subunit of the SARS-CoV-2 glycoprotein S domain, preferably the RBD-domain of SARS-CoV-2 glycoprotein S.

Competitive inhibition is measured by determining the amount of label bound to the solid surface or cells in the presence of the test antigen binding protein. Usually the antigen binding protein is present in excess.

Antigen binding proteins identified by competition assay (competing antigen-binding molecules) include antigen binding proteins binding specifically to the same epitope as the reference antigen binding proteins and antigen binding proteins binding specifically to an adjacent epitope sufficiently proximal to the epitope bound by the reference antigen binding protein for steric hindrance to occur.

Usually, when a competing antigen binding protein is present in excess, it will inhibit (e.g., reduce) specific binding of a reference antigen binding protein to a common antigen by at least 40%, at least 45%, at least 50%, at least 55%, at least 60%, at least 65%, at least 70% or at least 75% or more.

In some instances, binding is inhibited by at least 80%, at least 85%, at least 90%, at least 95%, or at least 97% or more.

In one embodiment binding is inhibited by at least 95%.

The term "epitope" is a region of an antigen that is bound by an antibody. Epitopes may be defined as structural or functional. Functional epitopes are generally a subset of the structural epitopes and have those residues that directly contribute to the affinity of the interaction. Epitopes may also be conformational, that is, composed of non-linear amino acids. In certain embodiments, epitopes may include determinants that are chemically active surface groupings of molecules such as amino acids, sugar side chains, phosphoryl groups, or sulfonyl groups, and, in certain embodiments, may have specific three-dimensional structural characteristics, and/or specific charge characteristics.

The therapeutic antibodies and antigen-binding fragments thereof of the present invention bind preferably to the RBD-domain of the S1-subunit of the SARS-CoV-2 glycoprotein S. In preferred embodiments the therapeutic antibodies and antigen-binding fragments thereof of the present invention bind both to the RBD-domain as well as the S1-subunit of the SARS-CoV-2 glycoprotein S.

The reference antibodies which are selected from a group of amino acids depicted in SEQ IDs NO: 906, 908, 926, 928, 1066, 1068, 1166, 1168, or any combination and/or antigen-binding fragment thereof, define a "sweet-spot" for binding specifically to the RDB and/or S1-subunit of the SARS-CoV-2 glycoprotein S.

In one embodiment the antibodies YU505-A01 (SEQ ID NOs: 889 - 908), YU505-A02 (SEQ ID NOs: 909 - 928), YU505-E01 (SEQ ID NOs: 1049 - 1068) and YU505-F05 (SEQ ID NOs: 1149 - 1168) have been selected as reference antibodies.

In a further embodiment the antibodies YU534-C09 (SEQ ID Nos: 149 - 168), YU534-C12 (SEQ ID NOs: 169 - 188), YU534-D09 (SEQ ID NOs: 509 - 528), YU535-A02 (SEQ ID NOs: 189 - 208), YU536-D04 (SEQ ID NOs: 349 - 368), YU537-H08 (SEQ ID NOs: 769 - 788) and YU537-H11 (SEQ ID NOs: 389 - 408), and STE90-C11 (SEQ ID NO: 1305 - 1312) have been selected as reference antibodies.

In one embodiment the antibody YU505-A02 (SEQ ID NOs: 909 - 928) has been selected as reference antibody. That is, any other antibody or antigen-binding fragment thereof which competes with YU505-A02 for binding specifically to the wild-type RBD of SEQ ID No. 06 and/or for binding specifically to the S1-7PM-mutant (SEQ ID NO. 1730) in a competition assay, is considered a therapeutic antibody or antigen-binding fragment thereof of the present invention.

In other embodiments YU536-D04 (SEQ ID NOs: 349 - 368) is another preferred reference antibody, in yet another embodiment YU537-H11 (SEQ ID NOs: 389 - 408) and/or STE90-C11 (SEQ ID NOs: 1305 -1312) are preferred reference antibodies.

Thus, in one embodiment the "reference antibody" is selected from YU505-A02 (SEQ ID NOs: 909 - 928), YU536-D04 (SEQ ID NOs: 349 - 368), YU537-H11 (SEQ ID NOs: 389 - 408) and/or STE90-C11 (SEQ ID NOs: 1305 -1312).

The term "sweet spot" as used herein means an epitope of specific preferred quality with respect to the in one embodiment desired characteristics of the neutralizing antibody or antigen-binding fragment thereof. Thus, the binding specifically to the sweet spot is desired in order to achieve therapeutic antibodies with SARS-CoV-2 neutralizing activity.

In one embodiment the "sweet spot" comprises the amino acids 437 to 463 of the SARS-CoV-2 S-protein, which sequence is: 437-NSNNLDSKVGGNYNYLYRLFRKSNLKP-463.

Therefore, in one embodiment a therapeutic antibody and/or antigen binding fragment, competes with ACE2 and/or one of the reference antibodies, and/or antigen binding fragment thereof, for binding with at least to one of amino acid to one of the epitopes selected from the group comprising Serine 438, Asparagine 439, Leucine 441, Serine 443, Lysine 444, Valine 445, Glycine 446, Leucine 452, Leucine 455, Phenylalanine 456, Lysine 458, Serine 459, Asparagine 460 and Lysine 462; or any combination thereof, is preferred by this invention.

In yet another embodiment a therapeutic antibody and/or antigen binding fragment thereof, which competes with ACE2 and/or one of the reference antibodies, and/or antigen binding fragment thereof, for binding with at least to one of amino acid to one of the epitopes selected from the group comprising 445-VGGNYNY-451, 445- VGGNYNYLYRL-455, 445-VGG-NYNYLYRLFRKS-459 and 449-YNYLYRLFRKS-459, or any combination thereof, is more preferred. In yet another embodiment a therapeutic antibody and/or antigen binding fragment, which competes with ACE2 and/or one of the reference antibodies, and/or antigen binding fragment thereof, for binding with at least to one of amino acid of the epitope 445-VGGNYNY-451 is most preferred.

In other embodiments a therapeutic antibody, and/or antigen binding fragment thereof is preferred, which does not bind to the "sweet spot", but competes with ACE2 and/or one of the reference antibodies, and/or antigen binding fragment thereof, for binding with at least to one of amino acid to one of the epitopes selected from the group comprising 342-VFNTRFASVY-351, 349-SVYAWNRKRIS-359, 485-GFNCYFP-491 and 497-FQPTNV-502, or any combination thereof.

**Properties of the therapeutic antibodies and/or fragments thereof according to the present invention**

In one embodiment the therapeutic antibody has a ACE2:RBD inhibition with 5 $\mu$g/ml IgG of between 24.0% and 102.2%. In another embodiment the therapeutic antibody and/or antigen-binding fragment thereof has a virus neutralization at 1 $\mu$g/ml IgG of between 96% and 100%. In yet another embodiment the therapeutic antibody and/or antigen-binding fragment thereof has an isoelectric point (pI) of human IgG of between 7.75 and 8.31. In yet another embodiment the therapeutic antibody specifically binds with a $K_D$ of between $1 \times 10^{-8}$ and $1 \times 10^{-10}$ to an epitope within the RBD and/or S1-subunit. In yet another embodiment the therapeutic antibody and/or antigen-binding fragment thereof has a germinality index VH of between 93.6 and 100%. In yet another embodiment the therapeutic antibody and/or antigen-binding fragment thereof has a germinality index VL of between 95.9 and 100%. In yet another embodiment the therapeutic antibody and/or antigen-binding fragment thereof has a total hydrophobicity score of between 3.41 and 6.02. In yet another embodiment the therapeutic antibody and/or antigen-binding fragment thereof has a CDR hydrophobicity score of between 0.5 and 3.14. In yet another embodiment the therapeutic antibody and/or antigen-binding fragment thereof has a CDR total charge of between -0.48 and -0.85. In yet another embodiment the therapeutic antibody and/or antigen-binding fragment thereof has a $T_{m\ onset}$ of between 57.1 °C and 65.3 °C. In yet another embodiment the therapeutic antibody and/or antigen-binding fragment thereof has a $T_{m1}$ of between 60.3 °C and 70.3 °C. In yet another embodiment the therapeutic antibody and/or antigen-binding fragment thereof has a $T_{m2}$ of between 68.0 °C and 81.9 °C. In yet another embodiment the therapeutic antibody and/or antigen-binding fragment thereof has a $T_{agg}$ of between 68.4 °C and 81.2 °C. In yet another embodiment the therapeutic antibody shows a "contamination ratio" of less than 0.2 in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In one embodiment the therapeutic antibody and/or antigen-binding fragment thereof has a ACE2:RBD inhibition with 5 $\mu$g/ml IgG of between 24.0% and 102.2% and a virus neutralization at 1 $\mu$g/ml IgG of between 96% and 100%. In yet another embodiment the therapeutic antibody and/or antigen-binding fragment thereof has an isoelectric point (pI) of human IgG of between 7.75 and 8.31, and specifically binds with a $K_D$ of between $1 \times 10^{-8}$ and $1 \times 10^{-10}$ to an epitope within the RBD and/or S1-subunit. In yet another embodiment the therapeutic antibody and/or antigen-binding fragment thereof has a germinality index VH of between 93.6 and 100% and a germinality index VL of between 95.9 and 100%. In yet another embodiment the therapeutic antibody and/or antigen-binding fragment thereof has a total hydrophobicity score of between 3.41 and 6.02, a CDR hydrophobicity score of between 0.5 and 3.14 and a CDR total charge of between -0.48 and -0.85. In yet another embodiment the therapeutic antibody and/or antigen-binding fragment thereof has a $T_{m\ onset}$ of between 57.1 °C and 65.3 °C and a $T_{m1}$ of between 60.3 °C and 70.3 °C. In yet another embodiment the therapeutic antibody and/or antigen-binding fragment thereof has a $T_{m2}$ of between 68.0 °C and 81.9 °C and a $T_{agg}$ of between 68.4 °C and 81.2 °C. In yet another embodiment the therapeutic antibody shows a "contamination ratio" of less than 0.2 in SEC-HLPC and has no unfavourable cysteines and/or glycosylations. In yet another embodiment the therapeutic antibody does not induce ADE or cytokine storm syndrome within a patient.

In one embodiment the therapeutic antibody and/or antigen-binding fragment thereof has a ACE2:RBD inhibition with 5 $\mu$g/ml IgG of between 24.0% and 102.2%, a virus neutralization at 1 $\mu$g/ml IgG of between 96% and 100%, and an isoelectric point (pI) of human IgG of between 7.75 and 8.31, and specifically binds with a $K_D$ of between $1 \times 10^{-8}$ and $1 \times 10^{-10}$ to an epitope within the RBD and/or S1-subunit. In yet another embodiment the therapeutic antibody and/or antigen-binding fragment thereof has a germinality index VH of between 93.6 and 100%, a germinality index VL of between 95.9 and 100%, a total hydrophobicity score of between 3.41 and 6.02, a CDR hydrophobicity score of between 0.5 and 3.14, and a CDR total charge of between -0.48 and -0.85. In yet another embodiment the therapeutic antibody and/or antigen-binding fragment thereof has a $T_{m\ onset}$ of between 57.1 °C and 65.3 °C, a $T_{m1}$ of between 60.3 °C and 70.3 °C, a $T_{m2}$ of between 68.0 °C and 81.9 °C, and a $T_{agg}$ of between 68.4 °C and 81.2 °C. In yet another embodiment the therapeutic antibody shows a "contamination ratio" of less than 0.2 in SEC-HLPC, has no unfavourable cysteines and/or glycosylations and does not induce ADE or cytokine storm syndrome within a patient.

In one embodiment the therapeutic antibody and/or antigen-binding fragment thereof has a ACE2:RBD inhibition with 5 $\mu$g/ml IgG of between 24.0% and 102.2%, a virus neutralization at 1 $\mu$g/ml IgG of between 96% and 100%, an isoelectric point (pI) of human IgG of between 7.75 and 8.31, specifically binds with a $K_D$ of between $1 \times 10^{-8}$ and $1 \times 10^{-10}$ to an epitope within the RBD and/or S1-subunit, has a germinality index VH of between 93.6 and 100%, a germinality index VL of between 95.9 and 100%, a total hydrophobicity score of between 3.41 and 6.02, a CDR hydrophobicity score of between 0.5 and 3.14, a CDR total charge of between -0.48 and - 0.85, a $T_{m\ onset}$ of between 57.1 °C and 65.3 °C,

a $T_{m1}$ of between 60.3 °C and 70.3 °C, a $T_{m2}$ of between 68.0 °C and 81.9 °C, and a $T_{agg}$ of between 68.4 °C and 81.2 °C, shows a "contamination ratio" of less than 0.2 in SEC-HLPC, has no unfavourable cysteines and/or glycosylations and does not induce ADE or cytokine storm syndrome within a patient.

Properties of the therapeutic antibodies and/or fragments thereof according to the present invention

In one embodiment the therapeutic antibody has a ACE2:RBD inhibition with 5 µg/ml IgG of about 102.2%. In another embodiment the therapeutic antibody and/or antigen-binding fragment thereof has a virus neutralization at 1 µg/ml IgG of at least 99%. In yet another embodiment the therapeutic antibody and/or antigen-binding fragment thereof has an isoelectric point (pI) of human IgG of about 8.11. In yet another embodiment the therapeutic antibody specifically binds with a $K_D$ of 3.41 x 10$^{-9}$ to an epitope within the RBD and/or S1-subunit. In yet another embodiment the therapeutic antibody and/or antigen-binding fragment thereof has a germinality index VH of about 100%. In yet another embodiment the therapeutic antibody and/or antigen-binding fragment thereof has a germinality index VL of about 98%. In yet another embodiment the therapeutic antibody and/or antigen-binding fragment thereof has a total hydrophobicity score of about 3.41. In yet another embodiment the therapeutic antibody and/or antigen-binding fragment thereof has a CDR hydrophobicity score of about 0.5. In yet another embodiment the therapeutic antibody and/or antigen-binding fragment thereof has a CDR total charge of about - 0.58. In yet another embodiment the therapeutic antibody and/or antigen-binding fragment thereof has a $T_{m\ onset}$ of at least 61.6 °C. In yet another embodiment the therapeutic antibody and/or antigen-binding fragment thereof has a $T_{m1}$ of at least 68.8 °C. In yet another embodiment the therapeutic antibody and/or antigen-binding fragment thereof has a $T_{m2}$ of at least 81.9 °C. In yet another embodiment the therapeutic antibody and/or antigen-binding fragment thereof has a $T_{agg}$ of at least 71.3 °C. In yet another embodiment the therapeutic antibody shows no multimer or dimer peak in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In yet another embodiment the therapeutic antibody and/or antigen-binding fragment thereof has a ACE2:RBD inhibition with 5 µg/ml IgG of about 102.2% and a virus neutralization at 1 µg/ml IgG of at least 99%. In yet another embodiment the therapeutic antibody and/or antigen-binding fragment thereof has an isoelectric point (pI) of human IgG of about 8.11 and specifically binds with a $K_D$ of 3.41 x 10$^{-9}$ to an epitope within the RBD and/or S1-subunit. In yet another embodiment the therapeutic antibody and/or antigen-binding fragment thereof has a germinality index VH of about 100% and a germinality index VL of about 98%. In yet another embodiment the therapeutic antibody and/or antigen-binding fragment thereof has a total hydrophobicity score of about 3.41 and a CDR hydrophobicity score of about 0.5 and a CDR total charge of about -0.58. In yet another embodiment the therapeutic antibody and/or antigen-binding fragment thereof has a $T_{m\ onset}$ of at least 61.6 °C, a $T_{m1}$ of at least 68.8 °C, a $T_{m2}$ of at least 81.9 °C and a $T_{agg}$ of at least 71.3 °C. In yet another embodiment the therapeutic antibody shows no multimer or dimer peak in SEC-HLPC, no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 µg/ml IgG of about 102.2%, a virus neutralization at 1 µg/ml IgG of at least 99% and an isoelectric point (pI) of human IgG of about 8.11, and specifically binds with a $K_D$ of 3.41 x 10$^{-9}$ to an epitope within the RBD and/or S1-subunit. In yet another embodiment the therapeutic antibody and/or antigen-binding fragment thereof has a germinality index VH of about 100%, a germinality index VL of about 98%, a total hydrophobicity score of about 3.41 and a CDR hydrophobicity score of about 0.5 and a CDR total charge of about -0.58. In yet another embodiment the therapeutic antibody and/or antigen-binding fragment thereof has a $T_{m\ onset}$ of at least 61.6 °C, a $T_{m1}$ of at least 68.8 °C, a $T_{m2}$ of at least 81.9 °C and a $T_{agg}$ of at least 71.3 °C, and shows no multimer or dimer peak in SEC-HLPC, no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 µg/ml IgG of about 102.2%, a virus neutralization at 1 µg/ml IgG of at least 99%, an isoelectric point (pI) of human IgG of about 8.11, specifically binds with a $K_D$ of 3.41 x 10$^{-9}$ to an epitope within the RBD and/or S1-subunit, has a germinality index VH of about 100%, a germinality index VL of about 98%, a total hydrophobicity score of about 3.41, a CDR hydrophobicity score of about 0.5, a CDR total charge of about -0.58, a $T_{m\ onset}$ of at least 61.6 °C, a $T_{m1}$ of at least 68.8 °C, a $T_{m2}$ of at least 81.9 °C and a $T_{agg}$ of at least 71.3 °C, shows no multimer or dimer peak in SEC-HLPC, no unfavourable cysteines and/or glycosylations and does not induce ADE or cytokine storm syndrome within a patient.

In one embodiment the therapeutic antibody has a ACE2:RBD inhibition with 5 µg/ml IgG of about 43.2%. In another embodiment the therapeutic antibody and/or antigen-binding fragment thereof has a virus neutralization at 1 µg/ml IgG of at least 100%. In yet another embodiment the therapeutic antibody and/or antigen-binding fragment thereof has an isoelectric point (pI) of human IgG of about 7.75. In yet another embodiment the therapeutic antibody specifically binds with a $K_D$ of 1.07 x 10$^{-8}$ to an epitope within the RBD and/or S1-subunit. In yet another embodiment the therapeutic antibody and/or antigen-binding fragment thereof has a germinality index VH of about 99.1%. In yet another embodiment the therapeutic antibody and/or antigen-binding fragment thereof has a germinality index VL of about 100%. In yet another embodiment the therapeutic antibody and/or antigen-binding fragment thereof has a total hydrophobicity score of about 6.02. In yet another embodiment the therapeutic antibody and/or antigen-binding fragment thereof has a CDR hydrophobicity score of about 3.14. In yet another embodiment the therapeutic antibody and/or antigen-binding fragment thereof has a CDR total charge of about - 0.63. In yet another embodiment the therapeutic antibody and/or antigen-binding fragment thereof has a $T_{m \, onset}$ of at least 60.1 °C. In yet another embodiment the therapeutic antibody and/or antigen-binding fragment thereof has a $T_{m1}$ of at least 70.0 °C. In yet another embodiment the therapeutic antibody and/or antigen-binding fragment thereof has a $T_{m2}$ of at least 79.7 °C. In yet another embodiment the therapeutic antibody and/or antigen-binding fragment thereof has a $T_{agg}$ of at least 78.6 °C. In yet another embodiment the therapeutic antibody shows no multimer or dimer peak in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In yet another embodiment the therapeutic antibody and/or antigen-binding fragment thereof has a ACE2:RBD inhibition with 5 μg/ml IgG of about 43.2% and a virus neutralization at 1 μg/ml IgG of at least 100%. In yet another embodiment the therapeutic antibody and/or antigen-binding fragment thereof has an isoelectric point (pI) of human IgG of about 7.75 and specifically binds with a $K_D$ of 1.07 x $10^{-8}$ to an epitope within the RBD and/or S1-subunit. In yet another embodiment the therapeutic antibody and/or antigen-binding fragment thereof has a germinality index VH of about 99.1% and a germinality index VL of about 100%. In yet another embodiment the therapeutic antibody and/or antigen-binding fragment thereof has a total hydrophobicity score of about 6.02 and a CDR hydrophobicity score of about 3.14 and a CDR total charge of about -0.63. In yet another embodiment the therapeutic antibody and/or antigen-binding fragment thereof has a $T_{m \, onset}$ of at least 60.1 °C and a $T_{m1}$ of at least 70.0 °C. In yet another embodiment the therapeutic antibody and/or antigen-binding fragment thereof has a $T_{m2}$ of at least 79.7 °C and a $T_{agg}$ of at least 78.6 °C. In yet another embodiment the therapeutic antibody shows a "contamination ratio" of less than 0.2 in SEC-HLPC and has no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 μg/ml IgG of about 43.2%, a virus neutralization at 1 μg/ml IgG of at least 100%, an isoelectric point (pI) of human IgG of about 7.75 and specifically binds with a $K_D$ of 1.07 x $10^{-8}$ to an epitope within the RBD and/or S1-subunit. In yet another embodiment the therapeutic antibody and/or antigen-binding fragment thereof has a germinality index VH of about 99.1%, a germinality index VL of about 100%, a total hydrophobicity score of about 6.02, a CDR hydrophobicity score of about 3.14 and a CDR total charge of about -0.63. In yet another embodiment the therapeutic antibody and/or antigen-binding fragment thereof has a $T_{m \, onset}$ of at least 60.1 °C, a $T_{m1}$ of at least 70.0 °C, a $T_{m2}$ of at least 79.7 °C and a $T_{agg}$ of at least 78.6 °C, and shows a "contamination ratio" of less than 0.2 in SEC-HLPC and has no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 μg/ml IgG of about 43.2%, a virus neutralization at 1 μg/ml IgG of at least 100%, an isoelectric point (pI) of human IgG of about 7.75, specifically binds with a $K_D$ of 1.07 x $10^{-8}$ to an epitope within the RBD and/or S1-subunit, has a germinality index VH of about 99.1%, a germinality index VL of about 100%, a total hydrophobicity score of about 6.02, a CDR hydrophobicity score of about 3.14 and a CDR total charge of about -0.63, a $T_{m \, onset}$ of at least 60.1 °C, a $T_{m1}$ of at least 70.0 °C, a $T_{m2}$ of at least 79.7 °C, a $T_{agg}$ of at least 78.6 °C, shows a "contamination ratio" of less than 0.2 in SEC-HLPC, has no unfavourable cysteines and/or glycosylations and does not induce ADE or cytokine storm syndrome within a patient.

In one embodiment the therapeutic antibody has a ACE2:RBD inhibition with 5 μg/ml IgG of about 24.0%. In another embodiment the therapeutic antibody and/or antigen-binding fragment thereof has a virus neutralization at 1 μg/ml IgG of at least 97%. In yet another embodiment the therapeutic antibody and/or antigen-binding fragment thereof has an isoelectric point (pI) of human IgG of about 7.88. In yet another embodiment the therapeutic antibody specifically binds with a $K_D$ of 4.84 x $10^{-9}$ to an epitope within the RBD and/or S1-subunit. In yet another embodiment the therapeutic antibody and/or antigen-binding fragment thereof has a germinality index VH of about 93.6%. In yet another embodiment the therapeutic antibody and/or antigen-binding fragment thereof has a germinality index VL of about 97%. In yet another embodiment the therapeutic antibody and/or antigen-binding fragment thereof has a total hydrophobicity score of about 5.18. In yet another embodiment the therapeutic antibody and/or antigen-binding fragment thereof has a CDR hydrophobicity score of about 1.86. In yet another embodiment the therapeutic antibody and/or antigen-binding fragment thereof has a CDR total charge of about - 0.85. In yet another embodiment the therapeutic antibody and/or antigen-binding fragment thereof has a $T_{m \, onset}$ of at least 57.1 °C. In yet another embodiment the therapeutic antibody and/or antigen-binding fragment thereof has a $T_{m1}$ of at least 60.3 °C. In yet another embodiment the therapeutic antibody and/or antigen-binding fragment thereof has a $T_{m2}$ of at least 68.2 °C. In yet another embodiment the therapeutic antibody and/or antigen-binding fragment thereof has a $T_{agg}$ of at least 68.4 °C. In yet another embodiment the therapeutic antibody shows no multimer or dimer peak in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In yet another embodiment the therapeutic antibody and/or antigen-binding fragment thereof has a ACE2:RBD inhibition with 5 $\mu$g/ml IgG of about 24.0% and a virus neutralization at 1 $\mu$g/ml IgG of at least 97%. In yet another embodiment the therapeutic antibody and/or antigen-binding fragment thereof has an isoelectric point (pI) of human IgG of about 7.88 and specifically binds with a $K_D$ of 4.84 x $10^{-9}$ to an epitope within the RBD and/or S1-subunit. In yet another embodiment the therapeutic antibody and/or antigen-binding fragment thereof has a germinality index VH of about 93.6% and a germinality index VL of about 97%. In yet another embodiment the therapeutic antibody and/or antigen-binding fragment thereof has a total hydrophobicity score of about 5.18, a CDR hydrophobicity score of about 1.86 and a CDR total charge of about -0.85. In yet another embodiment the therapeutic antibody and/or antigen-binding fragment thereof has a $T_{m\,onset}$ of at least 57.1 °C and a $T_{m1}$ of at least 60.3 °C. In yet another embodiment the therapeutic antibody and/or antigen-binding fragment thereof has a $T_{m2}$ of at least 68.2 °C and a $T_{agg}$ of at least 68.4 °C. In yet another embodiment the therapeutic antibody shows no multimer or dimer peak in SEC-HLPC, has no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 $\mu$g/ml IgG of about 24.0%, a virus neutralization at 1 $\mu$g/ml IgG of at least 97%, an isoelectric point (pI) of human IgG of about 7.88 and specifically binds with a $K_D$ of 4.84 x $10^{-9}$ to an epitope within the RBD and/or S1-subunit. In yet another embodiment the therapeutic antibody and/or antigen-binding fragment thereof has a germinality index VH of about 93.6%, a germinality index VL of about 97%, a total hydrophobicity score of about 5.18, a CDR hydrophobicity score of about 1.86 and a CDR total charge of about -0.85. In yet another embodiment the therapeutic antibody and/or antigen-binding fragment thereof has a $T_{m\,onset}$ of at least 57.1 °C, a $T_{m1}$ of at least 60.3 °C, a $T_{m2}$ of at least 68.2 °C and a $T_{agg}$ of at least 68.4 °C, and shows no multimer or dimer peak in SEC-HLPC, has no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 $\mu$g/ml IgG of about 24.0%, a virus neutralization at 1 $\mu$g/ml IgG of at least 97%, an isoelectric point (pI) of human IgG of about 7.88, specifically binds with a $K_D$ of 4.84 x $10^{-9}$ to an epitope within the RBD and/or S1-subunit, has a germinality index VH of about 93.6%, a germinality index VL of about 97%, a total hydrophobicity score of about 5.18, a CDR hydrophobicity score of about 1.86, a CDR total charge of about -0.85, a $T_{m\,onset}$ of at least 57.1 °C, a $T_{m1}$ of at least 60.3 °C, a $T_{m2}$ of at least 68.2 °C, a $T_{agg}$ of at least 68.4 °C, and shows no multimer or dimer peak in SEC-HLPC, has no unfavourable cysteines and/or glycosylations and does not induce ADE or cytokine storm syndrome within a patient.

In one embodiment the therapeutic antibody has a ACE2:RBD inhibition with 5 $\mu$g/ml IgG of about 26.7%. In another embodiment the therapeutic antibody and/or antigen-binding fragment thereof has a virus neutralization at 1 $\mu$g/ml IgG of at least 96%. In yet another embodiment the therapeutic antibody and/or antigen-binding fragment thereof has an isoelectric point (pI) of human IgG of about 8.31. In yet another embodiment the therapeutic antibody specifically binds with a $K_D$ of 1.31 x $10^{-8}$ to an epitope within the RBD and/or S1-subunit. In yet another embodiment the therapeutic antibody and/or antigen-binding fragment thereof has a germinality index VH of about 93.6%. In yet another embodiment the therapeutic antibody and/or antigen-binding fragment thereof has a germinality index VL of about 95.9%. In yet another embodiment the therapeutic antibody and/or antigen-binding fragment thereof has a total hydrophobicity score of about 4.5. In yet another embodiment the therapeutic antibody and/or antigen-binding fragment thereof has a CDR hydrophobicity score of about 0.44. In yet another embodiment the therapeutic antibody and/or antigen-binding fragment thereof has a CDR total charge of about -0.48. In yet another embodiment the therapeutic antibody and/or antigen-binding fragment thereof has a $T_{m\,onset}$ of at least 57.3 °C. In yet another embodiment the therapeutic antibody and/or antigen-binding fragment thereof has a $T_{m1}$ of at least 64.5 °C. In yet another embodiment the therapeutic antibody and/or antigen-binding fragment thereof has a $T_{m2}$ of at least 81.6 °C. In yet another embodiment the therapeutic antibody and/or antigen-binding fragment thereof has a $T_{agg}$ of at least 69.5 °C. In yet another embodiment the therapeutic antibody shows a "contamination ratio" of less than 0.2 in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In yet another embodiment the therapeutic antibody and/or antigen-binding fragment thereof has a ACE2:RBD inhibition with 5 $\mu$g/ml IgG of about 26.7% and a virus neutralization at 1 $\mu$g/ml IgG of at least 96%. In yet another embodiment the therapeutic antibody and/or antigen-binding fragment thereof has an isoelectric point (pI) of human IgG of about 8.31 and specifically binds with a $K_D$ of 1.31 x $10^{-8}$ to an epitope within the RBD and/or S1-subunit. In yet another embodiment the therapeutic antibody and/or antigen-binding fragment thereof has a germinality index VH of about 93.6% and a germinality index VL of about 95.9%. In yet another embodiment the therapeutic antibody and/or antigen-binding fragment thereof has a total hydrophobicity score of about 4.5, a CDR hydrophobicity score of about 0.44 and a CDR total charge of about -0.48. In yet another embodiment the therapeutic antibody and/or antigen-binding fragment thereof has a $T_{m\,onset}$ of at least 57.3 °C and a $T_{m1}$ of at least 64.5 °C. In yet another embodiment the therapeutic antibody and/or antigen-binding fragment thereof has a $T_{m2}$ of at least 81.6 °C and a $T_{agg}$ of at least 69.5 °C. In yet another embodiment the therapeutic antibody shows a "contamination ratio" of less than 0.2 in SEC-HLPC, has no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 $\mu$g/ml IgG of about 26.7%, a virus neutralization at 1 $\mu$g/ml IgG of at least 96%, an isoelectric point (pI) of human IgG of about 8.31 and specifically binds with a $K_D$ of 1.31 x 10$^{-8}$ to an epitope within the RBD and/or S1-subunit. In yet another embodiment the therapeutic antibody and/or antigen-binding fragment thereof has a germinality index VH of about 93.6%, a germinality index VL of about 95.9%, a total hydrophobicity score of about 4.5, a CDR hydrophobicity score of about 0.44 and a CDR total charge of about -0.48. In yet another embodiment the therapeutic antibody and/or antigen-binding fragment thereof has a $T_{m\ onset}$ of at least 57.3 °C, a $T_{m1}$ of at least 64.5 °C, a $T_{m2}$ of at least 81.6 °C and a $T_{agg}$ of at least 69.5 °C, shows a "contamination ratio" of less than 0.2 in SEC-HLPC, has no unfavourable cysteines and/or glycosylations and does not induce ADE or cytokine storm syndrome within a patient.

In yet another embodiment the therapeutic antibody and/or antigen-binding fragment thereof has a ACE2:RBD inhibition with 5 $\mu$g/ml IgG of about 26.7% and a virus neutralization at 1 $\mu$g/ml IgG of at least 96%, has an isoelectric point (pI) of human IgG of about 8.31, specifically binds with a $K_D$ of 1.31 x 10$^{-8}$ to an epitope within the RBD and/or S1-subunit. has a germinality index VH of about 93.6%, a germinality index VL of about 95.9%, a total hydrophobicity score of about 4.5, a CDR hydrophobicity score of about 0.44, a CDR total charge of about -0.48, a $T_{m\ onset}$ of at least 57.3 °C, a $T_{m1}$ of at least 64.5 °C, a $T_{m2}$ of at least 81.6 °C, a $T_{agg}$ of at least 69.5 °C and shows a "contamination ratio" of less than 0.2 in SEC-HLPC, has no unfavourable cysteines and/or glycosylations and does not induce ADE or cytokine storm syndrome within a patient.

In one embodiment the therapeutic antibodies show a $T_{m\ onset}$ above 60°C, more preferred above 65°C, most preferred above 70°C. In another embodiment the therapeutic antibodies show a $T_{m1}$ above 60°C, more preferred above 65°C, most preferred above 70°C. In another embodiment the therapeutic antibodies show a $T_{m2}$ above 70°C, more preferred above 75°C, most preferred above 80°C. In another embodiment the therapeutic antibodies show a $T_{agg}$ above 70°C, more preferred above 75°C, most preferred above 80°C.

In one embodiment the therapeutic antibody has a ACE2:RBD inhibition of the scFV of 87%. In yet another embodiment the therapeutic antibody and/or antigen-binding fragment thereof has an isoelectric point (pI) of human IgG of 8.11. In yet another embodiment the therapeutic antibody and/or antigen-binding fragment thereof has a germinality index VH of 96.3%. In yet another embodiment the therapeutic antibody and/or antigen-binding fragment thereof has a germinality index VL of 99%. In yet another embodiment the therapeutic antibody and/or antigen-binding fragment thereof has a total hydrophobicity score of 4.64. In yet another embodiment the therapeutic antibody and/or antigen-binding fragment thereof has a CDR hydrophobicity score of 1.29. In yet another embodiment the therapeutic antibody and/or antigen-binding fragment thereof has an scFv charge of 0.82. In yet another embodiment the therapeutic antibody and/or antigen-binding fragment thereof has a CDR total charge of -0.19. In yet another embodiment the therapeutic antibody and/or antigen-binding fragment thereof has a $T_{m\ onset}$ of about 63.7 °C. In yet another embodiment the therapeutic antibody and/or antigen-binding fragment thereof has a $T_{m1}$ of about 70.8 °C. In yet another embodiment the therapeutic antibody and/or antigen-binding fragment thereof has a $T_{agg}$ of about 71.7 °C. In yet another embodiment the therapeutic antibody shows a "contamination ratio" of less than 0.2 in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In one embodiment the therapeutic antibody and/or antigen-binding fragment thereof has a ACE2:RBD inhibition of the scFV of 87% and has an isoelectric point (pI) of human IgG of 8.11. In yet another embodiment the therapeutic antibody and/or antigen-binding fragment thereof has a germinality index VH of 96.3% and a germinality index VL of 99%. In yet another embodiment the therapeutic antibody and/or antigen-binding fragment thereof has a total hydrophobicity score of 4.64 and a CDR hydrophobicity score of 1.29. In yet another embodiment the therapeutic antibody and/or antigen-binding fragment thereof has an scFv charge of 0.82 and a CDR total charge of -0.19. In yet another embodiment the therapeutic antibody and/or antigen-binding fragment thereof has a $T_{m\ onset}$ of about 63.7 °C, a $T_{m1}$ of about 70.8 °C and a $T_{agg}$ of about 71.7 °C. In yet another embodiment the therapeutic antibody shows a "contamination ratio" of less than 0.2 in SEC-HLPC and no unfavourable cysteines and/or glycosylations. In yet another embodiment the therapeutic antibody does not induce ADE or cytokine storm syndrome within a patient.

In one embodiment the therapeutic antibody and/or antigen-binding fragment thereof has a ACE2:RBD inhibition of the scFV of 87%, an isoelectric point (pI) of human IgG of 8.11, a germinality index VH of 96.3% and a germinality index VL of 99%. In yet another embodiment the therapeutic antibody and/or antigen-binding fragment thereof has a total hydrophobicity score of 4.64, a CDR hydrophobicity score of 1.29, an scFv charge of 0.82 and a CDR total charge of - 0.19. In yet another embodiment the therapeutic antibody and/or antigen-binding fragment thereof has a $T_{m\ onset}$ of about 63.7 °C, a $T_{m1}$ of about 70.8 °C and a $T_{agg}$ of about 71.7 °C, shows a "contamination ratio" of less than 0.2 in SEC-HLPC, no unfavourable cysteines and/or glycosylations and does not induce ADE or cytokine storm syndrome within a patient.

In one embodiment the therapeutic antibody and/or antigen-binding fragment thereof has a ACE2:RBD inhibition of the scFV of 87%, an isoelectric point (pI) of human IgG of 8.11, a germinality index VH of 96.3%, a germinality index VL of 99%, a total hydrophobicity score of 4.64, a CDR hydrophobicity score of 1.29, an scFv charge of 0.82, a CDR total charge of -0.19, a $T_{m\,onset}$ of about 63.7 °C, a $T_{m1}$ of about 70.8 °C, a $T_{agg}$ of about 71.7 °C, shows a "contamination ratio" of less than 0.2 in SEC-HLPC, no unfavourable cysteines and/or glycosylations and does not induce ADE or cytokine storm syndrome within a patient.

In one embodiment the therapeutic antibody has a ACE2:RBD inhibition of the scFV of 27%. In yet another embodiment the therapeutic antibody and/or antigen-binding fragment thereof has an isoelectric point (pI) of human IgG of 8.11. In yet another embodiment the therapeutic antibody and/or antigen-binding fragment thereof has a germinality index VH of 84.4.3%. In yet another embodiment the therapeutic antibody and/or antigen-binding fragment thereof has a germinality index VL of 100%. In yet another embodiment the therapeutic antibody and/or antigen-binding fragment thereof has a total hydrophobicity score of 5.08. In yet another embodiment the therapeutic antibody and/or antigen-binding fragment thereof has a CDR hydrophobicity score of 2.09. In yet another embodiment the therapeutic antibody and/or antigen-binding fragment thereof has an scFv charge of 0.18. In yet another embodiment the therapeutic antibody and/or antigen-binding fragment thereof has a CDR total charge of -0.83. In yet another embodiment the therapeutic antibody shows a "contamination ratio" of less than 0.2 in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In one embodiment the therapeutic antibody and/or antigen-binding fragment thereof has a ACE2:RBD inhibition of the scFV of 27% and an isoelectric point (pI) of human IgG of 8.11. In yet another embodiment the therapeutic antibody and/or antigen-binding fragment thereof has a germinality index VH of 84.4.3% and a germinality index VL of 100%. In yet another embodiment the therapeutic antibody and/or antigen-binding fragment thereof has a total hydrophobicity score of 5.08 and a CDR hydrophobicity score of 2.09. In yet another embodiment the therapeutic antibody and/or antigen-binding fragment thereof has an scFv charge of 0.18 and a CDR total charge of -0.83. In yet another embodiment the therapeutic antibody shows a "contamination ratio" of less than 0.2 in SEC-HLPC and no unfavourable cysteines and/or glycosylations. In yet another embodiment the therapeutic antibody does not induce ADE or cytokine storm syndrome within a patient.

In one embodiment the therapeutic antibody and/or antigen-binding fragment thereof has a ACE2:RBD inhibition of the scFV of 27%, an isoelectric point (pI) of human IgG of 8.11, a germinality index VH of 84.4.3% and a germinality index VL of 100%. In yet another embodiment the therapeutic antibody and/or antigen-binding fragment thereof has a total hydrophobicity score of 5.08, a CDR hydrophobicity score of 2.09, an scFv charge of 0.18 and a CDR total charge of - 0.83. In yet another embodiment the therapeutic antibody shows a "contamination ratio" of less than 0.2 in SEC-HLPC, no unfavourable cysteines and/or glycosylations and does not induce ADE or cytokine storm syndrome within a patient.

In one embodiment the therapeutic antibody and/or antigen-binding fragment thereof has a ACE2:RBD inhibition of the scFV of 27%, an isoelectric point (pI) of human IgG of 8.11, a germinality index VH of 84.4.3%, a germinality index VL of 100%, a total hydrophobicity score of 5.08, a CDR hydrophobicity score of 2.09, an scFv charge of 0.18, a CDR total charge of -0.83, shows a "contamination ratio" of less than 0.2 in SEC-HLPC, no unfavourable cysteines and/or glycosylations and does not induce ADE or cytokine storm syndrome within a patient.

In one embodiment the therapeutic antibody has a ACE2:RBD inhibition of the scFV of 49%. In yet another embodiment the therapeutic antibody and/or antigen-binding fragment thereof has an isoelectric point (pI) of human IgG of 8.39. In yet another embodiment the therapeutic antibody and/or antigen-binding fragment thereof has a germinality index VH of 95.4%. In yet another embodiment the therapeutic antibody and/or antigen-binding fragment thereof has a germinality index VL of 96.9%. In yet another embodiment the therapeutic antibody and/or antigen-binding fragment thereof has a total hydrophobicity score of 4.37. In yet another embodiment the therapeutic antibody and/or antigen-binding fragment thereof has a CDR hydrophobicity score of 1.45. In yet another embodiment the therapeutic antibody and/or antigen-binding fragment thereof has an scFv charge of 1.22. In yet another embodiment the therapeutic antibody and/or antigen-binding fragment thereof has a CDR total charge of -0.25. In yet another embodiment the therapeutic antibody and/or antigen-binding fragment thereof has a $T_{m\,onset}$ of about 62.4 °C. In yet another embodiment the therapeutic antibody and/or antigen-binding fragment thereof has a $T_{m1}$ of about 69.3 °C. In yet another embodiment the therapeutic antibody and/or antigen-binding fragment thereof has a $T_{m2}$ of about 81.4 °C. In yet another embodiment the therapeutic antibody and/or antigen-binding fragment thereof has a $T_{agg}$ of about 69.9 °C. In yet another embodiment the therapeutic antibody shows a "contamination ratio" of less than 0.2 in SEC-HLPC. In yet another embodiment the therapeutic antibody and/or antigen-binding fragment thereof has no unfavourable cysteines and/or glycosylations. In yet another embodiment the therapeutic antibody does not induce ADE or cytokine storm syndrome within a patient.

In one embodiment the therapeutic antibody and/or antigen-binding fragment thereof has a ACE2:RBD inhibition of the scFV of 49% and an isoelectric point (pI) of human IgG of 8.39. In yet another embodiment the therapeutic antibody and/or antigen-binding fragment thereof has a germinality index VH of 95.4% and a germinality index VL of 96.9%. In yet another embodiment the therapeutic antibody and/or antigen-binding fragment thereof has a total hydrophobicity score of 4.37 and a CDR hydrophobicity score of 1.45. In yet another embodiment the therapeutic antibody and/or antigen-binding fragment thereof has an scFv charge of 1.22 and a CDR total charge of -0.25. In yet another embodiment the therapeutic antibody and/or antigen-binding fragment thereof has a $T_{m\,onset}$ of about 62.4 °C and a $T_{m1}$ of about 69.3 °C. In yet another embodiment the therapeutic antibody and/or antigen-binding fragment thereof has a $T_{m2}$ of about 81.4 °C and a $T_{agg}$ of about 69.9 °C. In yet another embodiment the therapeutic antibody shows a "contamination ratio" of less than 0.2 in SEC-HLPC. In yet another embodiment the therapeutic antibody and/or antigen-binding fragment thereof has no unfavourable cysteines and/or glycosylations and does not induce ADE or cytokine storm syndrome within a patient.

In one embodiment the therapeutic antibody and/or antigen-binding fragment thereof has a ACE2:RBD inhibition of the scFV of 49%, an isoelectric point (pI) of human IgG of 8.39, a germinality index VL of 96.9%, a total hydrophobicity score of 4.37, a CDR hydrophobicity score of 1.45, an scFv charge of 1.22, a CDR total charge of -0.25, a $T_{m\,onset}$ of about 62.4 °C and a $T_{m1}$ of about 69.3 °C. In yet another embodiment the therapeutic antibody and/or antigen-binding fragment thereof has a $T_{m2}$ of about 81.4 °C, a $T_{agg}$ of about 69.9 °C, a "contamination ratio" of less than 0.2 in SEC-HLPC, no unfavourable cysteines and/or glycosylations and does not induce ADE or cytokine storm syndrome within a patient.

In one embodiment the therapeutic antibody and/or antigen-binding fragment thereof has a ACE2:RBD inhibition of the scFV of 49%, an isoelectric point (pI) of human IgG of 8.39, a germinality index VL of 96.9%, a total hydrophobicity score of 4.37, a CDR hydrophobicity score of 1.45, an scFv charge of 1.22, a CDR total charge of -0.25, a $T_{m\,onset}$ of about 62.4 °C, a $T_{m1}$ of about 69.3 °C, a $T_{m2}$ of about 81.4 °C, a $T_{agg}$ of about 69.9 °C, a "contamination ratio" of less than 0.2 in SEC-HLPC, no unfavourable cysteines and/or glycosylations and does not induce ADE or cytokine storm syndrome within a patient.

In one embodiment the therapeutic antibody and/or antigen-binding fragment thereof has a $T_{m\,onset}$ of 61.6 ± 0.1 °C and/or a $T_{m1}$ of 68.8 ± 0.1 °C, and/or a $T_{m2}$ of 81.9 ± 0.1 °C, and/or a $T_{agg}$ of 71.3 ± 0.1 °C.

In another embodiment the therapeutic antibody and/or antigen-binding fragment thereof has a $T_{m\,onset}$ of 60.1 ± 0.2 °C and/or a $T_{m1}$ of 70.0 ± 0.2 °C, and/or a $T_{m2}$ of 79.7 ± 0.1 °C, and/or a $T_{agg}$ of 78.6 ± 0.2 °C.

In yet another embodiment the therapeutic antibody and/or antigen-binding fragment thereof has a $T_{m\,onset}$ of 57.1 ± 0.3 °C and/or a $T_{m1}$ of 60.3 ± 0.0 °C, and/or a $T_{m2}$ of 68.2 ± 0.1 °C, and/or a $T_{agg}$ of 68.4 ± 0.1 °C.

In yet another embodiment the therapeutic antibody and/or antigen-binding fragment thereof has a $T_{m\,onset}$ of 57.3 ± 0.3 °C and/or a $T_{m1}$ of 64.5 ± 0.0 °C, and/or a $T_{m2}$ of 81.6 ± 0.1 °C, and/or a $T_{agg}$ of 69.5 ± 0.3 °C.

In another embodiment the therapeutic antibody and/or antigen-binding fragment thereof has a $T_{m\,onset}$ of 65.3 ± 0.8 °C and/or a $T_{m1}$ of 69.5 ± 0.0 °C, and/or a $T_{m2}$ of 81.4 ± 0.7 °C, and/or a $T_{agg}$ of 71.2 ± 0.0 °C.

In yet another embodiment the therapeutic antibody and/or antigen-binding fragment thereof has a $T_{m\,onset}$ of 62.4 ± 0.1 °C and/or a $T_{m1}$ of 68.9 ± 0.0 °C, and/or a $T_{m2}$ of 81.4 ± 0.1 °C, and/or a $T_{agg}$ of 69.9 ± 0.0 °C.

In yet another embodiment the therapeutic antibody and/or antigen-binding fragment thereof has a $T_{m\,onset}$ of 62.2 ± 0.1 °C and/or a $T_{m1}$ of 70.8 ± 0.2 °C, and/or a $T_{m2}$ of 81.9 ± 0.2 °C, and/or a $T_{agg}$ of 75.9 ± 0.0 °C.

In another embodiment the therapeutic antibody and/or antigen-binding fragment thereof has a $T_{m\,onset}$ of 62.4 ± 0.0 °C and/or a $T_{m1}$ of 69.3 ± 0.1 °C, and/or a $T_{m2}$ of 80.9 ± 0.0 °C, and/or a $T_{agg}$ of 81.2 ± 0.3 °C.

In yet another embodiment the therapeutic antibody and/or antigen-binding fragment thereof has a $T_{m\,onset}$ of 56.9 ± 0.2 °C and/or a $T_{m1}$ of 62.1 ± 0.2 °C, and/or a $T_{m2}$ of 68.0 ± 0.2 °C, and/or a $T_{agg}$ of 70.1 ± 0.1 °C.

In yet another embodiment the therapeutic antibody and/or antigen-binding fragment thereof has a $T_{m\,onset}$ of 63.7 ± 0.0 °C and/or a $T_{m1}$ of 70.0 ± 0.2 °C, and/or a $T_{agg}$ of 71.7 ± 0.2 °C. A further aspect, the present invention relates to a combination comprising at least 2, 3, 4, 5, 6 or more of the above monoclonal antibodies, particularly monoclonal antibodies or antigen-binding fragments thereof. In such a combination, the individual antibodies or fragments are present in suitable molar ratios. Typically, the molar ratios are in the range of about 1:5 to 5:1, particularly about 1:2 to 2:1. In certain embodiments, the antibody combination may comprise a single composition wherein the antibodies and antibody fragments in said composition consist of a predetermined number of different species of monoclonal antibodies or antibody fragments as described above.

The antibody combination may comprise a plurality of compositions each comprising a different species of monoclonal antibody or antibody fragment as described above. The combination of the present invention may be free from other antibodies or antibody fragments, particularly from polyclonal antibodies or antibody fragments.

In certain embodiments, the combination of antibodies or antigen-binding fragments has an $IC_{50}$ value of about 20 nM or less, of about 10 nM or less, or of about 5 nM or less, particularly when determined in molar ratios of 1:1 of the individual antibodies or antibody fragments as described in the present examples.

In certain embodiments, the combination of antibodies or antigen-binding fragments has an $EC_{50}$ value of about 20 nM or less, of about 10 nM or less, or of about 5 nM or less, particularly when determined in molar ratios of 1:1 of the individual antibodies or antibody fragments as described in the present examples.

In particular preferred are combinations of at least two antibodies selected from the group consisting of antibodies STE90-C11, YU505-A02, YU536-D04 and YU537-H11.

In a further embodiment, the combination is selected from the group consisting of antibodies YU534-C09 and YU534-C12, YU534-C09 and YU534-D09, YU534-C09 and YU535-A02, YU534-C09 and YU536-D04, YU534-C09 and YU537-H08, YU534-C09 and YU537-H11, YU534-C09 and STE90-C11, YU534-C12 and YU534-D09, YU534-C12 and YU535-A02, YU534-C12 and YU536-D04, YU534-C12 and YU537-H08, YU534-C12 and YU537-H11, YU534-C12 and STE90-C11, YU534-D09 and YU535-A02, YU534-D09 and YU536-D04, YU534-D09 and YU537-H08, YU534-D09 and YU537-H11, YU534-D09 and STE90-C11, YU536-D04 and YU537-H08, YU536-D04 and YU537-H11, YU536-D04 and STE90-C11, YU537-H08 and YU537-H11, YU537-H08 and STE90-C11, YU537-H11 and STE90-C11.

In a further embodiment, the combination is selected from the group consisting of antibodies STE70-1E12 and STE72-8E1, STE70-1E12 and STE73-9G3, STE70-1E12 and STE73-2G8, STE72-8A6 and STE73-9G3, STE72-8A6 and STE73-2E9, STE72-8E1 and STE73-2B2, STE72-8E1 and STE72-8A2, STE73-9G3 and STE73-2B2, STE73-9G3 and STE72-8A2, STE73-2B2 and STE73-2C2, STE73-2B2 and STE73-2E9, STE73-2B2 and STE73-2G8, STE72-8E1 and STE72-2G4, STE72-8E1 and STE73-6C8, STE73-2B2 and STE73-6C1, STE73-2C2 and STE73-6C8, STE73-2E9 and STE72-2G4, STE73-2G8 and STE73-6C8, STE73-6C1 and STE72-2G4, STE73-6C1 and STE73-6C8, STE72-4C10 and STE73-2B2, STE72-4C10 and STE72-8A2 and STE72-4C10 and STE72-8E1.

The CDR, VH and/or VL sequences of the corresponding antibodies can be taken from the sequence listing and/or tables 1, 4-7.

The term "antigen" refers to a molecule or a portion of a molecule capable of being bound by a selective binding agent, such as an antigen binding protein (including, e.g., an antibody or immunological functional fragment thereof). In some embodiments, the antigen is capable of being used in an animal to produce antibodies capable of binding specifically to that antigen. An antigen can possess one or more epitopes that are capable of interacting with different antigen binding proteins, e.g., antibodies.

In specific embodiments, the antibody or antigen binding fragment thereof for use in the method of the invention may be monospecific, bispecific, or multispecific. Multispecific antibodies may be specific for different epitopes of one target polypeptide or may contain antigen-binding domains specific for epitopes of more than one target polypeptide.

The specific embodiments, antibody or antibody fragments of the invention may be conjugated to a therapeutic moiety ("immunoconjugate"), such as a cytotoxin, a chemotherapeutic drug, an immunosuppressant or a radioisotope.

Moreover, multi-specific antibodies (e.g., bispecifics) that bind to S1-protein and/or RBD and one or more additional antigens are nonetheless considered antibodies that "specifically bind' S1-protein or RBD, as used herein.

The term "therapeutically effective amount" means an amount that produces the desired effect for which it is administered. The exact amount will depend on the purpose of the treatment, and will be ascertainable by one skilled in the art using known techniques (see, for example, Lloyd (1999) The Art, Science and Technology of Pharmaceutical Compounding).

In another embodiment, the invention pertains to a method for treating COVID-19 which is ameliorated, improved, inhibited or prevented with the antibodies and antigen-binding fragments therein, comprising administering a therapeutic amount of the pharmaceutical composition to a subject in need thereof.

In yet another embodiment, the invention pertains to an antibody or antigen-binding fragment thereof for use to attenuate or inhibit a SARS-CoV-2 mediated disease or condition.

In yet another embodiment, the invention pertains to the use of an antibody or antigen-binding fragment thereof in the manufacture of a medicament for use to attenuate or inhibit a SARS-CoV-2-mediated disease or condition.

In yet another embodiment, the invention pertains to one of the uses mentioned herein, wherein the SARS-CoV-2-mediated disease or condition is pleurisy, pericarditis, lung consolidation, pulmonary oedema, pneumonia, serous exudation, fibrin exudation, pulmonary oedema, pneumocyte hyperplasia, large atypical pneumocytes, interstitial inflammation with lymphocytic infiltration and multinucleated giant cell formation, diffuse alveolar damage (DAD) with diffuse alveolar exudates, acute respiratory distress syndrome (ARDS), severe hypoxemia, exudates in alveolar cavities and pulmonary interstitial fibrosis plasmocytosis in bronchoalveolar lavage (BAL), disseminated intravascular coagulation (DIC), leukoerythroblastic reaction and microvesicular steatosis in the liver.

In yet another embodiment, the invention pertains to the diagnostic use the antibody or antigen-binding fragment thereof. Uses include but are not limited to the detection of infections, recognition of allergies and the measurement of viral load and/or other biological markers in blood.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, preferred methods and materials are now described.

**Preparation of Human Antibodies**

A fully human monoclonal antibody or antigen-binding fragment thereof can be produced in three ways.

In one approach, a mouse is used in which the immunoglobulin genes have been replaced with human genes. This mouse then makes antibody responses that use human antibody sequences.

A second approach uses a bacteriophage display library of human variable region sequences and selects for antigen binding in vitro. The selected genes are then combined with human constant region genes to reconstitute a complete IgG antibody.

A third approach utilizes B-cells for the production of antibodies.

As will be outlined in the examples in more detail, suspension panning may be used for high throughput antibody identification.

Notwithstanding, these human monoclonal antibody or antigen-binding fragment thereof can provoke immune responses (termed HAHA, or human anti-human antibody), probably largely akin to anti-idiotype responses that are themselves specific for the reagents' complementarity determining regions.

**Production of "antigens" and reference-proteins**

The antigens such as SARS-CoV-2 glycoprotein S and reference-proteins such as (human) ACE2 may be produced via isolation from an infected cell or host, or via conventional biotechnological methods, such as recombinant expression of the protein in a host cell, such as *E. coli,* yeast or the like.

Also fragments may be used according to this invention. As such the term "fragments" comprises preferably "functional fragments" which means parts of the full-length protein, which retains its specific function, such as for example binding activity, activation activity, inhibition activity and/or enzymatic activity. Normally such a functional fragment is a subunit and/or a domain of a protein, which is correctly three-dimensional folded when recombinantly expressed; or it is produced by isolating or recombinantly expressing the correctly folded full-length protein and then treating the full-length protein with a protease or other enzymatic or physical treatment which disconnects the subunit and/or domain of interest from the rest of the protein, thereby retaining its biological function.

Preferred functional fragments of the present invention include the receptor binding domain of the SARS-CoV-2 glycoprotein S, the S1 subunit of the SARS-CoV-2 glycoprotein S, the extra-cellular part of (human) ACE2, S1-mFc, S1-S2-His, RBD-hFc, IVIGs (intravenous immunoglobulins), as well as any fragment of an antibody which retains its binding ability to the antigen, etc.

**$K_D$-Measurement**

As will be outlined in more detail in the examples, the $K_D$ is measured for example with a protein A sensor loaded with the antibody or antigen-binding fragment thereof.

The antigen, for example a S1-HIS-fragment derived from the S1-subunit according SEQ-ID NO: 04, or a RBD of SEQ ID NO: 06, or the full S-protein of SARS-CoV-2 of SEQ-ID NO: 02 is then associated onto the antibody-loaded sensor. In this step the antigen is bound in different dilutions to the antibody-loaded sensor. Preferred dilutions range from 0 to 500 nM, in one embodiment a concentration regime of 0 nM, 7.8 nM, 15.6 nM, 31.2 nM, 62.5 nM, 125 nM, 250 nM and 500 nM antigen diluted in in 1% BSA-PBST (0.05% Tween 20).

Then each antibody-antigen loaded sensor is incubated with dissociation buffer and the dissociation is measured.

This measurement may be repeated after regeneration of the sensor by incubation with glycine in order to remove the antibody from the protein A and neutralization by incubation in PBS to neutralize the pH. Normally the measurement is repeated at least 5 times, 10 times, 15 times to achieve reliable $K_D$-values.

The sensor-material (protein A) without any antibody serves as a negative control in these measurements.

Further, the present invention relates to a nucleic acid molecule, e.g. a DNA molecule, encoding an antibody VH region, or an antibody VL region, or encoding a complete antibody or an antibody fragment as indicated above, a vector or vector system, i.e. a plurality of vectors, comprising said nucleic acid molecule(s) as indicated above in operative linkage with an expression control sequence, particularly with a heterologous expression control sequence. Furthermore, the invention relates to a cell comprising a nucleic acid molecule or a vector or vector system as described above. Vectors for the recombinant production of antibodies are well known in the art. The cell may be known host cell for producing antibodies or antibody fragments, e.g. a prokary-otic cell such as an E. coli cell, a yeast cell, an insect cell or a mammalian cell, e.g. a CHO cell or a hybridoma cell.

Still a further aspect of the present invention is a method of recombinantly producing an antibody or antibody fragment by growing a cell as described above in a culture medium and obtaining the antibody or antibody fragment from the cell or the culture medium. Suitable culture media and culture conditions are well known in the art.

Binding of therapeutic antibodies to SARS-CoV-2-"escape"-mutants

In one embodiment the present invention provides a therapeutic antibody or antigen-binding fragment thereof, which does not only bind to the glycoprotein S with the amino acid sequence of SEQ ID NO: 02 and neutralizes the infectious activity of SARS-CoV-2, but which also shows specific binding specifically to the "escape"-mutants of the glycoprotein S of SARS-CoV-2 in order to neutralize the infectious activity of these SARS-CoV-2-mutants. This is in contrast to prior-art anti-SARS-CoV-2 antibodies, whose binding is blocked by known RBD mutations, endowing the antibody of the present invention with intrinsic higher resistance to possible escape mutants.

The "escape"-mutation mentioned herein is selected from the list consisting of S1-V367F, S1-N439K, S1-G476S, S1-V483A, S1-E484K, S1-G485R, S1-F486V and S1-7PM (SEQ ID NO: 1730).

The mutations were published in the following sources:

- V367F: GISAID EpiFlu™ Database

- N439K: GISAID EpiFlu™ Database

- G476S: Shi et al. (https://doi.org/10.1038/s41586-020-2381-y)

- V483A: Shi et al.

- E484K: Baum et al. (https://doi.org/10.1126/science.abd0831)

- G485R: GISAID EpiFlu™ Database

- F486V: Baum et al.

All of these mutations lie in the three regions identified by Yi et al. (Cell. & Mol. Imm., 15 May 2020) which are in the regions of between aa 439 to 456; aa 473 to 476 and aa 483 to 505.

In one aspect the present invention discloses even a therapeutic antibody or antigen-binding fragment thereof, which binds specifically to the S1-7PM-mutant (SEQ ID NO: 1730), i.e. a mutant which comprises all seven "escape"-mutations in one molecule.

Thus, one advantage of the inventive antibodies is the recognition of a large number of known "escape"-mutations. This allows the treatment of a wide range of patients with just one antibody-composition, simplifying the administration scheme and lowering costs for production and market-approval significantly. This is advantageous especially in comparison to compositions comprising cocktails of two or more monoclonal antibodies.

Furthermore, without being bound to theory, it seems that the inventive antibodies recognize a "sweet spot" in the glycoprotein S, which seems to be important for the interaction of the virus with the host cell and, thus, cannot be changed without loss of infectivity or viral function. Thus, another technical advantage of the inventive antibodies is that the likelihood of the virus mutating in a way which "escapes" the antibody is significantly reduced.

Some experiments were conducted with a number of antibodies against the seven "escape"-mutants. As control the two published antibodies RGN10933 and RGN10987 were used.

ELISA titrating Abs on wild-type and mutated S1-protein

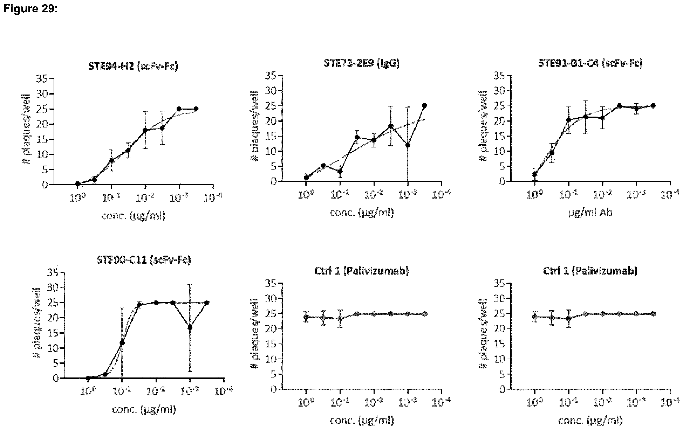

It turned out that all antibodies produced by the selected method showed a good binding profile to all "escape"-mutants including the S1-7PM-mutant (cf. example 11 and figures 29 - 31). In contrast, RGN10933 did not bind to the S1-7PM-mutant and showed reduced binding specifically to at least the F486V-mutant.

An affinity test of the selected Abs against mutated S1-protein

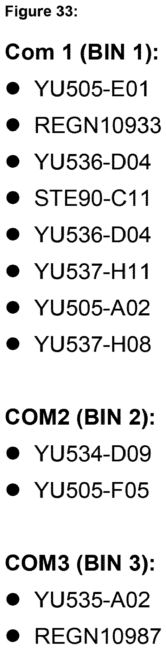

The overall affinity of the inventive antibodies against the targets was tested in a BLI-assay (cf. example 13 and figures 33). The affinity was still high enough to qualify the antibodies as therapeutic antibodies, although reduced against the "escape"-mutants as compared to the "wild-type"-S1-protein.

Again, RGN10933 showed a significant weaker binding specifically to some mutants, and the binding specifically to F486V and S1-7PM was so low that it could not be measured.

**Preventive use of the inventive antibodies**

As already described in other parts of this specification, the therapeutic use of the antibodies or antigen-binding fragments thereof of the present invention, may include

(1) the prevention or reduction of the infectivity of SARS-CoV-2;

(2) the prevention or reduction of any overreaction of the immune-system of the host (e.g. ADE-reaction or cytokine storm syndrome), and/or

(3) the prevention or reduction of any condition associated with COVID-19, such as fever, cough, fatigue, shortness of breath, loss of smell and taste, pleurisy, pericarditis, lung consolidation, pulmonary oedema, pneumonia, serous exudation, fibrin exudation, pulmonary oe-dema, pneumocyte hyperplasia, large atypical pneumocytes, interstitial inflammation with lymphocytic infiltration and multinucleated giant cell formation, diffuse alveolar damage (DAD), diffuse alveolar exudates, acute respiratory distress syndrome (ARDS), severe hypoxemia, exudates in alveolar cavities and pulmonary interstitial fibrosis plasmocytosis in bronchoalveolar lavage (BAL), disseminated intravascular coagulation (DIC), leukoerythroblastic reaction, microvesicular steatosis in the liver and any combination thereof.

In one aspect of the present invention the antibodies or antigen-binding fragments thereof of the present invention are applied if the patient shows at least one of the following symptoms: manifested, productive and unproductive cough, fever, cold, disturbance of the olfactory and / or taste sensors, pneumonia, sore throat, shortness of breath, headache and body aches, loss of appetite, weight loss, nausea, abdominal pain, vomiting, diarrhea, conjunctivitis, skin rash, lymph node swelling, apathy, somnolence.

In yet another aspect, the person to be treated with the antibodies or antigen-binding fragments thereof of the present invention may be asymptomatic or pre-symptomatic, but has an elevated risk of developing a COVID-19 disease, because, for example, belonging to a risk group, e.g. with an age above 60 years, above 65 years, above 70 years, above 75 years; and/or because the person having a disease which is known to elevate the risk of a severe COVID-19 disease, such as diabetes, asthma, obesity, cardiovascular diseases, COPD, immune-deficiency, cancer, or the like; and/or because the person was exposed or will be exposed to another person which was tested positive for a COVID-19 disease; and/or because the person has a biomarker (e.g. blood group, IL-6-levels, etc.), which deviation from the norm (e.g. presence, elevation or decrease as compared to control group) is associated with an elevated risk of a severe COVID-19 disease.

In another aspect of the invention the antibodies or antigen-binding fragments thereof of the present invention are applied if the patient is pregnant in order to prevent a COVID-19 disease.

In yet another aspect of the invention the antibodies or antigen-binding fragments thereof of the present invention are applied, if the patient is suspected to develop or has already developed a multisystem inflammatory syndrome, which mainly seems to occur in children (MIS-C).

According to the CDC classification, one speaks of a MIS-C if the following criteria are met:

- A person aged <21 years with fever, inflammation in the laboratory AND

- Signs of a clinically serious illness that requires hospitalization with a multisystem (> 2) organ involvement (cardiac, renal, respiratory, hematological, gastrointestinal, dermatological or neurological) AND

- no alternative plausible diagnoses are available AND

- positive for a current or recent SARS-CoV-2 infection (detection by RT-PCR, serology or Antigen test or COVID-19 exposure within 4 weeks prior to the onset of symptoms).

**Therapeutic Administration and Formulations**

The invention provides therapeutic compositions comprising the antibodies or antigen-binding fragments thereof of the present invention. The administration of therapeutic compositions in accordance with the invention will be administered with suitable carriers, excipients, and other agents that are incorporated into formulations to provide improved transfer, delivery, tolerance, and the like.

A multitude of appropriate formulations can be found in the formulary known to all pharmaceutical chemists: Remington's Pharmaceutical Sciences, Mack Publishing Company, Easton, PA.

These formulations include, for example, aqueous solutions, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax.

The dose may vary depending upon the age and the size of a subject to be administered, severity of the target disease, individual condition of the subject, route of administration, and the like.

The antibody and antigen-binding fragment thereof is used for treating various conditions and diseases associated with SARS-CoV-2, including fever, cough, fatigue, shortness of breath, loss of smell and taste, pleurisy, pericarditis, lung consolidation, pulmonary oedema, pneumonia, serous exudation, fibrin exudation, pulmonary oedema, pneumocyte hyperplasia, large atypical pneumocytes, interstitial inflammation with lymphocytic infiltration and multinucleated giant cell formation, diffuse alveolar damage (DAD), diffuse alveolar exudates, acute respiratory distress syndrome (ARDS), severe hypoxemia, exudates in alveolar cavities and pulmonary interstitial fibro-sis plasmocytosis in bronchoalveolar lavage (BAL), disseminated intravascular coagulation (DIC), leukoerythroblastic reaction, microvesicular steatosis in the liver and any combination thereof.

Depending on the severity of the condition, the frequency and the duration of the treatment can be adjusted.

Various delivery systems are known and can be used to administer the pharmaceutical composition of the invention, e.g., encapsulation in liposomes, microparticles, microcapsules, recombinant cells capable of expressing the mutant viruses, receptor mediated endocytosis.

Methods of introduction include, but are not limited to, intradermal, intramuscular, intraperitoneal, intravenous, subcutaneous, intranasal, epidural, inhalative and oral routes.

The composition may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

The pharmaceutical composition can be also delivered in a vesicle, in particular a liposome.

In certain embodiments, the pharmaceutical composition can be delivered in a controlled release system, such as a pre-filled syringe, a dermal patch, infusion, pen- or auto-injector-device (single-dose, multi-dose, disposable, re-usable), syrette, large-volume pump-device, and the like.

In one embodiment a pre-filled syringe, or auto-injector, or pump device is preferred.

In another embodiment, polymeric materials can be used. In yet another embodiment, a controlled release system can be placed in proximity of the composition's target, thus requiring only a fraction of the systemic dose.

The injectable preparations may include dosage forms for intravenous, subcutaneous, intracuta-neous and intramuscular injections, drip infusions, etc.

These injectable preparations may be prepared by methods publicly known.

For example, the injectable preparations may be prepared, e.g., by dissolving, suspending or emulsifying the antibody or its salt described above in a sterile aqueous medium or an oily medium conventionally used for injections.

As the aqueous solution for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc.

As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

A pharmaceutical composition of the present invention can be delivered subcutaneously or intravenously with a standard needle and syringe.

In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused.

In a disposable pen delivery device, there is no replaceable cartridge. Rather, the disposable pen delivery device comes prefilled with the pharmaceutical composition held in a reservoir within the device.

Once the reservoir is emptied of the composition, the entire device is discarded.

Numerous reusable pen and auto-injection delivery devices have applications in the subcutaneous delivery of a pharmaceutical composition of the present invention. Examples include, but certainly are not limited to AUTOPEN™ (Owen Mumford, Inc., Woodstock, UK), DI-SETRONIC™ pen (Disetronic Medical Systems, Burghdorf, Switzerland), HUMALOG MIX 75/25™ pen, HUMALOG™ pen, HUMALIN 70/30™ pen (Eli Lilly and Co., Indianapolis, IN), NO-VOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™ OPTIPEN STARLET™, and OPTICLIK™ (sanofi-aventis, Frankfurt, Germany), to name only a few.

Examples of disposable pen delivery devices having applications in subcutaneous delivery of a pharmaceutical composition of the present invention include, but certainly are not limited to the SOLOSTAR™ pen (sanofi-aventis), the FLEXPEN™ (Novo Nordisk), and the KWIKPEN™ (Eli Lilly).

Advantageously, the pharmaceutical compositions for oral or parenteral use described above are prepared into dosage forms in a unit dose suited to fit a dose of the active ingredients.

Such dosage forms in a unit dose include, for example, tablets, pills, capsules, injections (ampoules), suppositories, etc.

The amount of the aforesaid antibody contained is generally about 5 to about 500 mg per dosage form in a unit dose; especially in the form of injection, it is preferred that the aforesaid antibody is contained in about 5 to about 100 mg and in about 10 to about 250 mg for the other dosage forms.

The invention provides therapeutic methods in which the antibody or antibody fragment of the invention is useful to treat COVID-19 associated with a variety of conditions involving SARS-CoV-2. The antibodies or antigen-binding fragments of the invention are particularly useful for the treatment of the viral infection with SARS-CoV-2.

Combination therapies may include the antibodies or antigen-binding fragments of the invention with, for example, one or more of any agent that

(1) prevents or reduces the infectivity of SARS-CoV-2;

(2) prevents or reduces secondary infections with other viruses (especially respiratory viruses such as influenza), bacteria (especially bacteria residing in the respiratory tract of the host) and/or parasites;

(3) prevents or reduces any overreaction of the immune-system of the host (e.g. ADE-reaction or cytokine storm syndrome)

(4) prevents or reduces any conditions associated with COVID-19, such as fever, cough, fatigue, shortness of breath, loss of smell and taste, pleurisy, pericarditis, lung consolidation, pulmonary oedema, pneumonia, serous exudation, fibrin exudation, pulmonary oe-dema, pneumocyte hyperplasia, large atypical pneumocytes, interstitial inflammation with lymphocytic infiltration and multinucleated giant cell formation, diffuse alveolar damage (DAD), diffuse alveolar exudates, acute respiratory distress syndrome (ARDS), severe hypoxemia, exudates in alveolar cavities and pulmonary interstitial fibrosis plasmocytosis in bronchoalveolar lavage (BAL), disseminated intravascular coagulation (DIC), leukoerythroblastic reaction, microvesicular steatosis in the liver and any combination thereof.

Examples of such therapeutic agents to be used in combination with the antibodies or antigen-binding fragments of the invention in a pharmaceutical composition include, but are not limited to antiviral drugs including Indinavir, Saquinavir, Lopinavir, Ritonavir, interferone-beta, Remdesivir, Favipiravir, Oseltamivir, Chloroquin, Hydroxychloroquin, Umifenovir; serine proteases, including TMPRSS2 or Camostatmesilat; antibiotics, including Linezolid, Azithromycin, Nemonoxacin or Fluorchinolone; interleukin-6-rezeptor-antagonists, including Tocilizumab or Sarilumab in mono-therapy or combined with Methotrexate; anti-parasite treatments, including Ivermectin; anticoagulants and any combination thereof.

In a further embodiment the antibodies or antigen-binding fragments of the invention in a pharmaceutical composition may be combined with at least one member of the group comprising Tocilizumab, Dexamethasone, Remdesivir, Lopinavir, Hydroxychloroquine, Hydrocortisone, Methylprednisolone, Prednisolone, antibiotics, and any combination thereof.

The term "melting temperature of a protein ($T_m$)" as used herein means a value which is a thermodynamic parameter of a protein that can only strictly be determined under thermodynamic equilibrium conditions and with well-known baseline parameters for the required thermodynamic fit. However, thermal unfolding experiments are often not in equilibrium, because irreversible protein aggregation processes keep removing unfolded protein from the reaction, making the unfolding process irreversible. Heating rates faster than 1 °C/min additionally decrease the equilibrium-resemblance of the unfolding reaction. $T_{m1}$ as used herein refers to the unfolding of the a first domain (for example Fc) of the tested antibody; $T_{m2}$ as used herein refers to the unfolding of a second domain (for example Fab) of the tested antibody.

The term "onset temperature of thermal melting ($T_m$ onset)" as used herein means the temperature at which thermal protein unfolding starts. It is determined from the first derivative curve of the thermal unfolding signal (either 350/330 nm ratio signal or individual 350 and 330 nm signals).

The term "Inflection point (IP)" as used herein means that due to constraints, in nanoDSF, thermal melting temperatures of proteins are determined directly from the measured data by using a smoothing algorithm and detection of inflection points in the data. Assuming an equilibrium, determined IPs correspond to the $T_m$ values of protein.

The term "onset temperature of protein aggregation ($T_{agg}$)" as used herein means the temperature at which the protein sample starts to aggregate. It is determined from the first derivate curve of the back-scattering signal. Typically, $T_{agg}$ correlates with $T_m$. For proteins that show more than one unfolding event, aggregation may start as a consequence of the first unfolding event or of later ones.

The term "ratio 350/330" as used herein means the measure for a spectral shift in the fluorescence emission profile of the Tryptophan (Trp) residues. This shift occurs in most unfolding events and is caused by the environmental change that the Trp residues undergo upon unfolding of the protein: In the folded state, Trp residues are often buried in the hydrophobic core of a protein, which leads to the fluorescence emission peaking around 330 nm. They then become surface-exposed during unfolding, which often shifts the fluorescence emission peak toward 350 nm. Since the ratio 350 nm / 330 nm typically obliterates effects of auto-fluorescent additives and general fluorescence decay with increasing temperature, this detection mode is usually more robust than single wavelength detection. Therefore, clear IP points and $T_m$s can be derived which might not be visible in the single wavelength detection modes. Vice versa, it is also possible that unfolding events which do not trigger an emission peak shift are visible in the single wavelength data, but not in the ratio.

The term "scattering" as used herein means the phenomenon that any particle struck by light will reradiate its energy in all directions with an intensity depending on the direction. The intensity of light passing through a sample containing particles (e.g. aggregates) will decrease as the light encounters each particle and gets scattered. The Prometheus™ system measures this using backreflection optics in order to understand a sample's aggregation behavior.

The term "germinality index" (GI) describes herein the percentage of identity to the IMGT human germline gene. CDR3 is excluded from the analysis, since it is not encoded in the germline gene. For VH a homology of >90.8% the tool score is +1 and for a homology <80.7% the tool score is -1. For VL a homology of >95.1% the tool score is +1 and for a homology <85.5% the tool score is -1.

The term "hydrophobicity score" as used herein is calculated and summed to total score (similar to SAP (Spatial-Aggregation-Propensity) algorithm; Chennamsetty, 2009. For the total hydrophobicity score the scores of all VH and VL residues are summed up if they are >0.05 (hydrophobic and on the surface). The tool score is +1 for a total score < 5.1 and the tool score is -1 for a total score > 5.1. For the total hydrophobicity score the scores of all CDR residues of VH and VL (Kabat annotation) are summed up if they are >0.05 (hydrophobic and on the surface). The tool score is +1 for a CDR score < 2.3 and the tool score is -1 for a CDR score >2.5.

The term pI (isoelectric point) as used herein is the pH at which the net charge of a protein is zero. For polypeptides, the isoelectric point depends primarily on the dissociation constants (pKa) for the ionizable groups of seven charged amino acids: Glutamate (δ-carboxyl group), aspartate (ß-carboxyl group), cysteine (thiol group), tyrosine (phenol group), histidine (imidazole side chains), lysine (ε-ammonium group), arginine (guanidinium group) and terminal groups (NH2 and COOH). The tool uses the EMBOSS (Rice, 2000) pKa values:

| NH2 | COOH | C | D | E | H | K | R | Y |
|------|------|-----|-----|-----|-----|------|------|------|
| 8,6 | 3,6 | 8,5 | 3,9 | 4,1 | 6,5 | 10,8 | 12,5 | 10,1 |

A protein has its lowest solubility at its pI and for antibodies it is generally observed that increases in net positive charge of antibodies result in increased blood clearance and increased tissue retention with shorter half-life, whereas with lower pI antibodies have decreased tissue uptake and longer half-life. Therefore antibodies with a pI < 7.6 or > 8.4 get a tool score of -1 and antibodies with a pI between 7.6 - 8.4 get a tool score of +1.

The term "surface charge" as used herein is the prediction of the surface charge the hydrophobicity values of all amino acids changed to charge values:

D and E have a value of -1, K and R have a value of 1, H has a value of 0.1 and all other amino acids have a value of 0. For all surface residues the charge score is calculated.

For the scFv charge score the scores of all surface D, E, K, R, H residues of VH and VL are summed up. The tool score is +1 for a total charge score between -4.5 and 3.5 and the tool score is -1 for a total score < -4.5 and >3.5.

For the total CDR charge score the scores of all D, E, K, R, H residues of VH and VL CDRs are summed up. The tool score is +1 for a total CDR charge score between -2.1 and 1 and the tool score is -1 for a total score < -2.1 and >1.

For the negative CDR charge score the scores of all D, E residues of VH and VL CDRs are summed up. The tool score is +1 for a negative CDR charge score > -3.5 and the tool score is - 1 for a total score < -4.5.

For the positive CDR charge score the scores of all D, E residues of VH and VL CDRs are summed up. The tool score is +1 for a positive CDR charge score < 2.5 and the tool score is -1 for a total score < 3.

In one aspect of the present invention the antibody or antigen-binding fragment or a substantially similar variant thereof can be used for a test-assay, either in a laboratory setup (e.g. a serological test for SARS-CoV-2 antigen) and/or in a quick-test-format.

In one aspect the present invention pertains to a humanized or a human therapeutic monoclonal antibody or antigen-binding fragment, or a substantially similar variant thereof, that binds specifically to SARS-CoV-2 glycoprotein S with the amino acid sequence of SEQ ID NO: 02 and neutralizes the infectious activity of SARS-CoV-2.

In another aspect the antibody or antigen-binding fragment or a substantially similar variant thereof is monoclonal.

In yet another aspect the antibody or antigen-binding fragment or a substantially similar variant thereof is a humanized or a human monoclonal antibody, or humanized or a human antigen-binding fragment thereof.

In another aspect the therapeutic antibody or antigen-binding fragment or a substantially similar variant thereof shows a neutralization of the infectious activity of SARS-CoV-2 which results in confluence score of at least 95% as measured with an infectious SARS-CoV-2 VERO E6 neutralizing assay.

In yet another aspect the therapeutic antibody or antigen-binding fragment or a substantially similar variant thereof has a specific binding to the RBD with a $K_D$ of 65 x $10^{-9}$ M (65 nM) or less

In yet another aspect the therapeutic antibody or antigen-binding fragment or a substantially similar variant thereof binds specifically to an epitope within the receptor binding domain (RBD) of the S1 domain of SARS-CoV-2 glycoprotein (SEQ ID NO: 06) and binds specifically to at least one of the escape-mutations of the S1 domain of SARS-CoV-2 glycoprotein selected from the list consisting of S1-V367F, S1-N439K, S1-G476S, S1-V483A, S1-E484K, S1-G485R, S1-F486V, S1-7PM-mutant (SEQ ID NO: 1730), and any combination thereof.

In yet another aspect the therapeutic antibody or antigen-binding fragment or a substantially similar variant thereof binds specifically both to an epitope comprising the amino acids 400-FVIRGDEVRQIAPQTGKIADDYN-422 within the RBD (SEQ ID NO: 06) as well as to the S1-7PM-mutant (SEQ ID NO: 1730).

In yet another aspect the therapeutic antibody or antigen-binding fragment or a substantially similar variant thereof competes for binding to an epitope within the RBD (SEQ ID NO: 06) with an antibody comprising a heavy chain variable region of the amino acid sequence in SEQ ID NO: 926 and a light chain variable region of the amino acid sequence in SEQ ID NO: 928; and wherein the contamination ratio is less than 0.2.

In yet another aspect the therapeutic antibody or antigen-binding fragment or a substantially similar variant thereof has a contamination ratio of 0.1 or less, of 0.05 or less, of 0.025 or less or even of 0.001 or less.

In yet another aspect the therapeutic antibody or antigen-binding fragment or a substantially similar variant thereof competes for binding to the epitope 165-AKRVNCYFPLQSYGFQ-180 within the S1-7PM-mutant (SEQ ID NO: 1730) with an antibody comprising a heavy chain variable region of the amino acid sequence in SEQ ID NO: 1305; and a light chain variable region of the amino acid sequence in SEQ ID NO: 1309 or with an antibody comprising a heavy chain variable region of the amino acid sequence in SEQ ID NO: 926 and a light chain variable region of the amino acid sequence in SEQ ID NO: 928.

In yet another aspect the antibody or antigen-binding fragment or a substantially similar variant thereof competes for binding to the RBD-domain of the S1 domain of SARS-CoV-2 glycoprotein of SEQ ID No: 06 with the ACE2-receptor of the sequence depicted in SEQ ID NO: 08 in a competition assay.

In yet another aspect the antibody or antigen-binding fragment or a substantially similar variant thereof competes for binding to an epitope within an amino acid sequence selected from the group of SEQ ID No: 04 and/or SEQ ID No: 06 with an antibody comprising a heavy chain variable region of the amino acid sequence in SEQ ID NO: 406; and a light chain variable region of the amino acid sequence in SEQ ID NO: 408.

In yet another aspect the therapeutic antibody or antigen-binding fragment or a substantially similar variant thereof comprises:

a heavy chain CDR1 (H-CDR1) domain with an amino acid sequence selected from the group consisting of SEQ ID NO: 910, 350, 390, and 1306;

a heavy chain CDR2 (H-CDR2) domain with an amino acid sequence selected from the group consisting of SEQ ID NO: 912, 352, 392, and 1307;

a heavy chain CDR3 (H-CDR3) domain with an amino acid sequence selected from the group consisting of SEQ ID NO: 914, 354, 394, and 1308;

and

a light chain CDR1 (L-CDR1) domain with an amino acid sequence selected from the group consisting of SEQ ID NO: 916, 356, 396, and 1310;

a light chain CDR2 (L-CDR2) domain with an amino acid sequence selected from the group consisting of SEQ ID NO: 918, 358, 398, and 1311;

a light chain CDR3 (L-CDR3) domain with an amino acid sequence selected from the group consisting of SEQ ID NO: 920, 360, 400, and 1312.

In yet another aspect the therapeutic antibody or antigen-binding fragment or a substantially similar variant thereof is selected from the group of antibodies comprising a heavy chain variable region of the amino acid sequence in SEQ ID NO: 926 and a light chain variable region of the amino acid sequence in SEQ ID NO: 928; a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another aspect the therapeutic antibody or antigen-binding fragment or a substantially similar variant thereof comprises a heavy chain variable region of the amino acid sequence in SEQ ID NO: 1305 and a light chain variable region of the amino acid sequence in SEQ ID NO: 1309.

In yet another aspect the therapeutic antibody or antigen-binding fragment or a substantially similar variant thereof comprises:

a heavy chain CDR1 (H-CDR1) domain with an amino acid sequence selected from the group consisting of SEQ ID NO: 10, 30, 50, 70, 90, 110, 130, 150, 170, 190, 210, 230, 250, 270, 290, 310, 330, 350, 370, 390, 410, 430, 450, 470, 490, 510, 530, 550, 570, 590, 610, 630, 650, 670, 690, 710, 730, 750, 770, 790, 810, 830, 850, 870, 890, 910, 930, 950, 970, 990, 1110, 1130, 1150, 1170, 1190, 1210, 1230, 1250, 1270, 1290, 1298, 1306, 1314, 1322, 1330, 1338, 1346, 1354, 1362, 1370, 1378, 1386, 1394, 1402, 1410, 1418, 1426, 1434, 1442, 1450, 1458, 1466, 1474, 1482, 1490, 1498, 1506, 1514, 1522, 1530, 1538, 1546, 1554, 1562, 1570, 1578, 1586, 1594, 1602, 1610, 1618, 1626, 1634, 1642, 1650, 1658, 1666, 1674, 1682, 1690, 1698, 1706, 1714, and 1722;

a heavy chain CDR2 (H-CDR2) domain with an amino acid sequence selected from the group consisting of SEQ ID NO: 12, 32, 52, 72, 92, 112, 132, 152, 172, 192, 212, 232, 252, 272, 292, 312, 332, 352, 372, 392, 412, 432, 452, 472, 492, 512, 532, 552, 572, 592, 612, 632, 652, 672, 692, 712, 732, 752, 772, 792, 812, 832, 852, 872, 892, 912, 932, 952, 972, 992, 1112, 1132, 1152, 1172, 1192, 1212, 1232, 1252, 1272, 1291, 1299, 1307, 1315, 1323, 1331, 1339, 1347, 1355, 1363, 1371, 1379, 1387, 1395, 1403, 1411, 1419, 1427, 1435, 1443, 1451, 1459, 1467, 1475, 1483, 1491, 1499, 1507, 1515, 1523, 1531, 1539, 1547, 1555, 1563, 1571, 1579, 1587, 1595, 1603, 1611, 1619, 1627, 1635, 1643, 1651, 1659, 1667, 1675, 1683, 1691, 1699, 1707, 1715, and 1723;

a heavy chain CDR3 (H-CDR3) domain with an amino acid sequence selected from the group consisting of SEQ ID NO: 14, 34, 54, 74, 94, 114, 134, 154, 174, 194, 214, 234, 254, 274, 294, 314, 334, 354, 374, 394, 414, 434, 454, 474, 494, 514, 534, 554, 574, 594, 614, 634, 654, 674, 694, 714, 734, 754, 774, 794, 814, 834, 854, 874, 894, 914, 934, 954, 974, 994, 1114, 1134, 1154, 1174, 1194, 1214, 1234, 1254, 1274, 1292, 1300, 1308, 1316, 1324, 1332, 1340, 1348, 1356, 1364, 1372, 1380, 1388, 1396, 1404, 1412, 1420, 1428, 1436, 1444, 1452, 1460, 1468, 1476, 1484, 1492, 1500, 1508, 1516, 1524, 1532, 1540, 1548, 1556, 1564, 1572, 1580, 1588, 1596, 1604, 1612, 1620, 1628, 1636, 1644, 1652, 1660, 1668, 1676, 1684, 1692, 1700, 1708, 1716, and 1724;

and

a light chain CDR1 (L-CDR1) domain with an amino acid sequence selected from the group consisting of SEQ ID NO: 16, 36, 56, 76, 96, 116, 136, 156, 176, 196, 216, 236, 256, 276, 296, 316, 336, 356, 376, 396, 416, 436, 456, 476, 496, 516, 536, 556, 576, 596, 616, 636, 656, 676, 696, 716, 736, 756, 776, 796, 816, 836, 856, 876, 896, 916, 936, 956, 976, 996, 1116, 1136, 1156, 1176, 1196, 1216, 1236, 1256, 1276, 1294, 1302, 1310, 1318, 1326, 1334, 1342, 1350, 1358, 1366, 1374, 1382, 1390, 1398, 1406, 1414, 1422, 1430, 1438, 1446, 1454, 1462, 1470, 1478, 1486, 1494, 1502, 1510, 1518, 1526, 1534, 1542, 1550, 1558, 1566, 1574, 1582, 1590, 1598, 1606, 1614, 1622, 1630, 1638, 1646, 1654, 1662, 1670, 1678, 1686, 1694, 1702, 1710, 1718, and 1726;

a light chain CDR2 (L-CDR2) domain with an amino acid sequence selected from the group consisting of SEQ ID NO: 18, 38, 58, 78, 98, 118, 138, 158, 178, 198, 218, 238, 258, 278, 298, 318, 338, 358, 378, 398, 418, 438, 458, 478, 498, 518, 538, 558, 578, 598, 618, 638, 658, 678, 698, 718, 738, 758, 778, 798, 818, 838, 858, 878, 898, 918, 938, 958, 978, 998, 1118, 1138, 1158, 1178, 1198, 1218, 1238, 1258, 1278, 1295, 1303, 1311, 1319, 1327, 1335, 1343, 1351, 1359, 1367, 1375, 1383, 1391, 1399, 1407, 1415, 1423, 1431, 1439, 1447, 1455, 1463, 1471, 1479, 1487, 1495, 1503, 1511, 1519, 1527, 1535, 1543, 1551, 1559, 1567, 1575, 1583, 1591, 1599, 1607, 1615, 1623, 1631, 1639, 1647, 1655, 1663, 1671, 1679, 1687, 1695, 1703, 1711, 1719, and 1727;

a light chain CDR3 (L-CDR3) domain with an amino acid sequence selected from the group consisting of SEQ ID NO: 20, 40, 60, 80, 100, 120, 140, 160, 180, 200, 220, 240, 260, 280, 300, 320, 340, 360, 380, 400, 420, 440, 460, 480, 500, 520, 540, 560, 580, 600, 620, 640, 660, 680, 700, 720, 740, 760, 780, 800, 820, 840, 860, 880, 900, 920, 940, 960, 980, 1000, 1120, 1140, 1160, 1180, 1200, 1220, 1240, 1260, 1280, 1296, 1304, 1312, 1320, 1328, 1336, 1344, 1352, 1360, 1368, 1376, 1384, 1392, 1400, 1408, 1416, 1424, 1432, 1440, 1448, 1456, 1464, 1472, 1480, 1488, 1496, 1504, 1512, 1520, 1528, 1536, 1544, 1552, 1560, 1568, 1576, 1584, 1592, 1600, 1608, 1616, 1624, 1632, 1640, 1648, 1656, 1664, 1672, 1680, 1688, 1696, 1704, 1712, 1720, and 1728.

In yet another aspect the therapeutic antibody or antigen-binding fragment or a substantially similar variant thereof comprises a variable heavy chain ($V_H$) with an amino acid sequence selected from the group consisting of SEQ ID NO: 24, 44, 64, 84, 104, 124, 144, 164, 184, 204, 224, 244, 264, 284, 304, 324, 344, 364, 384, 404, 424, 444, 464, 484, 504, 524, 544, 564, 584, 604, 624, 644, 664, 684, 704, 724, 744, 764, 784, 804, 824, 844, 864, 884, 904, 924, 944, 964, 984, 1004, 1024, 1044, 1064, 1084, 1104, 1124, 1144, 1164, 1184, 1204, 1224, 1244, 1264, 1284, 1289, 1297, 1305, 1313, 1321, 1329, 1337, 1345, 1353, 1361, 1369, 1377, 1385, 1393, 1401, 1409, 1417, 1425, 1433, 1441, 1449, 1457, 1465, 1473, 1481, 1489, 1497, 1505, 1513, 1521, 1529, 1537, 1545, 1553, 1561, 1569, 1577, 1585, 1593, 1601, 1609, 1617, 1625, 1633, 1641, 1649, 1657, 1665, 1673, 1681, 1689, 1697, 1705, 1713, and 1721; and

a variable light chain ($V_L$) selected from the group consisting of SEQ ID NO: 22, 42, 62, 82, 102, 122, 142, 162, 182, 202, 222, 242, 262, 282, 302, 322, 342, 362, 382, 402, 422, 442, 462, 482, 502, 522, 542, 562, 582, 602, 622, 642, 662, 682, 702, 722, 742, 762, 782, 802, 822, 842, 862, 882, 902, 922, 942, 962, 982, 1002, 1022, 1042, 1062, 1082, 1102, 1122, 1142, 1162, 1182, 1202, 1222, 1242, 1262, 1282, 1293, 1301, 1309, 1317, 1325, 1333, 1341, 1349, 1357, 1365, 1373, 1381, 1389, 1397, 1405, 1413, 1421, 1429, 1437, 1445, 1453, 1461, 1469, 1477, 1485, 1493, 1501, 1509, 1517, 1525, 1533, 1541, 1549, 1557, 1565, 1573, 1581, 1589, 1597, 1605, 1613, 1621, 1629, 1637, 1645, 1653, 1661, 1669, 1677, 1685, 1693, 1701, 1709, 1717, and 1725.

The therapeutic antibody or antigen-binding fragment thereof comprises a heavy chain with an amino acid sequence selected from the group consisting of SEQ ID NO: 26, 46, 66, 86, 106, 126, 146, 166, 186, 206, 226, 246, 266, 286, 306, 326, 346, 366, 386, 406, 426, 446, 466, 486, 506, 526, 546, 566, 586, 606, 626, 646, 666, 686, 706, 726, 746, 766, 786, 806, 826, 846, 866, 886, 906, 926, 946, 966, 986, 1006, 1026, 1046, 1066, 1086, 1106, 1126, 1146, 1166, 1186, 1206, 1226, 1246, 1266, and 1286; and

a light chain with an amino acid sequence selected from the group consisting of SEQ ID NO: 28, 48, 68, 88, 108, 128, 148, 168, 188, 208, 228, 248, 268, 288, 308, 328, 348, 368, 388, 408, 428, 448, 468, 488, 508, 528, 548, 568, 588, 608, 628, 648, 668, 688, 708, 728, 748, 768, 788, 808, 828, 848, 868, 888, 908, 928, 948, 968, 988, 1008, 1028, 1048, 1068, 1088, 1108, 1128, 1148, 1168, 1188, 1208, 1228, 1248, 1268, and 1288.

In yet another aspect the therapeutic antibody or antigen-binding fragment or a substantially similar variant thereof is characterized by one or more of the following:

I. capable of binding specifically to the S1- domain of SARS-CoV-2 Glycoprotein with an affinity ($K_D$) of $1.5 \times 10^{-8}$ M or less, preferably less than $3 \times 10^{-9}$ M,

II. capable of reducing or preventing an antibody-dependent enhancement (ADE) reaction in a patient,

III. capable of preventing, reducing or healing a viral infection with SARS-CoV-2,

IV. capable of reducing or preventing the cytokine storm syndrome (CSS) in a patient,

V. capable of reducing the load of viral particles in the sputum of a patient by at least 50% measured 48 hours after administration of the therapeutic antibody or antigen-binding fragment thereof.

VI. having an $IC_{50}$ of less than 0.036 µg/ml, preferably of 0.026 µg/ml or less.

In yet another aspect an isolated nucleic acid molecule encoding the therapeutic antibody or antigen-binding fragment is encompassed.

In yet another aspect an expression vector is encompassed comprising said nucleic acid molecule.

In yet another aspect a method of producing the therapeutic antibody or antigen-binding fragment or a substantially similar variant thereof is encompassed comprising the steps of

i. introducing the expression vector mentioned before into an isolated host cell,

ii. growing the cell under conditions permitting production of the antibody or antigen-binding fragment thereof,

iii. recovering the antibody or fragment so produced.

In yet another aspect a pharmaceutical composition is encompassed comprising said therapeutic antibody or antigen-binding fragment or a substantially similar variant thereof.

In yet another aspect said pharmaceutical composition further comprises at least a second therapeutic agent, wherein the second therapeutic agent is selected from antiviral drugs including Indinavir, Saquinavir, Lopinavir, Ritonavir, interferon-beta, Remdesivir, Favipiravir, Oseltamivir, Chloroquin, Hydroxychloroquin, Umifenovir; serine proteases, including TMPRSS2 or Camostatmesilat; antibiotics, including Linezolid, Azithromycin, Nemonoxacin or Fluorchinolone; interleukin-6-receptor-antagonists, including Tocilizumab or Sarilumab in mono-therapy or combined with Methotrexate; anti-parasite treatments, including Ivermectin; anticoagulants and any combination thereof.

In yet another aspect the therapeutic antibody or antigen-binding fragment or a substantially similar variant thereof according to present invention is for the use in the treatment of a SARS-CoV-2-related disease or condition in a patient.

In yet another aspect said SARS-CoV-2-related disease or condition is selected from the group comprising fever, cough, fatigue, shortness of breath, loss of smell and taste, pleurisy, pericarditis, lung consolidation, pulmonary oedema, pneumonia, serous exudation, fibrin exudation, pulmonary oedema, pneumocyte hyperplasia, large atypical pneumocytes, interstitial inflammation with lymphocytic infiltration and multinucleated giant cell formation, diffuse alveolar damage (DAD), diffuse alveolar exudates, acute respiratory distress syndrome (ARDS), severe hypoxemia, exudates in alveolar cavities and pulmonary interstitial fibrosis plasmocytosis in bronchoalveolar lavage (BAL), disseminated intravascular coagulation (DIC), leukoerythroblastic reaction, microvesicular steatosis in the liver, any symptom associated with COVID-19 and any combination thereof.

In yet another aspect the therapeutic antibody or antigen-binding fragment or a substantially similar variant thereof according to the present invention competes for binding to an epitope within an amino acid sequence selected from the group of SEQ ID No: 04 and/or SEQ ID No: 06 with an antibody selected from a group comprising a heavy chain variable region of the amino acid sequence in SEQ ID NO: 906 and a light chain variable region of the amino acid sequence in SEQ ID NO: 908; a heavy chain variable region of the amino acid sequence in SEQ ID NO: 926 and a light chain variable region of the amino acid sequence in SEQ ID NO: 928; a heavy chain variable region of the amino acid sequence in SEQ ID NO: 1066 and a light chain variable region of the amino acid sequence in SEQ ID NO: 1068; a heavy chain variable region of the amino acid sequence in SEQ ID NO: 1166 and a light chain variable region of the amino acid sequence in SEQ ID NO: 1168; a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In one embodiment said therapeutic antibody or antigen-binding fragment or a substantially similar variant thereof binds specifically to at least one epitope within the S1-7PM-mutant (SEQ ID NO.: 1730), selected from 365-YSFLY-369, 436-WNSKNLD-443, and/or 474-QASSTPCNGAKRVNCY-489 (the numbering of the residues corresponds to the numbering of the amino acids within the S1 domain of SARS-CoV-2 glycoprotein S, i.e. SEQ ID NO: 02).

In yet another embodiment said therapeutic antibody or antigen-binding fragment or a substantially similar variant thereof competes for binding to at least one epitope within the S1-7PM-mutant, selected from 365-YSFLY-369, 436-WNSKNLD-443, and/or 474-QASSTPCNGAKRVNCY-489 (the numbering of the residues corresponds to the numbering of the amino acids within the S1 domain of SARS-CoV-2 glycoprotein S, i.e. SEQ ID NO: 02) with the ACE2-rezeptor.

In yet another embodiment therapeutic said antibody or antigen-binding fragment or a substantially similar variant thereof binds specifically to and/or competes for binding to at least two and/or all three epitopes within the S1-7PM-mutant, selected from 365-YSFLY-369, 436-WNSKNLD-443, and/or 474-QASSTPCNGAKRVNCY-489 (the numbering of the residues corresponds to the numbering of the amino acids within the S1 domain of SARS-CoV-2 glycoprotein S, i.e. SEQ ID NO: 02) with the ACE2-rezeptor.

In yet another aspect said therapeutic antibody or antigen-binding fragment or a substantially similar variant thereof competes for binding to the epitope 483-VEGFNCYF-490 within the RBD of the S1 domain of SARS-CoV-2 glycoprotein S with an antibody comprising a heavy chain variable region of the amino acid sequence in SEQ ID NO: 926; and a light chain variable region of the amino acid sequence in SEQ ID NO: 928.

In yet another aspect said therapeutic antibody or antigen-binding fragment or a substantially similar variant thereof competes for binding to the epitope 483-VEGFNCYF-490 within the RBD of the S1 domain of SARS-CoV-2 glycoprotein S with an antibody selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another aspect said therapeutic antibody or antigen-binding fragment or a substantially similar variant thereof competes for binding to the epitope 437-NSNNLDSKVGGNYNYLYRLFRKSNLKP-463 within the RBD of the S1 domain of SARS-CoV-2 glycoprotein with an antibody comprising a heavy chain variable region of the amino acid sequence in SEQ ID NO: 926; and a light chain variable region of the amino acid sequence in SEQ ID NO: 928.

In yet another aspect said therapeutic antibody or antigen-binding fragment or a substantially similar variant thereof competes for binding to the epitope 437-NSNNLDSKVGGNYNYLYRLFRKSNLKP-463 within the RBD of the S1 domain of SARS-CoV-2 glycoprotein with an antibody selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another aspect said therapeutic antibody or antigen-binding fragment or a substantially similar variant thereof competes for binding to the epitope 400-FVIRGDEVRQIAPQTGKIADYN-422 within the RBD of the S1 domain of SARS-CoV-2 glycoprotein with an antibody comprising a heavy chain variable region of the amino acid sequence in SEQ ID NO: 926; and a light chain variable region of the amino acid sequence in SEQ ID NO: 928.

In yet another aspect said therapeutic antibody or antigen-binding fragment or a substantially similar variant thereof competes for binding to the epitope 400-FVIRGDEVRQIAPQTGKIADYN-422 within the RBD of the S1 domain of SARS-CoV-2 glycoprotein with an antibody selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another aspect said therapeutic antibody or antigen-binding fragment or a substantially similar variant thereof competes for binding to the epitope 470-TEIYQAGSTPC-480 within the RBD of the S1 domain of SARS-CoV-2 glycoprotein with an antibody comprising a heavy chain variable region of the amino acid sequence in SEQ ID NO: 926; and a light chain variable region of the amino acid sequence in SEQ ID NO: 928.

In yet another aspect said therapeutic antibody or antigen-binding fragment or a substantially similar variant thereof competes for binding to the epitope 470-TEIYQAGSTPC-480 within the RBD of the S1 domain of SARS-CoV-2 glycoprotein with an antibody selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another aspect said therapeutic antibody or antigen-binding fragment or a substantially similar variant thereof competes for binding to at least one or two or three or more of the epitopes selected from 483-VEGFNCYF-490, 437-NSNNLDSKVGGNYNYLYRLFRKSNLKP-463, 400-FVIRGDEVRQIAPQTGKIADYN-422, and/or 470-TEIYQAGSTPC-480 within the RBD of the S1 domain of SARS-CoV-2 glycoprotein S with an antibody comprising a heavy chain variable region of the amino acid sequence in SEQ ID NO: 926; and a light chain variable region of the amino acid sequence in SEQ ID NO: 928.

In yet another aspect said therapeutic antibody or antigen-binding fragment or a substantially similar variant thereof competes for binding to at least one or two or three or more of the epitopes selected from 483-VEGFNCYF-490, 437-NSNNLDSKVGGNYNYLYRLFRKSNLKP-463, 400-FVIRGDEVRQIAPQTGKIADYN-422, and/or 470-TEIYQAGSTPC-480 within the RBD of the S1 domain of SARS-CoV-2 glycoprotein S with an antibody selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another aspect said therapeutic antibody or antigen-binding fragment or a substantially similar variant thereof competes for binding to the S1-7PM-mutant (SEQ ID NO: 1730) of the S1 domain of SARS-CoV-2 glycoprotein with an antibody comprising a heavy chain variable region of the amino acid sequence in SEQ ID NO: 926; and a light chain variable region of the amino acid sequence in SEQ ID NO: 928.

In yet another aspect said therapeutic antibody or antigen-binding fragment or a substantially similar variant thereof competes for binding to the S1-7PM-mutant (SEQ ID NO: 1730) of the S1 domain of SARS-CoV-2 glycoprotein with an antibody selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another embodiment said therapeutic antibody or antigen-binding fragment or a substantially similar variant thereof competes for binding to at least two and/or all three epitopes within the S1-7PM-mutant, selected from 365-YSFLY-369, 436-WNSKNLD-443, and/or 474-QASSTPCNGAKRVNCY-489 (the numbering of the residues corresponds to the numbering of the amino acids within the S1 domain of SARS-CoV-2 glycoprotein S, i.e. SEQ ID NO: 02) with an antibody comprising a heavy chain variable region of the amino acid sequence in SEQ ID NO: 926; and a light chain variable region of the amino acid sequence in SEQ ID NO: 928.

In yet another embodiment said therapeutic antibody or antigen-binding fragment or a substantially similar variant thereof competes for binding to at least two and/or all three epitopes within the S1-7PM-mutant, selected from 365-YSFLY-369, 436-WNSKNLD-443, and/or 474-QASSTPCNGAKRVNCY-489 (the numbering of the residues corresponds to the numbering of the amino acids within the S1 domain of SARS-CoV-2 glycoprotein S, i.e. SEQ ID NO: 02) with an antibody selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

In yet another aspect said therapeutic antibody or antigen-binding fragment or a substantially similar variant thereof binds specifically to at least one, or two, or three, or more of the epitopes selected from 483-VEGFNCYF-490, 437-NSNNLDSKVGGNYNYLYRLFRKSNLKP-463, 400-FVIRGDEVRQIAPQTGKIADYN-422, and/or 470-TEIYQAGSTPC-480 within the RBD of the S1 domain of SARS-CoV-2 glycoprotein, and binds specifically to at least one, or two, or three, or more of the epitopes selected from 483-VEGFNCYF-490, 437-NSNNLDSKVGGNYNYLYRLFRKSNLKP-463, 400-FVIRGDEVRQIAPQTGKIADYN-422, and/or 470-TEIYQAGSTPC-480.

In yet another aspect said therapeutic antibody or antigen-binding fragment or a substantially similar variant thereof competes for binding to at least one, or two, or three, or more of the epitopes selected from 483-VEGFNCYF-490, 437-NSNNLDSKVGGNYNYLYRLFRKSNLKP-463, 400-FVIRGDEVRQIAPQTGKIADYN-422, and/or 470-TEIYQAGSTPC-480 within the RBD of the S1 domain of SARS-CoV-2 glycoprotein, and competes for binding to at least one, or two, or three, or more of the epitopes selected from 483-VEGFNCYF-490, 437-NSNNLDSKVGGNYNYLYRLFRKSNLKP-463, 400-FVIRGDEVRQIAPQTGKIADYN-422, and/or 470-TEIYQAGSTPC-480 with an antibody comprising a heavy chain variable region of the amino acid sequence in SEQ ID NO: 926; and a light chain variable region of the amino acid sequence in SEQ ID NO: 928.

In yet another aspect the therapeutic antibody or antigen-binding fragment or a substantially similar variant thereof according to the present invention comprises at least one of the CDRs selected from

a heavy chain CDR1 (H-CDR1) domain with an amino acid sequence of SEQ ID NO: 1306;

a heavy chain CDR2 (H-CDR2) domain with an amino acid sequence of SEQ ID NO: 1307;

a heavy chain CDR3 (H-CDR3) domain with an amino acid sequence of SEQ ID NO: 1308;

and

a light chain CDR1 (L-CDR1) domain with an amino acid sequence of SEQ ID NO: 1310;

a light chain CDR2 (L-CDR2) domain with an amino acid sequence of SEQ ID NO: 1311;

a light chain CDR3 (L-CDR3) domain with an amino acid sequence of SEQ ID NO: 1312.

In yet another aspect of the invention said therapeutic antibody or antigen-binding fragment or a substantially similar variant thereof competes for binding to an epitope within the receptor binding domain (RBD) of the S1 domain of SARS-CoV-2 glycoprotein (SEQ ID NO: 06) and binding specifically to the S1-7PM-mutant of SEQ ID No: 1730, with an antibody comprising a heavy chain variable region of the amino acid sequence in SEQ ID NO: 1305; and a light chain variable region of the amino acid sequence in SEQ ID NO: 1309 or with an antibody comprising a heavy chain variable region of the amino acid sequence in SEQ ID NO: 926 and a light chain variable region of the amino acid sequence in SEQ ID NO: 928.

In yet another aspect of the invention said therapeutic antibody or antigen-binding fragment or a substantially similar variant thereof competes for binding to the epitope 483-VEGFNCYFPLQSYGFQPTNGV-505 within the RBD (SEQ ID NO.: 6) and to the S1-7PM-protein of SEQ ID No.: 1730 with an antibody comprising a heavy chain variable region of the amino acid sequence in SEQ ID NO: 1305; and a light chain variable region of the amino acid sequence in SEQ ID NO: 1309 or with an antibody comprising a heavy chain variable region of the amino acid sequence in SEQ ID NO: 926 and a light chain variable region of the amino acid sequence in SEQ ID NO: 928.

In yet another aspect of the invention the therapeutic antibody or antigen-binding fragment or a substantially similar variant thereof binds specifically to the epitope 437-NSNNLDSKVGGNYNYLYRLFRKSNLKP-463 within the RBD (SEQ ID NO.: 6) and competes for binding to 483-VEGFNCYF-490 within the RBD of the S1 domain of SARS-CoV-2 glycoprotein and binds specifically to the S1-7PM-mutant (SEQ ID NO: 1730) of the S1 domain of SARS-CoV-2 glycoprotein.

In yet another aspect of the invention the therapeutic antibody or antigen-binding fragment or a substantially similar variant thereof binds specifically to the epitope 437-NSNNLDSKVGGNYNYLYRLFRKSNLKP-463 within the RBD (SEQ ID NO.: 6) with a $K_D$ of 70 nM or less ("wild-type" RBD-mFc (i.e. RBD without point mutations) immobilized on an anti-Fmc capture surface) and shows a contamination ratio of 0.2 or less.

In yet another aspect of the invention the therapeutic antibody or antigen-binding fragment or a substantially similar variant thereof binds specifically to the RBD of the S1 domain of SARS-CoV-2 glycoprotein (SEQ ID NO.: 6) with a $K_D$ of 70 nM or less ("wild-type" RBD-mFc (i.e. RBD without point mutations) immobilized on an anti-Fmc capture surface) and shows a contamination ratio of 0.2 or less.

In yet another aspect of the invention the therapeutic antibody or antigen-binding fragment or a substantially similar variant thereof binds specifically to the RBD of the S1 domain of SARS-CoV-2 glycoprotein (SEQ ID NO.: 6) with a $K_D$ of 20 nM or less ("wild-type" RBD-mFc (i.e. RBD without point mutations) immobilized on an anti-Fmc capture surface) and shows a contamination ratio of 0.1 or less.

In yet another aspect of the invention the therapeutic antibody or antigen-binding fragment or a substantially similar variant thereof binds specifically to the RBD of the S1 domain of SARS-CoV-2 glycoprotein (SEQ ID NO.: 6) with a $K_D$ of 70 nM or less ("wild-type" RBD-mFc (i.e. RBD without point mutations) immobilized on an anti-Fmc capture surface) and binds specifically to the S1-7PM-mutant (SEQ ID NO: 1730) with a $K_D$ of 70 nM or less and shows a contamination ratio of 0.2 or less.

In yet another aspect of the invention the therapeutic antibody or antigen-binding fragment or a substantially similar variant thereof binds specifically to the RBD of the S1 domain of SARS-CoV-2 glycoprotein (SEQ ID NO.: 6) with a $K_D$ of 10 nM or less ("wild-type" RBD-mFc (i.e. RBD without point mutations) immobilized on an anti-Fmc capture surface) and binds specifically to the S1-7PM-mutant (SEQ ID NO: 1730) with a $K_D$ of 45 nM less (antibody against S1-7PM was immobilized on a Protein A coated biosensor) and shows a contamination ratio of 0.1 or less.

In yet another aspect of the invention the therapeutic antibody or antigen-binding fragment or a substantially similar variant thereof binds specifically to the epitope 437-NSNNLDSKVGGNYNYLYRLFRKSNLKP-463 within the RBD (SEQ ID NO.: 6) with a $K_D$ of 70 nM or less ("wild-type" RBD-mFc (i.e. RBD without point mutations) immobilized on an anti-Fmc capture surface) and binds specifically to the S1-7PM-mutant (SEQ ID NO: 1730) with a $K_D$ of 45 nM less (antibody against S1-7PM was immobilized on a Protein A coated biosensor) and shows a contamination ratio of 0.1 or less.

In yet another aspect therapeutic antibody or antigen-binding fragment or a substantially similar variant thereof is characterized by one or more of the following:

- capable of binding specifically to the RBD of SARS-CoV-2 Glycoprotein S with an affinity (KD) of 6.5 x 10-8 M or less, preferably less than 10 x 10-9 M,

- capable of reducing or preventing an antibody-dependent enhancement (ADE) reaction in a patient,

- capable of preventing, reducing or healing a viral infection with SARS-CoV-2,

- capable of reducing or preventing the cytokine storm syndrome (CSS) in a patient,

- capable of reducing the load of viral particles in the sputum of a patient by at least 50% for at least 48 hours after administration of the therapeutic antibody or antigen-binding fragment thereof,

- having a specific binding specifically to the S1-7PM-mutant (SEQ ID. 1730) with a KD of less than 3 x 10-9 M,

- having an IC50 of less than 0.036 μg/ml, preferably of 0.026 μg/ml or less.

In another aspect the present invention pertains to an isolated nucleic acid molecule encoding said therapeutic antibody or antigen-binding fragment or a substantially similar variant thereof.

In another aspect the present invention pertains to an expression vector comprising said nucleic acid molecule.

In another aspect the present invention pertains to a method of producing the therapeutic antibody or antigen-binding fragment or a substantially similar variant thereof comprising the steps of

introducing said expression vector according into an isolated host cell,

growing the cell under conditions permitting production of the antibody or antigen-binding fragment thereof,

recovering the antibody or fragment so produced.

In one embodiment the antibody may be produced in the animal and/or human body, e.g. in a body cell, by using for example a stabilized mRNA, DNA, plasmid, viral vector or other vector enabling the production of the therapeutic antibody or antigen-binding fragment or a substantially similar variant thereof within the animal or human body.

In another aspect the present invention pertains to a pharmaceutical composition comprising the therapeutic antibody or antigen-binding fragment or a substantially similar variant, and a pharmaceutically acceptable carrier.

In another aspect the present invention pertains to a therapeutic antibody or antigen-binding fragment for the use in the prevention and/or treatment of a SARS-CoV-2-related disease or condition in a patient.

Short Description of the Figures

Figure 1a - Representative depiction of a SEC-HLPC-measurement of a therapeutic antibody according to the present invention. A single peak indicates a high purity (i.e. low contamination) of the antibody. In the depiction is only the peak of the monomer visible.

Figure 1b - Representative depiction of a SEC-HLPC-measurement of a regular antibody in the prior art. Several peaks indicate the contamination with multimers and dimers besides the monomer. Such an antibody would not be suitable to be used as a therapeutic antibody due to undesired stimulatory effects on the host's immune system.

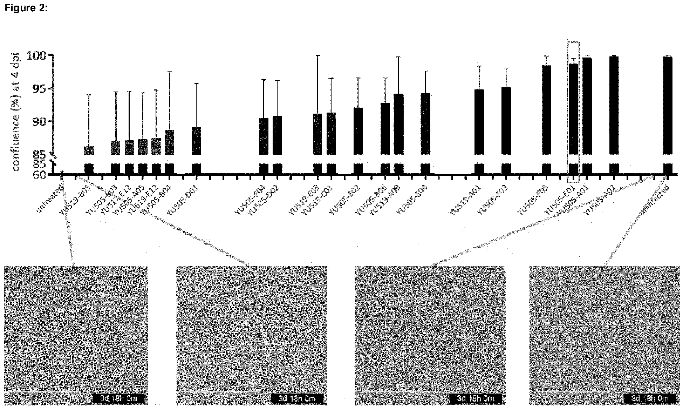

Figure 2 - Result of an infectious SARS-CoV-2 neutralizing assay (Confluent VERO E6-6-Test) with different antibodies. The higher the confluence the better the neutralizing activity of the antibody. For therapeutic purposes a confluence of at least 95% is desired.

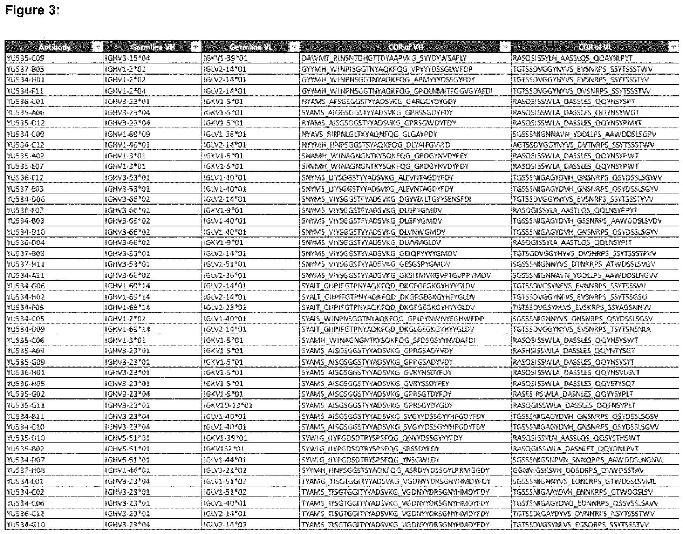

Figure 3 - List of some of the antibodies of the present invention with germline VH and VL as well as their CDR-sequences. Depicted are *inter alia* the therapeutic antibodies: YU505-A01, YU505-A02, YU505-E01, YU505-F05, YU534-C12, YU534-D09, YU535-A02, YU536-D04, YU537-H08 and YU537-H11. YU505-A02, YU536-D04 and YU537-H11 are preferred therapeutic antibodies.

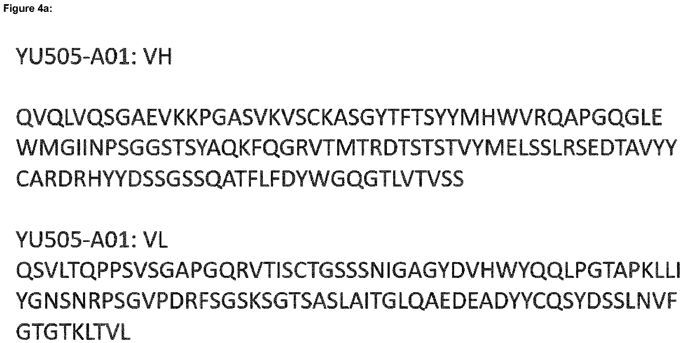

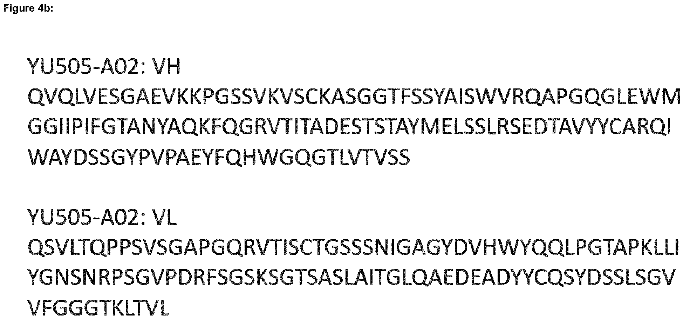

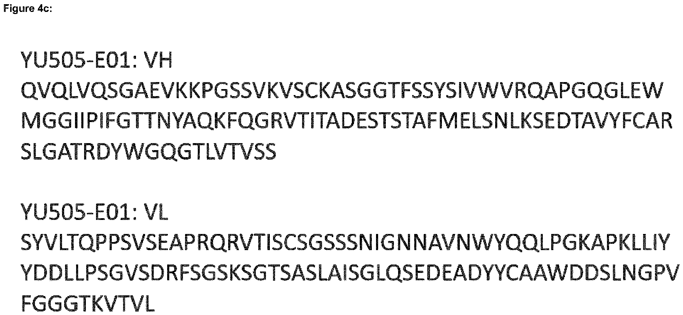

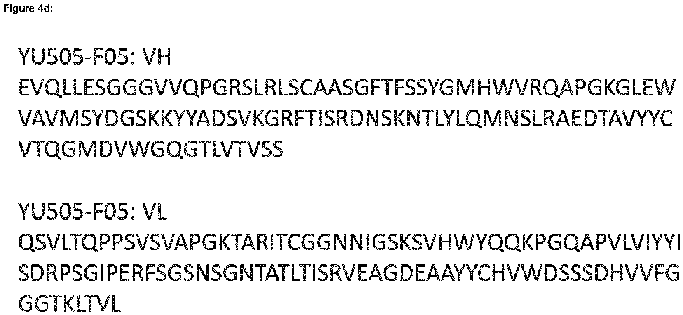

Figure 4 - Representation of the amino acid sequence of the VL and VH of the therapeutic antibodies YU505-A01 (a), YU505-A02 (b), YU505-E01 (c) and YU505-F05 (d). YU505-A02 (b) is useful both as a therapeutic antibody as well as a reference antibody. Any antibody competing with YU505-A02 (b) to the target epitope(s) is to be considered a useful therapeutic antibody according to the present invention.

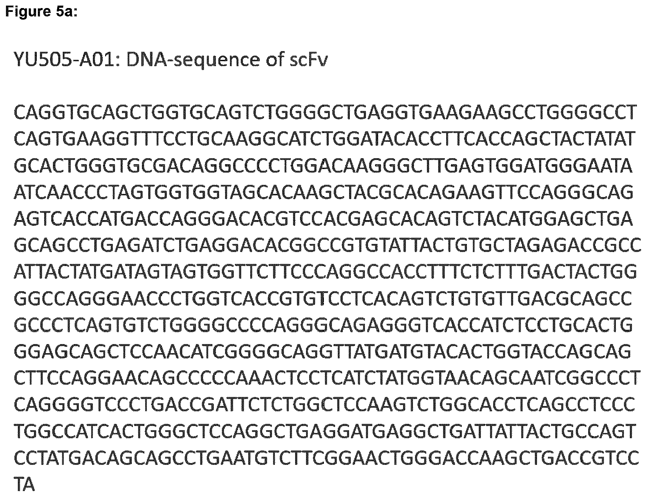

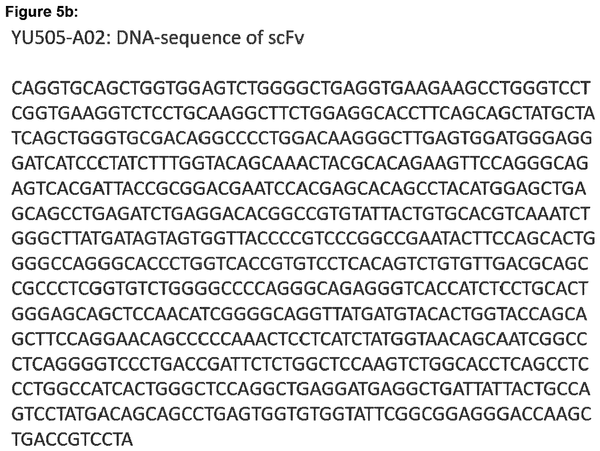

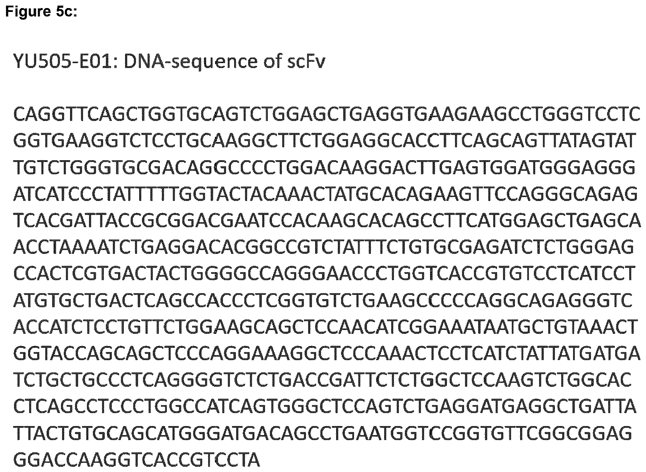

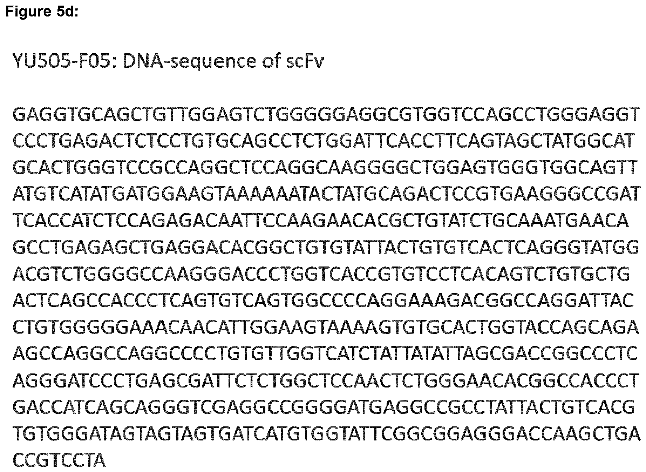

Figure 5 - Representation of the scFv-sequences of antibody-fragments from YU505-A01 (a), YU505-A02 (b), YU505-E01 (c) and YU505-F05 (d).

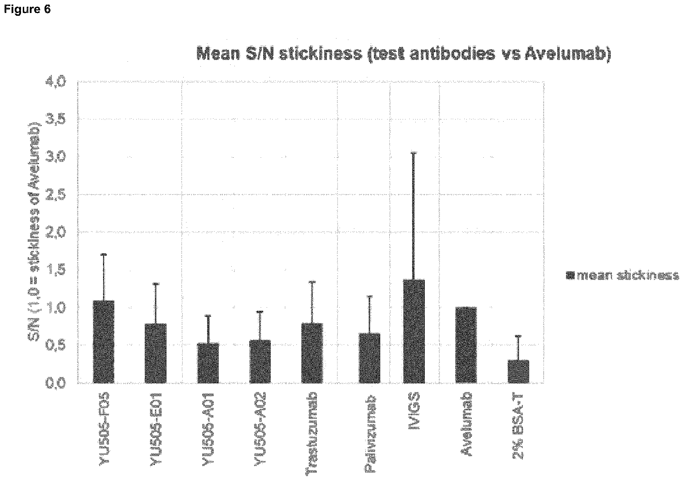

Figure 6 - Representation of a test for "stickyness"of several therapeutic antibodies as compared with known antibodies. The lower the signal the smaller is the unspecific binding of the antibodies to undesired targets.

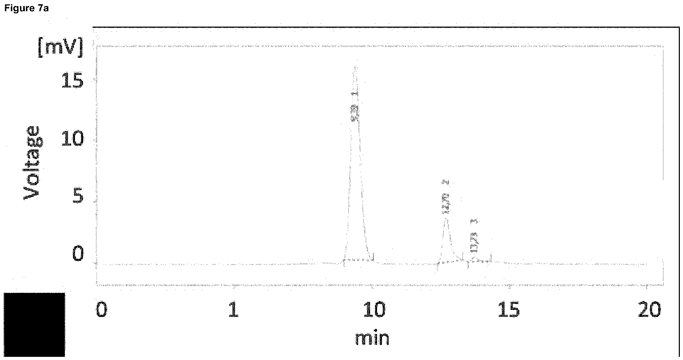

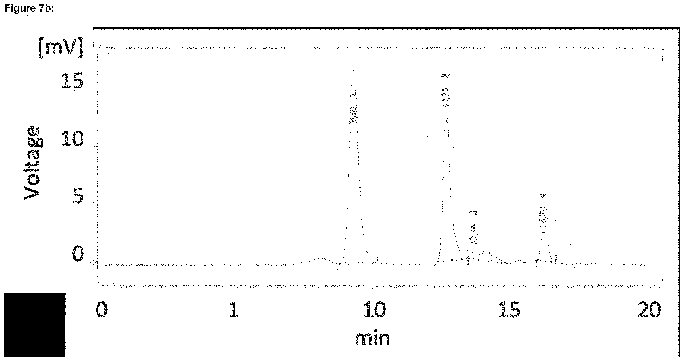

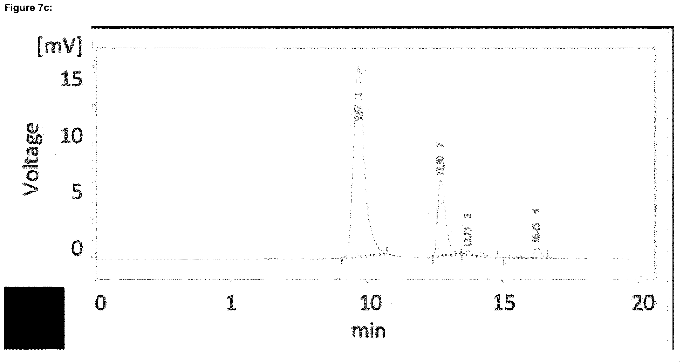

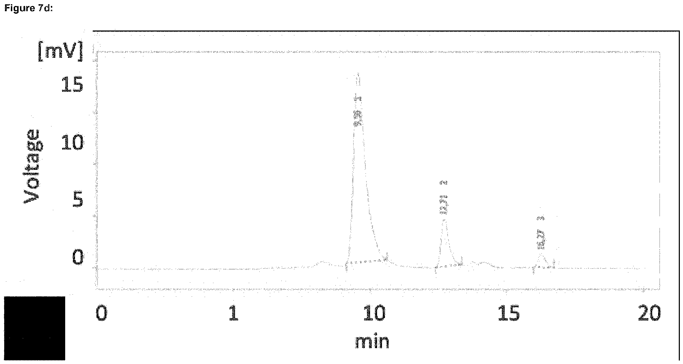

Figure 7 - Representation of a SEC-HPLC-measurement of the therapeutic antibodies YU505-A01 (a), YU505-A02 (b), YU505-E01 (c) and YU505-F05 (d). All antibodies show a high purity with a contamination ratio of well below 1. The peaks at time-points > 10 are due to impurities of the column and not associated with the antibodies.

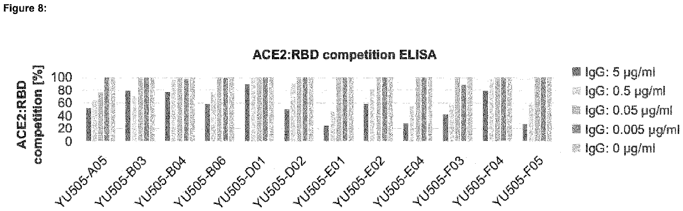

Figure 8 - Exemplary results of ACE2:RBD competition assay of some antibodies of the present invention at different concentrations. 100% represents no competition, 0% represents full competition. A competition of less than 70% at 0.5 $\mu$g/ml was considered necessary to qualify an antibody with sufficient activity for further testing.

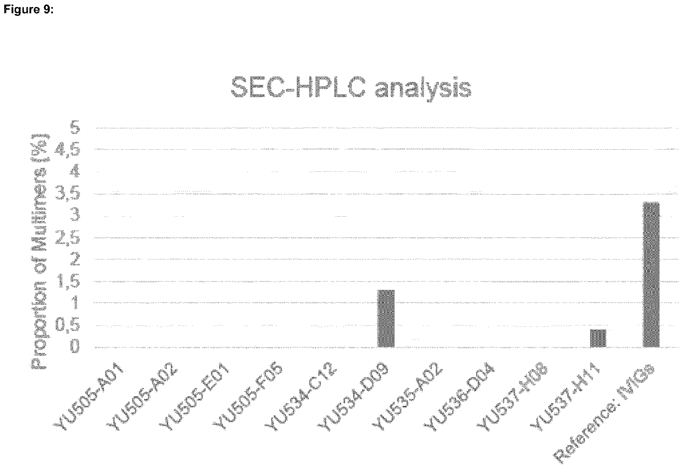

Figure 9 - Depiction of the contamination ratio (i.e. multimerization) of a selection of antibodies of the present invention. Measurement was done by SEC-HPLC and the multimerization was compared with IVIGs. A contamination ratio of less than 1 was considered necessary to qualify an antibody for further testing.

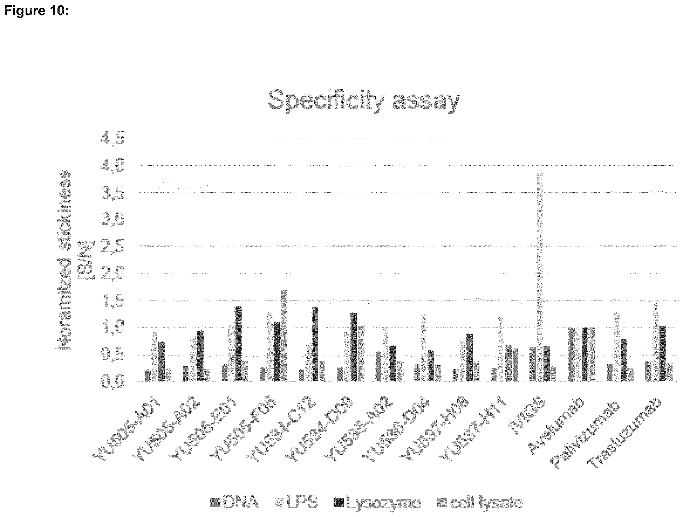

Figure 10 - Depiction of the specificity of a selection of therapeutic human antibodies according to the invention. Note: The binding specifically to the different negative antigens was normalized to the binding of avelumab (a fully human monoclonal antibody medication for the treatment of Merkel cell carcinoma, urothelial carcinoma, and renal cell carcinoma).

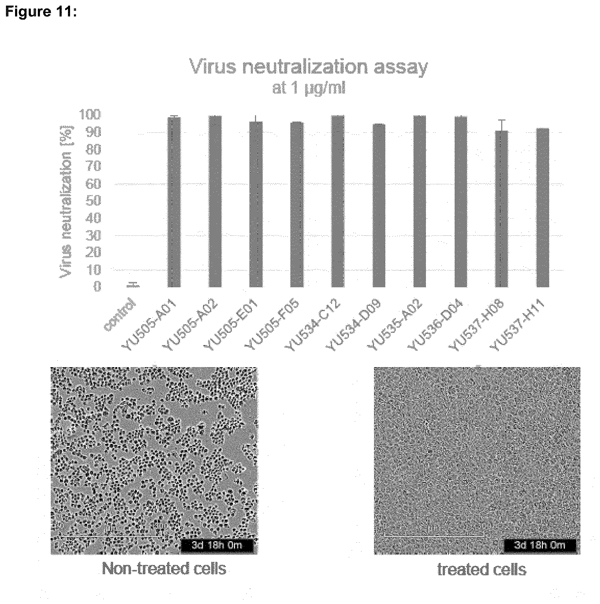

Figure 11 - Depiction of effective neutralization of the virus and blocking of the entry of virus into mammalian cells at 1 $\mu$g/ml with different antibodies of the present invention. All depicted antibodies show sufficient neutralization to be considered for therapeutic use.

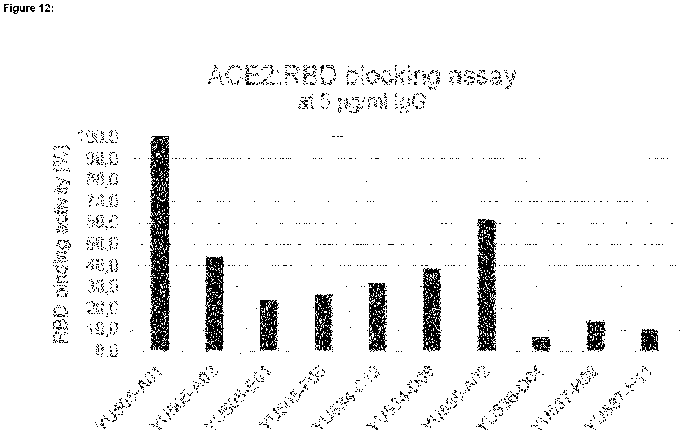

Figure 12 - Depiction of the effective blocking of the ACE2:RBD interaction site for a selection of inventive antibodies (all inventive antibodies were tested, data not shown). A blocking of at least 50% of the ACE2:RBD interaction site was considered necessary to qualify an antibody for further testing.

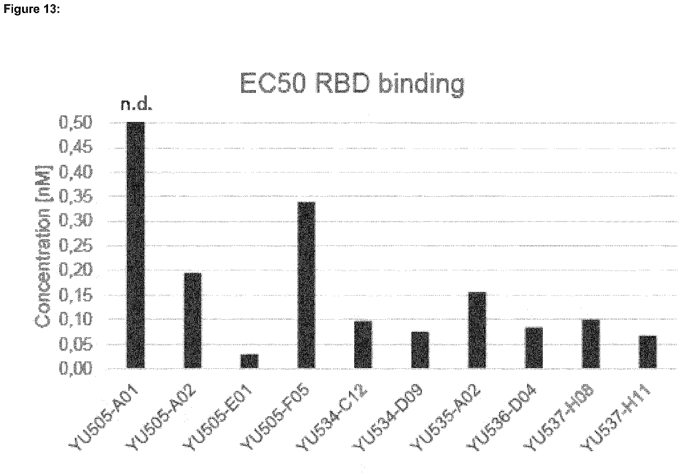

Figure 13 - Depiction of the effective $IC_{50}$-binding specifically to the RBD in subnanomolar range for a selection of inventive antibodies (all inventive antibodies were tested, data not shown). An IC50 of less than 0,2 nM was considered necessary to qualify an antibody for further testing. For YU505-A01 no saturation ELISA curve was detected.

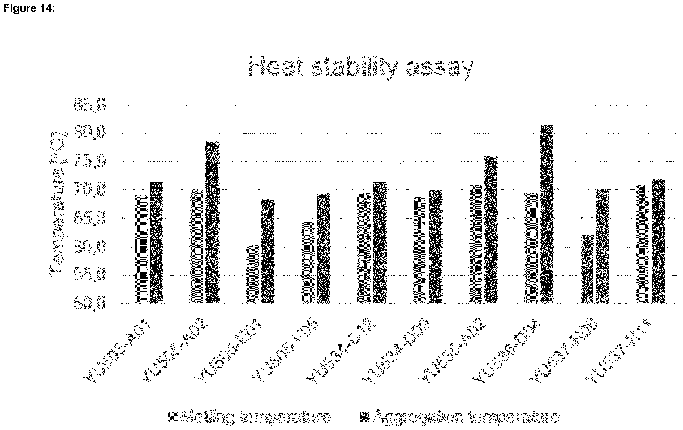

Figure 14 - Depiction of the heat stability test determined by NanoDSF for a selection of inventive antibodies (all inventive antibodies were tested, data not shown). Melting T ranges from about 60°C to about 70°C. Aggregation T ranges from about 68°C to 81 °C. An melting T of at least 65°C and aggregation T of at least 70°C was considered necessary to qualify an antibody for further testing.

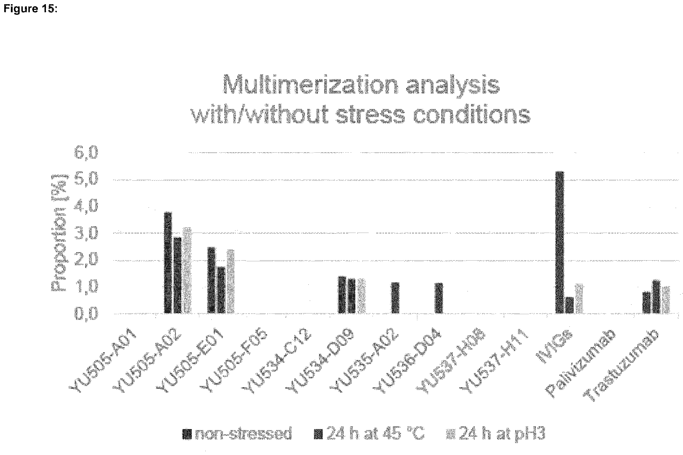

Figure 15 - Depiction of the multimer-formation under stress conditions for a selection of inventive antibodies (all inventive antibodies were tested, data not shown). No increased multimer-formation of above 4% could be detected showing the overall suitability in terms of purity and stability of the inventive antibodies..

Figure 16 - $IC_{50}$ measurement in a VERO E6 cell assay for a number of antibodies according to the present invention (all inventive antibodies were tested, data not shown). YU536-D04 and YU537-H11 show a preferred $IC_{50}$-profile.

Figure 17 - Table depicting the results of the $IC_{50}$ measurement in a VERO E6 cell assay for a number of antibodies according to the present invention (all inventive antibodies were tested, data not shown). An IC50 of less than 0.05 was considered necessary to qualify an antibody for further testing.

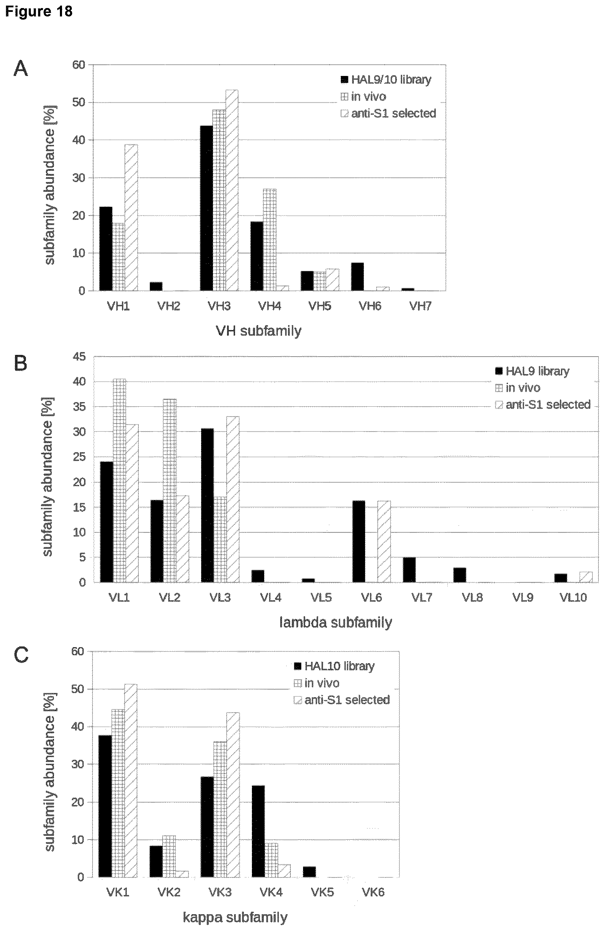

Figure 18 - Depiction of the antibody subfamily distribution in the universal HAL9/10 library (according to Kügler et al., 2015), the in vivo distribution of subfamilies (according to Tiller et al., 2013) and the distribution of antibodies against S1 selected from HAL9/10. (A) Abundance of VH. (B) Abundance of $V_K$. (C) Abundance of V$\lambda$.

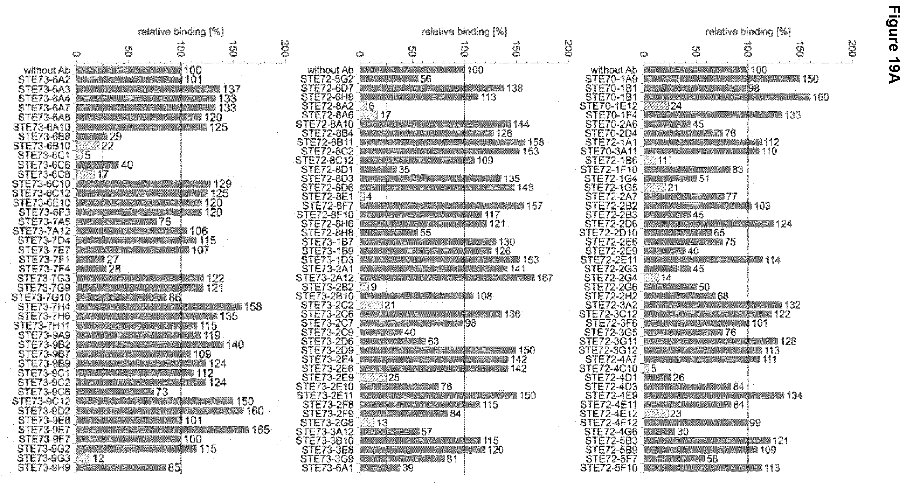

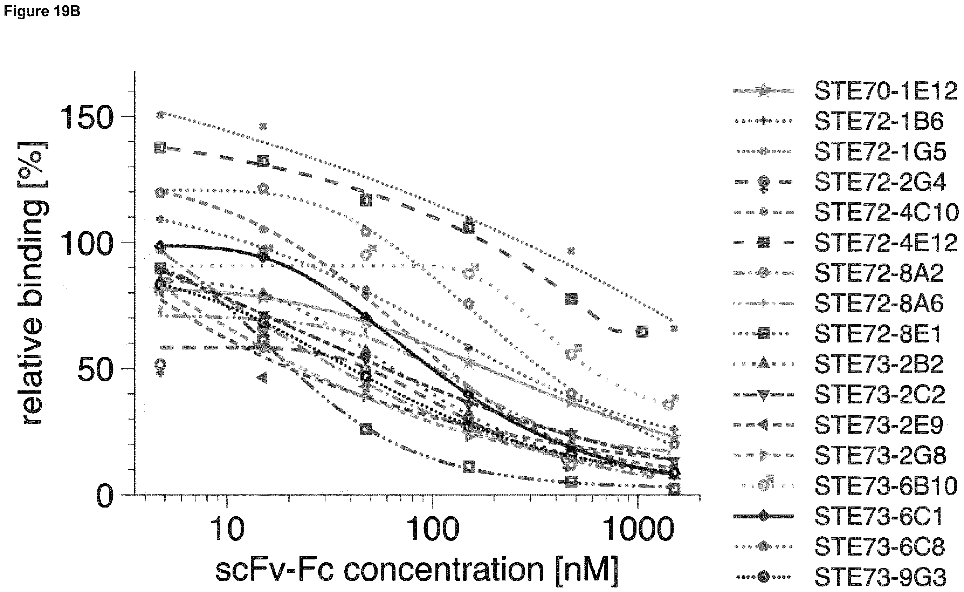

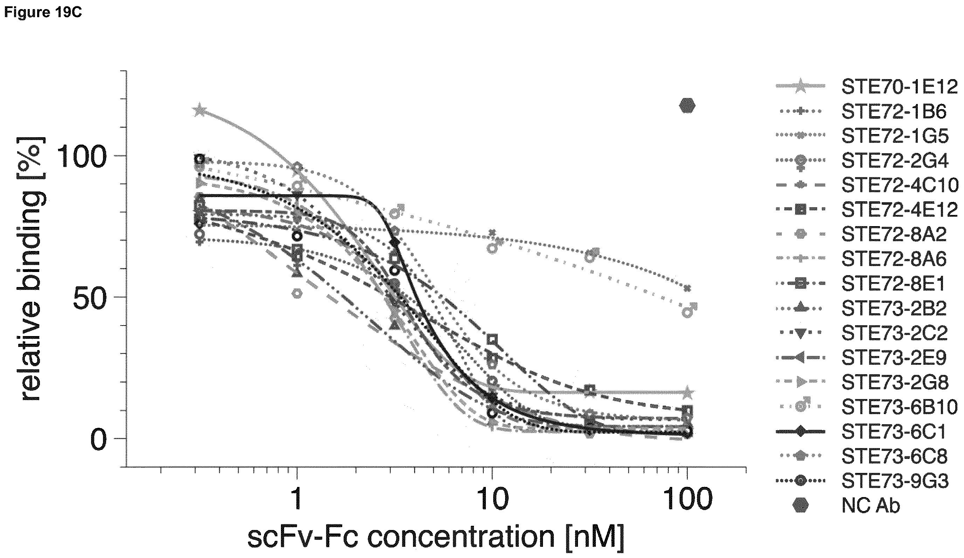

Figure 19 - Depiction of the flow cytometry analysis to determine the inhibition efficacy. A) screening analysis to determine the inhibition efficacy of scFv-Fc antibodies on ACE2 positive cells using 1500 nM antibody and 50 nM spike protein (30:1 ratio). The antibodies chosen for later analysis are marked in colors. The antibodies STE73-9-G3 and STE73-7H10 (marked with *) were identical. B) $IC_{50}$ determination by flow cytometry using 50 nM S1-S2 trimer and 4.7 - 1500 nM antibodies. C) $IC_{50}$ determination by flow cytometry using 10 nM RBD and 0.03-1000 nM antibodies.

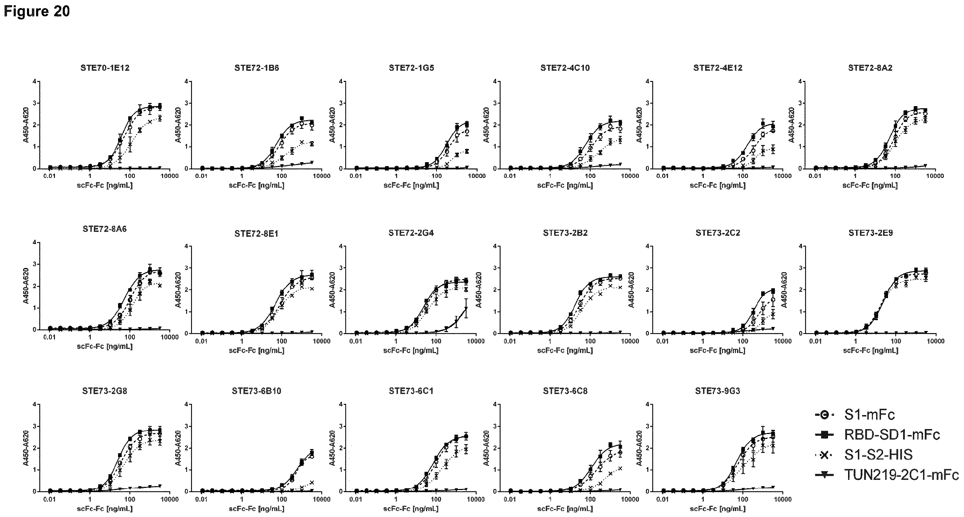

Figure 20 - The binding of 17 inhibiting antibodies were tested on RBD (with murine Fc part), S1 (with murine Fc part) or S1-S2 (with His tag) in titration ELISA to determine the $EC_{50}$. As controls, a control antibody with a murine Fc part (an antibody which binds specifically to c-myc), human HEK293 cell lysate, BSA and lysozyme was used.

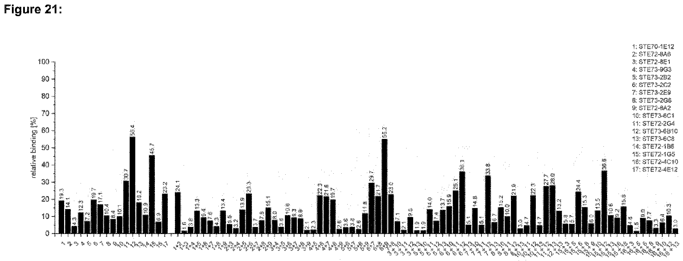

Figure 21 - Depiction of the flow cytometry analysis to determine the inhibition efficacy of antibody combinations on ACE2 positive cells and 50 nM S1-S2 and 1500 nM as a single antibody, respectively 750 nM of each antibody in a combination.

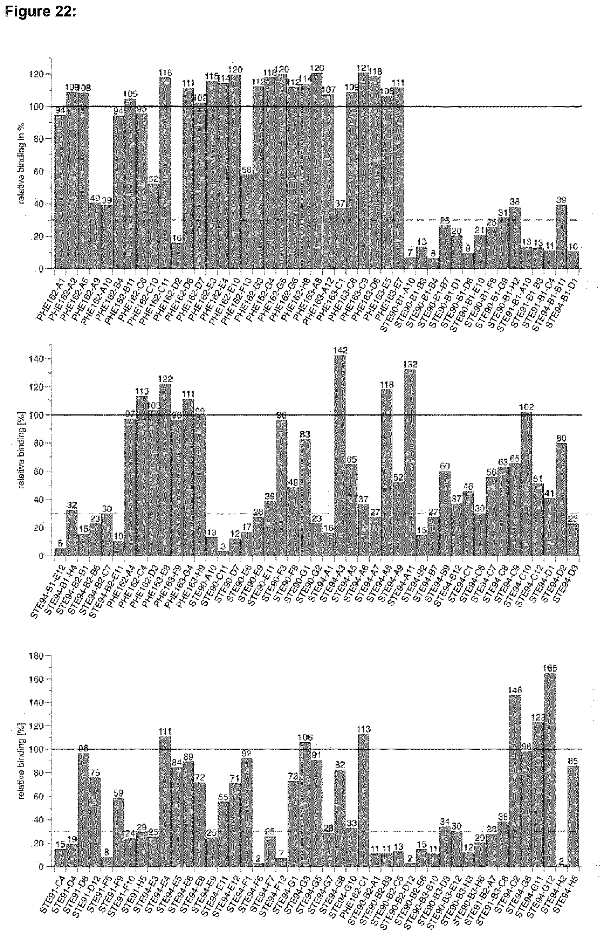

Figure 22 - Depiction of the flow cytometry analysis to determine the inhibition efficacy.

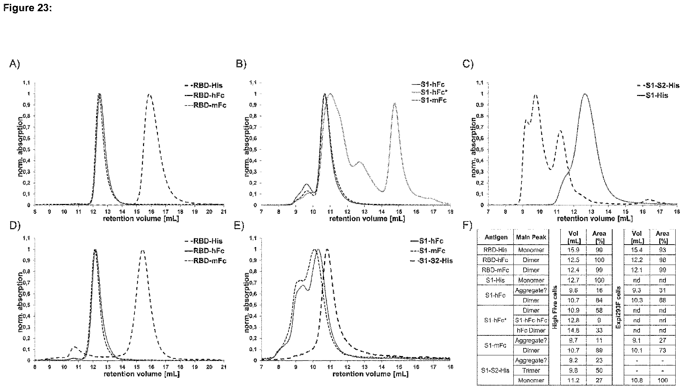

Figure 23 - Depiction of the SEC data of antigen production in insect High Five cells (A-C) and mammalian Expi293F cells (D-E). The table (F) indicates the most likely conformation of the protein due to the retention volume of the peaks with the corresponding area in percentage.

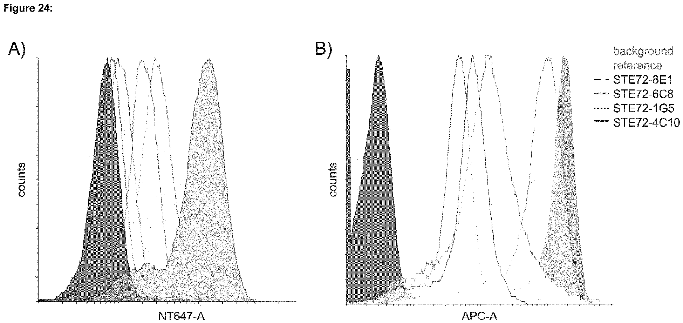

Figure 24 - A) Depiction of the flow cytometry inhibition analysis of spike S1-S2 trimer (50 nM in relation to monomer) binding specifically to living cells expressing ACE2 blocked by 1500 nM antibodies and B) Depiction of RBD-mFc (10 nM in relation to monomer) binding specifically to living cells expressing ACE2 blocked by 100 nM antibody. The antibodies STE72-1G5, STE72-4C10, STE72-8E1 and STE73-6C8 were used as example. The background control are transfected ACE2 cells and the reference are ACE2 positive cells incubated with labeled spike protein.

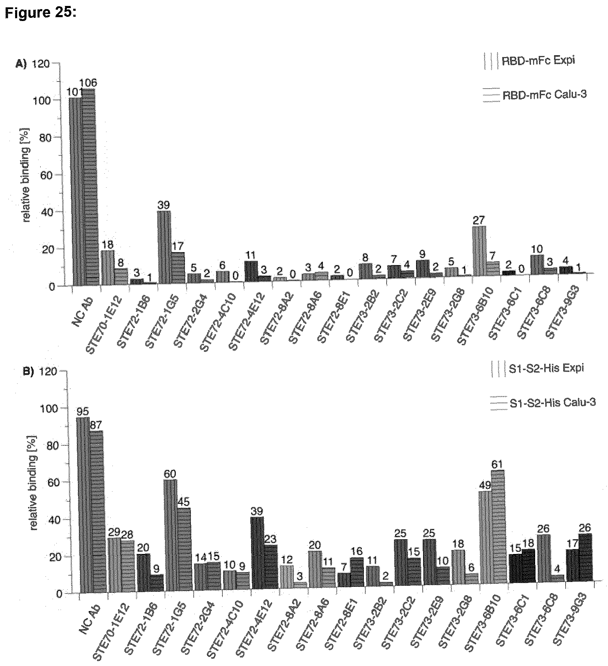

Figure 25 - Depiction of a flow cytometry inhibition analysis. A) Comparison of the inhibition of spike protein and RBD by flow cytometry on ACE2 positive Expi293F cells using 1000 nM scFv-Fc and 50 nM spike protein, respectively RBD (20:1 ratio). B) Inhibition of RBD binding in comparison of Expi293F transiently expressing ACE2 and Calu-3 cells. C) Inhibition of spike S1-S2 binding in comparison of Expi293F transiently expressing ACE2 and Calu-3. As negative control, an antibody which binds specifically to N-cadherin of *Danio rerio* was used.

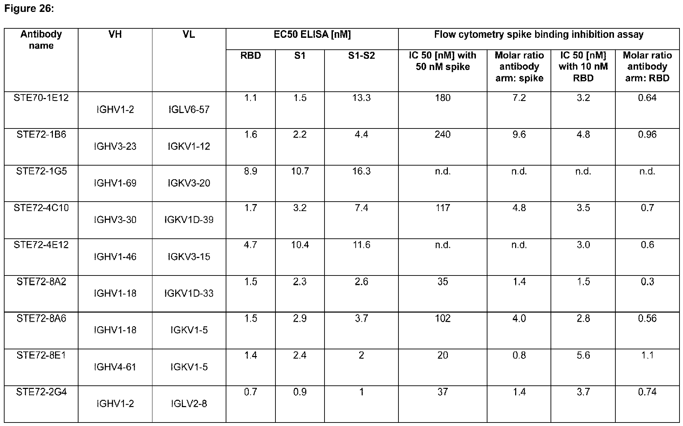

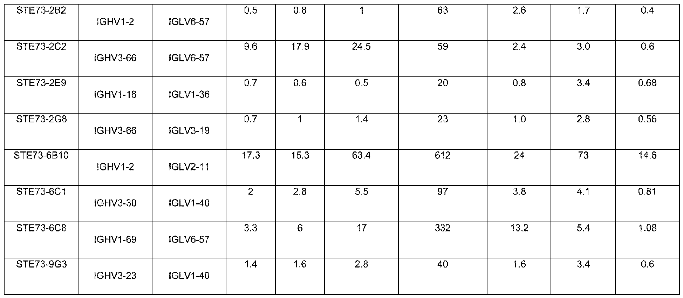

Figure 26 - Overview on inhibiting antibodies. V-genes were determined by VBASE2 (vbase2.org) (Mollova et al., 2010). The $EC_{50}$ were measured on 30 ng immobilized RBD-mFc, S1-mFc, S1-S2-His (trimer) by ELISA. The $IC_{50}$ was measured by flow cytometry using 50 nM (in relation to monomer) S1-S2 trimer, respectively 10 nM RBD and ACE2 positive cells. The molar ration of antibody binding site: S1-S2 or RBD is given for 50% inhibition. N.D.: not determinable.

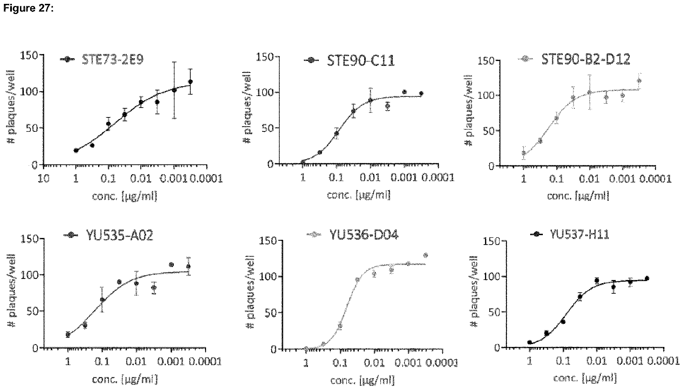

Figure 27 - Depiction of the result of an infectious SARS-CoV-2 neutralizing assay (Confluent VERO E6-6-Test) with different antibodies

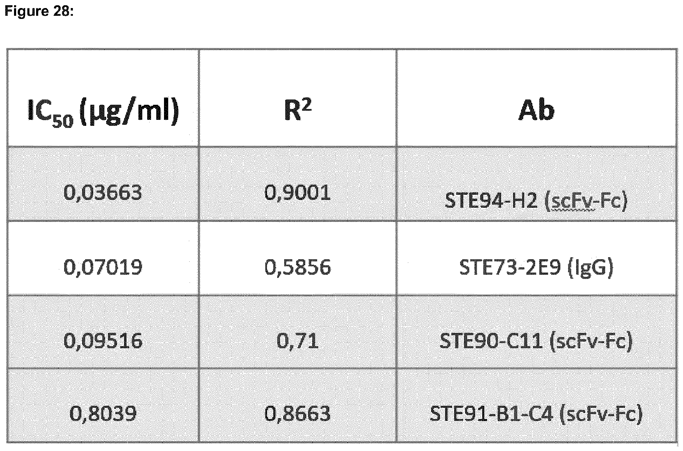

Figure 28 - Table depicting the results of the $IC_{50}$ measurement in a VERO E6 cell assay for a number of antibodies according to the present invention.

Figure 29 - $IC_{50}$ measurement in a VERO E6 cell assay for a number of antibodies according to the present invention. STE90-C11 shows a preferred $IC_{50}$-profile.

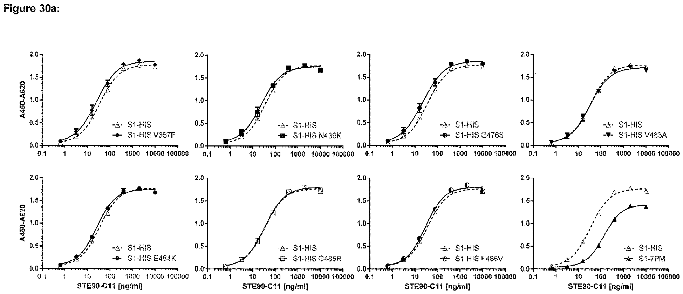

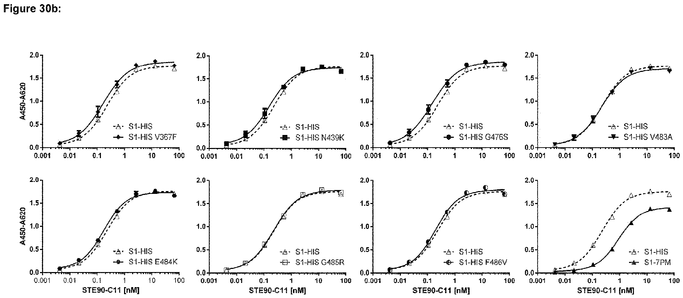

Figure 30 - A) The result of the binding study according to example 11 with antibody STE90-C11 of the present invention to the 8 "escape"-mutants. The binding was also compared to other reported antibodies CB6, RGN10933, RGN10987, CR3022. It could be shown that the inventive antibody STE90-C11 shows significant better binding specifically to all 7 single-point "escape"-mutants as compared to reference antibodies, and diminished, but still detectable binding specifically to the seven point mutation "S1-7PM". B) Same measurement but the depiction is in nM-concentration.

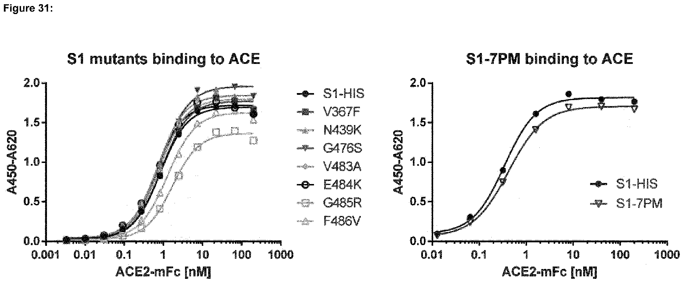

Figure 31 - The result of the binding study of the S1-protein with the seven single-point "escape" mutants as well as the seven point mutation "S1-7PM" to the ACE2-domain. This shows that all "escape"-mutants still bind to ACE2 and therefore are able to infect host-cells despite a significant altered RBD.

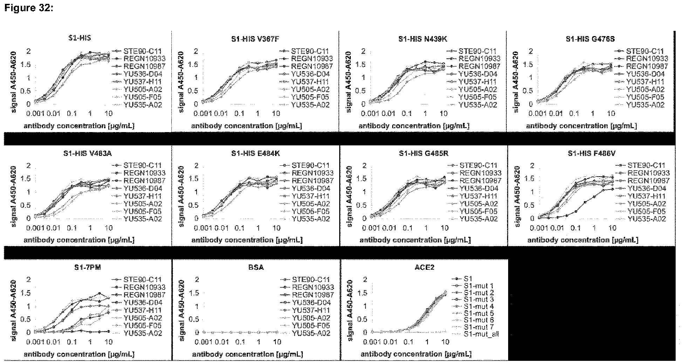

Figure 32 - The result of the binding study according to example 11 with a selection of antibodies of the present invention to the 8 "escape"-mutants. The binding was also compared to other reported antibodies CB6, RGN10933, RGN10987, CR3022. It could be shown that the inventive antibodies show significant better binding specifically to all 7 single-point "escape"-mutants as compared to reference antibodies, and partially diminished, but still detectable binding specifically to the seven point mutation "S1-7PM".

Figure 33 - Depiction of the results of the "binning study" according to example 11. "COM1", "COM2" and "COM3" refer to different bins ("BIN 1", "BIN 2", "BIN 3"), i.e. antibodies belonging to the same COM or bin compete with each other. All preferred therapeutic antibodies belong to "COM1" and compete with reference antibody YU505-A02.

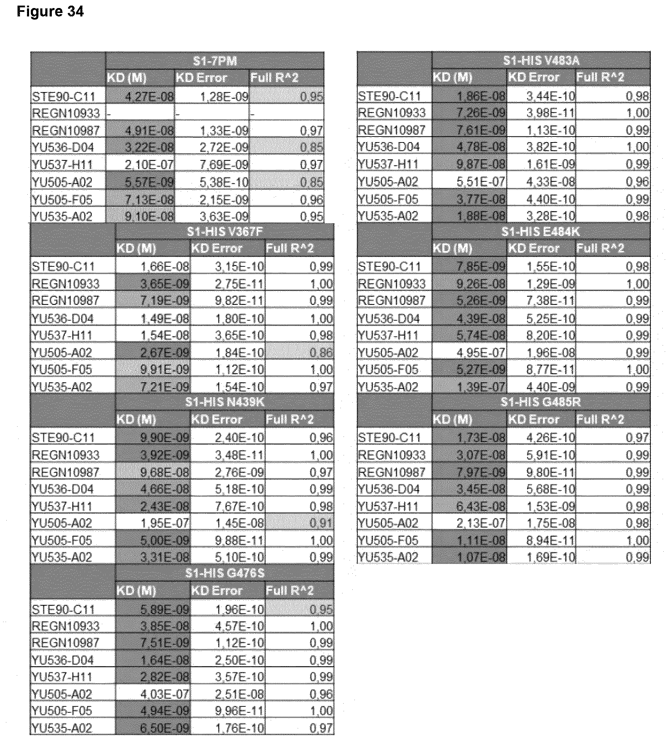

Figure 34 - Depiction of the results of $K_D$ - measurements of a number of antibodies according to the present invention as well as two comparative antibodies against different point mutations as well as the S1-7PM-mutation. The measurements were done as described in example 13 (BLI), the antibody was immobilized on a Protein A coated biosensor.

Examples

**Example 1: Antigen production**

As antigens of the full-length SARS-CoV-2 glycoprotein S (Spike) (SEQ ID NOs: 01 & 02), full-length S1-subunit of the SARS-CoV-2 glycoprotein S (SEQ ID NOs: 03 & 04), full-length of receptor binding domain (RBD) of the S1-subunit of SARS-CoV-2 glycoprotein S (SEQ ID NOs: 05 & 06), and the extra-cellular domain of human ACE2 (full-length human ACE2: SEQ ID NOs: 07 & 08) were produced by conventional recombinant methods or ordered by external providers.

For some applications the antigens were further modified to result in active fragments or by attachment of a HIS-tag.

1.1 Design of expression vectors

Production in Expi293F cells was performed using pCSE2.5-His-XP, pCSE2.6-hFc-XP or pCSE2.6-mFc-XP (Jäger et al., 2013) where the respective single chain variable fragment of the antibodies or antigens were inserted by NcoI/NotI (NEB Biolabs) digestion. Antigen production in High Five insect cells was performed using NcoI/NotI compatible variants of the OpiE2 plasmid (Bleckmann et al., 2015) containing an N-terminal signal peptide of the mouse Ig heavy chain, the respective antigen and C-terminal either 6xHis-tag, hFc or mFc.

1.2 Production of antigens in insect cells

Different domains or subunits of the Spike protein (GenBank: MN908947) and the extracellular domain of ACE2 were Baculovirus-free produced in High Five cells (Thermo Fisher Scientific) by transient transfection as previously described in Bleckmann et al. (Bleckmann et al., 2019). Briefly, High Five cells were cultivated at 27°C, 110 rpm in ExCell405 media (Sigma) and kept at a cell density between 0.3 - 5.5 x106 cells/mL. For transfection cells were centrifuged and resuspended in fresh media to a density of 4x106 cells/mL and transfected with 4 $\mu$g plasmid/mL and 16 $\mu$g/mL of PEI 40 kDa (Polysciences). 4 h up to 24 h after transfection cells were fed with 75% of the transfection volume. At 48 h after transfection cell culture medium was doubled. Cell supernatant was harvested five days after transfection in a two-step-centrifugation (4 min at 180xg and 20 min at above 3500xg) and 0.2 $\mu$m filtrated for purification.

1.3 Production of antigens and scFv-Fc in mammalian cells

Antibodies, different domains or subunits of the Spike protein and the extracellular domain of ACE2 were produced in Expi293F cells (Thermo Fisher Scientific). Expi293F cells were cultured at 37°C, 110 rpm and 5% CO2 in Gibco FreeStyle F17 expression media (Thermo Fisher Scientific) supplemented with 8 mM Glutamine and 0.1% Pluronic F68 (PAN Biotech). At the day of transfection cell density was between 1.5 - 2 x10$^6$ cells/mL and viability at least above 90%. For formation of DNA:PEI complexes 1 $\mu$g DNA/mL transfection volume and 5 $\mu$g of 40 kDa PEI (Polysciences) were first diluted separately in 5% transfection volume in supplemented F17 media. DNA and PEI was then mixed and incubated -25 min at RT before addition to the cells. 48 h later the culture volume was doubled by feeding HyClone SFM4Transfx-293 media (GE Healthcare) supplemented with 8 mM Glutamine. Additionally, HyClone Boost 6 supplement (GE Healthcare) was added with 10% of the end volume. One week after transfection supernatant was harvested by 15 min centrifugation at 1500xg.

1.4 Protein purification

Protein purification was done depending on the production scale in 24 well filter plate with 0.5 mL resin (10 mL scale) or 1 mL column on Äkta Go (GE Healthcare), Äkta Pure (GE Healthcare) or Profina System (BIO-RAD). MabSure Select (GE Healthcare) was used as resign for Protein A purification. For His-tag purification of Expi293F supernatant HisTrap FF Crude column (GE Healthcare) and for His-tag purification of insect cell supernatant HisTrap excel column (GE Healthcare) was used. All purifications were performed according to the manufactures manual. Indicated antigens were further purified by size exclusion chromatography by a 16/600 Superdex 200 kDa pg (GE Healthcare). All antigens, antibodies and scFv-Fc were run on Superdex 200 Increase 10/300GL (GE Healthcare) on Äkta or HPLC (Techlab) on an AdvanceBio SEC 300Å 2.7 $\mu$m, 7.8x300 mm (Agilent) for quality control.

1.5 Validation of spike protein binding specifically to ACE2

ACE2 binding specifically to the produced antigens was confirmed in ELISA and on cells in cytometer.

For ELISA 200 ng ACE2-mFc per well was immobilized on a Costar High binding 96 well plate (Corning) at 4°C over night. Next, the wells were blocked with 350 µL 2% MBPST (2% (w/v) milk powder in PBS; 0.05% Tween20) for 1 h at RT and then washed 3 times with MilliQ-$H_2O$ and 0.05% Tween20 (Tecan Hydro Speed). Afterwards, the respective antigen was added at the indicated concentrations and incubated 1 h at RT prior to another 3 times washing step. Finally, the antigen was detected using mouse-anti-polyHis conjugated with horseradish peroxidase (HRP) (A7058, Sigma) for His-tagged antigens, goat-anti-mIgG(Fc) conjugated with HRP (A0168, Sigma) for mFc tagged antigen versions or goat-anti-hIgG(Fc) conjugated with HRP (A0170, Sigma) if hFc-tagged antigens had to be detected. Bound antigens were visualized with tetramethylbenzidine (TMB) substrate (20 parts TMB solution A (30 mM Potassium citrate; 1 % (w/v) Citric acid (pH 4.1)) and 1 part TMB solution B (10 mM TMB; 10% (v/v) Acetone; 90% (v/v) Ethanol; 80 mM $H_2O_2$ (30%)) were mixed). After addition of 1 N $H_2SO_4$ to stop the reaction, absorbance at 450 nm with a 620 nm reference was measured in an ELISA plate reader (Epoch, BioTek).

To verify the ACE2-antigen interaction on living cells, Expi293F cells were transfected according to the protocol above using pCSE2.5-ACE2fl-His and 5% eGFP plasmid. Two days after transfection purified S1-S2-His, S1-His or RBD-His was labeled using Monolith NTTM His-Tag Labeling Kit RED-tris-NTA (Nanotemper) according to the manufacturer's protocol. Fc-tagged versions were labeled indirectly by using goat-anti-mFc-APC (Dianova) or mouse anti-hFcgamma-APC (Biolegend) antibody. 100, 50 and 25 nM of antigen were incubated with 5x105 ACE2 expressing or non-transfected Expi293F cells (negative control) 50 min on ice. After two washing steps fluorescence was measured in MACSQuant Analyzer (Miltenyi Biotec.).

1.6 Production of SARS CoV2 spike domains or subunits and human ACE2 in insect cells and mammalian cells

SARS-CoV-2 RBD-SD1 (aa319-591) according to Wrapp et al. 2020 (Wrapp et al., 2020b), S1 subunit (aa14-694), S1-S2 (aa14-1210, with proline substitutions at position 986 and 987 and "GSAS" substitution at the furin site) and extracellular ACE2 were produced in insect cells using a plasmid based baculovirus free system (Bleckmann et al., 2019) as well as in Expi293F cells. All antigens with exception of S1-S2 were produced with human IgG1 Fc part, murine IgG2a Fc part or with 6xHis tag in both expression systems. S1-S2 was only produced with 6xHis tag. The extracellular domain of ACE2 was produced with human IgG1 Fc part in Expi293F cells and 6xHis tagged in insect cells. The yields of all produced proteins are given in table 3. The proteins were analyzed by size exclusion chromatography (SEC).

S1 as well as S1-S2 were produced in higher amounts in insect cells compared to Expi293F cells. RBD was produced well in both production systems. The binding of the produced spike domains/proteins to ACE2 was validated by ELISA and flow cytometry analysis on ACE2 positive cells.

Maximum production yields of SARS-CoV-2 spike protein/domains and human ACE2 in insect cells (High Five) and mammalian cells (Expi293F). Proteins with His-tag produced in High Five cells and S1-hFc* were additionally purified by SEC. * with Furin site.

**Table 3: Binding Results.**

|  | High Five cells | | | Expi293F cells | | |
|---|---|---|---|---|---|---|
|  | Yield | Binding specifically to ACE2 in ELISA | Binding to ACE2 on cells | Yield | Binding specifically to ACE2 in ELISA | Binding to ACE2 on cells |
| **RBD-hFc** | 90 mg/L | yes | yes | 203 mg/L | yes | yes |
| **RBD-mFc** | 48 mg/L | yes | yes | 116 mg/L | yes | yes |
| **RBD-His** | 92 mg/L | yes | yes | 35 mg/L | yes | yes |
| **S1-hFc*** | 7 mg/L | yes | yes | <1 mg/L | no | no |
| **S1-hFc** | 50 mg/L | yes | yes | <1 mg/L | weak | yes |
| **S1-mFc** | 36 mg/L | yes | yes | <1 mg/L | yes | yes |

(continued)

| | High Five cells | | | Expi293F cells | | |
|---|---|---|---|---|---|---|
| | Yield | Binding specifically to ACE2 in ELISA | Binding to ACE2 on cells | Yield | Binding specifically to ACE2 in ELISA | Binding to ACE2 on cells |
| S1-His | 15 mg/L | yes | yes | <1 mg/L | weak | no |
| S1-S2-His | 8 mg/L | yes | yes | <1 mg/L | no | no |
| ACE2-hFc | tbd | - | - | 82.8 mg/L | - | - |
| ACE2-mFc | tbd | - | - | 18.6 mg/mL | - | - |
| ACE-His | 1 mg/L | - | - | - | - | - |

**Example 2: Production of therapeutic antibodies**

For identification of antibodies a phage-display technique was applied. As libraries a naïve fab library as well as an immune library was used. S1-HIS was used as immobilized target. As counter selection streptavidin, IVIGs and protein N standard were used.

In total 192 clones from lambda and kappa selection were isolated. 48 sequence clusters with blocking antibodies were identified.

The number of clones was reduced for IgG conversion based on a rational approach: From each cluster at least one antibody was selected. From large clusters two clones were selected.

The following steps were performed to deselect regular antibodies, which are not suitable as therapeutic antibodies:

Step 1: Deselection of antibodies with not acceptable post-translational modifications (PTMs) (i.e. unpaired cysteines & glycosylations)

Step 2: Remaining clones were selected based on number of developability flags

Step 3: Clones with identical number of flags were selected based on ACE2:RBD inhibition

Step 4: Clones with similar ACE2:RBD inhibition were selected based on binding characteristics (i.e. RBD & S1-S2 binding was preferred over RBD binding only).

2.1 Antibody selection using phage display

The antibody selection was performed as follows: For the panning procedure, the antigen was immobilized on a Costar High binding 96 well plate (Corning). 5 μg of S1-hFc (produced in High Five cells) was diluted in carbonate puffer (50 mM NaHCO3, pH 9.6) and coated in the wells at 4°C overnight. Next, the wells were blocked with 350 μL % MBPST (2% (w/v) milk powder in PBS; 0.05% Tween20) for 1h at RT and then washed 3 times with PBST (PBS; 0.05% Tween20).

Before adding the libraries to the coated wells, the libraries (5x1010 phage particles) were preincubated with 2% MPBST on blocked wells for 1h at RT. The libraries were transferred to the coated wells, incubated for 2h at RT and washed 10 times. Bound phage was eluted with 150 μL trypsin (10 μg/mL) at 37°C and was used for the next panning round.

The eluted phage solution was transferred to a 96 deep well plate (Greiner Bio-One, Frickenhausen, Germany) and incubated with 145 μL E. coli TG1 (OD600 = 0.5) firstly for 30 min at 37°C, then 30 min at 37°C and 650 rpm to infect the phage particles. 1 mL 2xYT-GA (1.6% (w/v) Tryptone; 1 % (w/v) Yeast extract; 0.5% (w/v) NaCl (pH 7.0), 100 mM D-Glucose, 100 μg/mL ampicillin) was added and incubated for 1 h at 37°C and 650 rpm, followed by addition of 1x1010 cfu M13KO7 helper phage.

Subsequently, the infected bacteria were incubated for 30 min at 37°C followed by 30 min at 37°C and 650 rpm before centrifugation for 10 min at 3220xg.

The supernatant was discarded and the pellet resuspended in fresh 2xYT-AK (1.6% (w/v) Tryptone; 1 % (w/v) Yeast extract; 0.5% (w/v) NaCl (pH 7.0), 100 $\mu$g/mL ampicillin, 50 $\mu$g/mL kanamycin). The phage antibodies were amplified overnight at 30°C and 650 rpm and used for the next panning round. In total four panning rounds were performed. In each round, the stringency of the washing procedures was increased (20x in panning round 2, 30x in panning round 3, 40x in panning round 4) and the amount of antigen was reduced (2.5 $\mu$g in panning round 2, 1.5 $\mu$g in panning round 3 and 1 $\mu$g in panning round 4). After the fourth, respectively third panning round, the titer plate was used to select monoclonal antibody clones for the screening ELISA or for batch cloning to pCSE2.6-hIgG1-Fc-XP.

Antibody selection strategies using the human naive antibody gene libraris HAL9/10. n.a. = not applicable.

| Antibody selection campaign | library | target | panning rounds | binders/ screened clones | unique antibodies | cloned as scFv-Fc | inhibiting antibodies |
|---|---|---|---|---|---|---|---|
| STE70 | HAL9/10 | S1-hFc (Hi5) | 4 | 7/ 94 | 7 | 7 | 1 |
| STE72 | HAL10 (kappa) | S1-hFc (Hi5) | 4 | 397/ 752 | 90 | 44 | 8 |
| STE73 | HAL9 (lambda) | S1-hFc (Hi5) | 4 | 519/ 846 | 209 | 59 | 8 |
| STE77 | HAL10 (kappa) | S1-hFc (Expi) | 4 | 7/ 564 | 2 | n.a. | n.a. |
| STE78 | HAL9 (lambda) | S1-hFc (Expi) | 4 | 10/282 | 1 | n.a. | n.a. |

## 2.2 Screening of monoclonal recombinant binders using E. coli scFv supernatant

Soluble antibody fragments (scFv) were produced in 96-well MTPs with polypropylene (U96 PP, Greiner Bio-One). 150 $\mu$L 2xYT-GA was inoculated with the bacteria bearing scFv expressing phagemids. MTPs were incubated overnight at 37°C and 800 rpm in a MTP shaker (Ther-moshaker PST-60HL-4, Lab4You, Berlin, Germany). A volume of 140 $\mu$L 2xYT-GA in a PP-MTP well was inoculated with 10 $\mu$L of the overnight culture and grown at 37°C and 800 rpm until bacteria reached an OD600 of 0.5. Bacteria were harvested by centrifugation for 10 min at 3220xg and the supernatant was discarded. To induce expression of the antibody genes, the pellets were resuspended in 150 $\mu$L 2xYT supplemented with 100 $\mu$g/mL ampicillin and 50 $\mu$M isopropyl-beta D thiogalacto pyranoside (IPTG) and incubated at 30°C and 800 rpm overnight.

Bacteria were pelleted by centrifugation for 10 min at 3220xg and 4°C. The scFv-containing supernatant was transferred to a new PP-MTP and stored at 4°C before ELISA analysis.

For the ELISA, 100 ng of antigen was coated on 96 well microtiter plates (MaxiSorp, Thermo Fisher Scientific) in PBS (pH 7.4) overnight at 4°C. After coating, the wells were washed three times with PBST and blocked with 2% MPBST for 1 h at RT, followed by three washing steps with PBST. Supernatants containing monoclonal scFv were mixed with 2% MPBST (1:2) and incubated in the antigen coated plates for 1.5 h at RT followed by three PBST washing cycles. Bound scFv were detected using murine mAb 9E10 which recognizes the C-terminal c-myc tag (1:50 diluted in 2% MPBST) and a goat anti-mouse serum conjugated with horseradish peroxidase (HRP) (A0168, Sigma) (1:50,000 dilution in 2% MPBST).

Bound antibodies were visualized with tetramethylbenzidine (TMB) substrate (20 parts TMB solution A (30 mM Potassium citrate; 1% (w/v) Citric acid (pH 4.1)) and 1 part TMB solution B (10 mM TMB; 10% (v/v) Acetone; 90% (v/v) Ethanol; 80 mM H2O2 (30%)) were mixed). After stopping the reaction by addition of 1 N H2SO4, absorbance at 450 nm with a 620 nm reference was measured in an ELISA plate reader (Epoch, BioTek). Monoclonal binders were sequenced and analyzed using VBASE2 (www.vbase2.org) (Mollova et al., 2010).

## 2.3 Inhibition of S1-S2 binding specifically to ACE2 expressing cells using MacsQuant

The inhibition tests in cytometer on EXPI293F cells were performed based on the protocol for validation of spike protein binding specifically to ACE2 (see above) but only binding specifically to S1-S2-His antigen (High Five cell produced) was analysed. The assay was done in two setups. In the first setup 50 nM antigen was incubated with min. 1 μM of different scFv-Fc and the ACE2 expressing cells. The resulting median antigen fluorescence of GFP positive living single cells was measured. For comparison of the different scFv-Fc first the median fluorescence background of cells without antigen was subtracted, second it was normalized to the antigen signal where no antibody was applied. All scFv-Fc showing an inhibition in this setup were further analysed by titration (max. 1500 nM- 4.7 nM) on S1-S2-His (High Five cell produced). The $IC_{50}$ was calculated using the equation f(x)=Amin+(Amax-Amin)/(1+(x0/x)^h)^s and parameters from Origin. In addition, pairwise combinations (max. 750 nM of each scFv-Fc) of the different inhibiting scFv-Fc were tested.

2.4 Characterization of the scFv-Fc in titration ELISA

The ELISA were performed as described above in "Screening of monoclonal recombinant binders using E.coli scFv supernatant". For titration ELISA the purified scFv-hFc were titrated from 10 μg/mL- 0.001 μg/mL on S1-S2-His (High Five cell produced), RBD-mFc (High Five cell produced), S1-mFc (High Five cell produced). In addition, all 10μg/μL scFv-hFc were also tested for cross reactivity on Expi293F cell lysate, BSA and lysozyme. The scFv-hFc were detected using goat-anti-hIgG(Fc)-HRP (A0170, Sigma). Titration assays were performed using 384 well microtiter plates using Precision XS microplate sample processor (BioTek), EL406 washer dispenser (BioTek) and BioStack Microplate stacker (BioTek). EC50 were calculated with by GraphPad Prism Version 6.1) fitting to a four-parameter logistic curve).

2.5 Selection of antibodies by phage display

Antibodies were selected against S1 in four panning rounds in microtiter plates. The complete S1 subunit was used for antibody generation to ensure a native conformation of the RBD. The antibody screening by antigen ELISA was performed with soluble monoclonal scFv produced in E. coli in 96 well MTPs. Subsequently, binders were DNA sequenced and unique antibodies were recloned as scFv-Fc. Three pannings were performed. In a first approach the lambda (HAL9) and kappa (HAL10 library) were combined and the antigen S1-hFc (with Furin site, produced in High Five cells) was immobilized in PBS.

In the second approach, the pannings were performed separately for HAL10 and HAL9 using S1-hFc (with Furin site, produced in High Five cells) immobilized in carbonate buffer. Here, unique antibodies were selected from HAL10 and from HAL9.

In a third approach, S1-hFc produced in Expi293F cells was used. Here, the panning resulted in only three unique antibodies and these antibodies were not further analyzed in inhibition assays.

The Antibody subfamily distribution was analyzed and compared to the subfamily distribution in the HAL9/10 library and in vivo. Antibodies were selected covering the main gene VH gene families VH1 and VH3 whereas only few antibodies were selected using VH4. From the selected antibodies the V-gene VH3-23 was selected 96x. Therefore, this V-gene was used in 30% of the antibodies. In case of the lambda, the V-gene distribution is similar the distribution in the library. Here, it's only the V-gene VL6-57. For kappa, VK2 and VK4 are underrepresented compared to the library.

2.6 Transient production of anti-SARS-CoV-2 scFv-Fc in mammalian cells

Because of throughput issues only a selection of the unique antibodies was chosen for production. In addition, antibodies with glycosylation sites in the CDRs were excluded from the production as scFv-Fc because of developability issues. From all antibodies some antibodies were further produced as scFv-Fc in 5 mL scale. The production yields were in the range of 20-440 mg/L.

2.7 Antibodies inhibit the binding of S1 to ACE2 positive cells

The inhibition was measured by flow cytometry using ACE2 positive cells and labelled S1-S2 trimer. The spike protein was used for this inhibition assay to mimic the viral binding specifically to ACE2 positive cells. In a first screening step, all cloned antibodies were measured with 1500 nM antibodies (molar ration antibody: S1-S2 30:1) and antibodies with minimum ~75% inhibition were selected for further analysis.

In a next step, the inhibiting antibodies were further tested in different concentrations from 1500 nM to 4.7 nM (molar ratio 30:1 to ~1:10) in the cell based flow cytometry assay. The results of these inhibitions assays are given in figure 2B and table 4. The antibodies showed 50% inhibition with molar ratios of antibody binding site (antibody arm) to spike monomer of 0.8:1 to 13:1. The best antibodies showed 50% inhibition with a molar ration of 0.8 antibody arms to 1 spike (monomer). To validate these results, we performed the inhibition ELISA also on RBD in the cell based flow cytometry assay because RBD-mFc can be used in a lower amount of 10 nM in this assay (Figure 2C). With the exception of two antibodies all antibodies show a good inhibition of RBD with best molar ratios of 0.3-0.6.

The inhibition of the antibodies was validated on human Calu-3 cells which are ACE2 positive using RBD and S1-S2 showing a stronger inhibition on Calu-3 compared to the transiently over-expressing ACE2 positive Exp293Fi cells. The Expi293F system allowed the better discrimination between the degree of inhibition when using the complete S1-S2 spike protein, because the S1-S2 is directly labelled with a fluorophore and the signals are not amplified in comparison to RBD with a murine Fc and a secondary fluorophore labelled antibody. This assay also showed, that all inhibiting antibodies selected against S1 are binding RBD.

2.8 Determination of the EC50 of the inhibiting antibodies

The EC50 was measured by titration ELISA for the inhibiting antibodies on RBD, S1 and S1-S2 spike (figure 3). It was shown, that all inhibiting antibodies bound RBD. The best antibodies showed a half maximal binding at 0.7 nM, respectively 0.6 nM, on RBD. The EC50 values were slightly different between binding specifically to RBD or S1 subunit. The EC50 on S1-S2 spike was reduced for some of the antibodies compared to RBD or S1.

2.9 Synergistic effects of antibody combinations

The best antibodies were tested in the flow cytometry inhibition assay in pairwise combinations using 1500 nM antibody and 50 nM spike. Here, the combinations showed an improved inhibition compared to the single antibodies. The most antibody combinations were chosen using a lineage tree analysis (Genious Tree Builder) to reduce the amount of combinations to be tested.

The following therapeutic antibodies as depicted in SEQ ID NOs 9 to 1288 were finally identified. In another round of experiments the further antibodies as depicted in SEQ ID NOs 1289 to 1728 were finally identified.

For reference please be referred to table 4, which lists sequences segment (e.g. CDR, VL, etc.), sequence type (DNA or Protein "PRT"), antibody-Name and SEQ ID NO of the first three antibodies.

**Table 4: Reference of antibodies and SEQ ID NOs**

| Sequence Segment | Type | Antibody-Name | SEQ ID NO. | Antibody-Name | SEQ ID NO. | Antibody-Name | SEQ ID NO. |
|---|---|---|---|---|---|---|---|
| CDR1 of heavy chain (H-CDR1) | DNA | YU535-C09 | 9 | YU537-B05 | 29 | YU534-H01 | 49 |
| CDR1 of heavy chain (H-CDR1) | PRT | YU535-C09 | 10 | YU537-B05 | 30 | YU534-H01 | 50 |
| CDR2 of heavy chain (H-CDR2) | DNA | YU535-C09 | 11 | YU537-B05 | 31 | YU534-H01 | 51 |
| CDR2 of heavy chain (H-CDR2) | PRT | YU535-C09 | 12 | YU537-B05 | 32 | YU534-H01 | 52 |
| CDR3 of heavy chain (H-CDR3) | DNA | YU535-C09 | 13 | YU537-B05 | 33 | YU534-H01 | 53 |
| CDR3 of heavy chain (H-CDR3) | PRT | YU535-C09 | 14 | YU537-B05 | 34 | YU534-H01 | 54 |
| CDR1 of light chain (L-CDR1) | DNA | YU535-C09 | 15 | YU537-B05 | 35 | YU534-H01 | 55 |
| CDR1 of light chain (L-CDR1) | PRT | YU535-C09 | 16 | YU537-B05 | 36 | YU534-H01 | 56 |
| CDR2 of light chain (L-CDR2) | DNA | YU535-C09 | 17 | YU537-B05 | 37 | YU534-H01 | 57 |
| CDR2 of light chain (L-CDR2) | PRT | YU535-C09 | 18 | YU537-B05 | 38 | YU534-H01 | 58 |
| CDR3 of light chain (L-CDR3) | DNA | YU535-C09 | 19 | YU537-B05 | 39 | YU534-H01 | 59 |
| CDR3 of light chain (L-CDR3) | PRT | YU535-C09 | 20 | YU537-B05 | 40 | YU534-H01 | 60 |

(continued)

| Sequence Segment | Type | Antibody-Name | SEQ ID NO. | Antibody-Name | SEQ ID NO. | Antibody-Name | SEQ ID NO. |
|---|---|---|---|---|---|---|---|
| variable light chain (VL) | DNA | YU535-C09 | 21 | YU537-B05 | 41 | YU534-H01 | 61 |
| variable light chain (VL) | PRT | YU535-C09 | 22 | YU537-B05 | 42 | YU534-H01 | 62 |
| variable heavy chain (VH) | DNA | YU535-C09 | 23 | YU537-B05 | 43 | YU534-H01 | 63 |
| variable heavy chain (VH) | PRT | YU535-C09 | 24 | YU537-B05 | 44 | YU534-H01 | 64 |
| complete light chain (L) | DNA | YU535-C09 | 25 | YU537-B05 | 45 | YU534-H01 | 65 |
| complete light chain (L) | PRT | YU535-C09 | 26 | YU537-B05 | 46 | YU534-H01 | 66 |
| complete heavy chain (H) | DNA | YU535-C09 | 27 | YU537-B05 | 47 | YU534-H01 | 67 |
| complete heavy chain (H) | PRT | YU535-C09 | 28 | YU537-B05 | 48 | YU534-H01 | 68 |

Corresponding to the same consecutive pattern outlined in table 4, the further antibodies correspond to the following SEQ ID NOs as outlined in tables 5 to 7:

**Table 5: Further references of antibodies and corresponding SEQ ID NOs**

| Antibody Name | SEQ ID NOs | Antibody Name | SEQ ID NOs | Antibody Name | SEQ ID NOs |
|---|---|---|---|---|---|
| YU534-F11 | 69 - 88 | YU534-D06 | 269 - 288 | YU534-F06 | 469 - 488 |
| YU536-C01 | 89 - 108 | YU536-E07 | 289 - 308 | YU534-C05 | 489 - 508 |
| YU535-A06 | 109 - 128 | YU534-B03 | 309 - 328 | YU534-D09 | 509 - 528 |
| YU535-D12 | 129 - 148 | YU534-D10 | 329 - 348 | YU535-C06 | 529 - 548 |
| YU534-C09 | 149 - 168 | YU536-D04 | 349 - 368 | YU535-A09 | 549 - 568 |
| YU534-C12 | 169 - 188 | YU537-B08 | 369 - 388 | YU535-G09 | 569 - 588 |
| YU535-A02 | 189 - 208 | YU537-H11 | 389 - 408 | YU536-H01 | 589 - 608 |
| YU535-E07 | 209 - 228 | YU534-A11 | 409 - 428 | YU536-H05 | 609 - 628 |
| YU536-E12 | 229 - 248 | YU534-G06 | 429 - 448 | YU535-G02 | 629 - 648 |
| YU537-E03 | 249 - 268 | YU534-H02 | 449 - 468 | YU535-G11 | 649 - 668 |

**Table 6: Further references of antibodies and corresponding SEQ ID NOs**

| Antibody Name | SEQ ID NOs | Antibody Name | SEQ ID NOs | Antibody Name | SEQ ID NOs |
|---|---|---|---|---|---|
| YU534-B11 | 669 - 688 | YU534-G10 | 869 - 888 | YU505-E02 | 1069 - 1088 |
| YU534-C10 | 689 - 708 | YU505-A01 | 889 - 908 | YU505-E04 | 1089 - 1108 |
| YU535-D10 | 709 - 728 | YU505-A02 | 909 - 928 | YU505-F03 | 1109-1128 |
| YU535-B02 | 729 - 748 | YU505-A05 | 929 - 948 | YU505-F04 | 1129-1148 |
| YU534-D07 | 749 - 768 | YU505-B03 | 949 - 968 | YU505-F05 | 1149-1168 |

(continued)

| Antibody Name | SEQ ID NOs | Antibody Name | SEQ ID NOs | Antibody Name | SEQ ID NOs |
|---|---|---|---|---|---|
| YU537-H08 | 769 - 788 | YU505-B04 | 969 - 988 | YU519-A01 | 1169-1188 |
| YU534-E01 | 789 - 808 | YU505-B06 | 989 - 1008 | YU519-A09 | 1189 - 1208 |
| YU534-C02 | 809 - 828 | YU505-D01 | 1009 - 1028 | YU519-B05 | 1209 - 1228 |
| YU534-C06 | 829 - 848 | YU505-D02 | 1029 - 1048 | YU519-C01 | 1229 - 1248 |
| YU536-C12 | 849 - 868 | YU505-E01 | 1049 - 1068 | YU519-E03 | 1249 - 1268 |
|  |  |  |  | YU519-E12 | 1269 - 1288 |

**Table 7: Further references of antibodies and corresponding SEQ ID NOs**

| Antibody Name | SEQ ID NOs | Antibody Name | SEQ ID NOs | Antibody Name | SEQ ID NOs |
|---|---|---|---|---|---|
| PHE162-D2 | 1289-1296 | STE91-B1-B3 | 1473-1480 | STE72-2G4 | 1657-1664 |
| STE90-A10 | 1297-1304 | STE94-A1 | 1481-1488 | STE73-2B2 | 1665-1672 |
| STE90-C11 | 1305-1312 | STE94-B2 | 1489-1496 | STE73-2C2 | 1673-1680 |
| STE90-D7 | 1313-1320 | STE94-D3 | 1497-1504 | STE73-2E9 | 1681-1688 |
| STE90-G2 | 1321-1328 | STE94-E3 | 1505-1512 | STE73-2G8 | 1689-1696 |
| STE90-B1-A10 | 1329-1336 | STE94-E9 | 1513-1520 | STE73-6B10 | 1697-1704 |
| STE90-B1-B4 | 1337-1344 | STE94-F6 | 1521-1528 | STE73-6C1 | 1705-1712 |
| STE90-B1-D1 | 1345-1352 | STE94-F7 | 1529-1536 | STE73-6C8 | 1713-1720 |
| STE90-B1-D6 | 1353-1360 | STE94-F12 | 1537-1544 | STE73-9G3 | 1721-1728 |
| STE90-B1-F8 | 1361-1368 | STE94-H2 | 1545-1552 |  |  |
| STE90-B2-A1 | 1369-1376 | STE94-B1-D1 | 1553-1560 |  |  |
| STE90-B2-B3 | 1377-1384 | STE94-B1-E12 | 1561-1568 |  |  |
| STE90-B2-C5 | 1385-1392 | STE94-B2-B1 | 1569-1576 |  |  |
| STE90-B2-D12 | 1393-1400 | STE94-B2-B6 | 1577-1584 |  |  |
| STE90-B2-E6 | 1401-1408 | STE94-B2-E11 | 1585-1592 |  |  |
| STE90-B3-B10 | 1409-1416 | STE70-1 E12 | 1593-1600 |  |  |
| STE90-B3-H3 | 1417-1424 | STE72-1 B6 | 1601-1608 |  |  |
| STE90-B3-H6 | 1425-1432 | STE72-1G5 | 1609-1616 |  |  |
| STE91-C4 | 1433-1440 | STE72-4C10 | 1617-1624 |  |  |
| STE91-D4 | 1441-1448 | STE72-4E12 | 1625-1632 |  |  |
| STE91-F6 | 1449-1456 | STE72-8A2 | 1633-1640 |  |  |
| STE91-F10 | 1457-1464 | STE72-8A6 | 1641-1648 |  |  |
| STE91-B1-A10 | 1465-1472 | STE72-8E1 | 1649-1656 |  |  |

In one embodiment from these antibodies YU505-A01 (SEQ ID NOs: 889 - 908), YU505-A02 (SEQ ID NOs: 909 - 928), YU505-E01 (SEQ ID NOs: 1049 - 1068) and YU505-F05 (SEQ ID NOs: 1149 - 1168) have been selected for further characterization.

In a further embodiment from these antibodies YU534-C09 (SEQ ID Nos: 149 - 168),YU534-C12 (SEQ ID NOs: 169 - 188), YU534-D09 (SEQ ID NOs: 509 - 528), YU535-A02 (SEQ ID NOs: 189 - 208), YU536-D04 (SEQ ID NOs: 349 - 368), YU537-H08 (SEQ ID NOs: 769 - 788) and YU537-H11 (SEQ ID NOs: 389 - 408) have been selected for further characterization.

The CDR-sequences of these four antibodies are also depicted in Figure 3, their VL and VH-sequences are depicted in Figures 4a to 4d. The scFv DNA-sequences of fragments of these antibodies are depicted in Figure 5a to 5d.

### Example 3: Binding and Competition Assays

Different binding characteristics of the four antibodies were analyzed.

| Name | Specificity | ACE2:RBD-com-petition at [5 μg/ml antibody] | ACE2:RBD-com-petition at [0.5 μg/ml antibody] | $K_D$ |
|---|---|---|---|---|
| YU505-A01 | RBD binding | less than 30% of ACE2 bound to RBD | less than 60% of ACE2 bound to RBD | $3.41 \times 10^{-9}$ |
| YU505-A02 | RBD binding | less than 30% of | less than 60% of | $1.07 \times 10^{-8}$ |
| | | ACE2 bound to RBD | ACE2 bound to RBD | |
| YU505-E01 | RBD binding | less than 25% of ACE2 bound to RBD | less than 50% of ACE2 bound to RBD | $4.84 \times 10^{-9}$ |
| YU505-F05 | RBD + S1 binding | less than 30% of ACE2 bound to RBD | less than 60% of ACE2 bound to RBD | $1.31 \times 10^{-8}$ |

### Example 4: Infectious SARS-CoV-2 neutralizing assay (Confluent VERO E6-Test)

Antibodies were diluted in culture medium and mixed with 50 μl (500 TCID50) SARS-CoV-2 for 1 hour. The mixture was then added to VERO E6 cells. As controls cells were grown either with medium only or with the SARS-CoV-2 mixture without adding any antibody. Cells were incubated for 1 hour, after which the cells were washed and further incubated in medium for 3d and 18h.
In another embodiment antibodies were diluted in 1:10-steps and mixed with 5 μl (50 TCID50) SARS-CoV-2-virus. The mix is then added to the cells. Cells are washed after 1h and incubated with methyl-cellulose-containing medium. After 3 days single plaques become visible within the cell layer, which can be counted.
Confluence of cells was measured at 4 dpi (cf. Figure 2).

**Table 8: Result of Infectious SARS-CoV-2 neutralizing assay (Confluent VERO E6-Test)**

| Antibody | Confluence Score in VERO E6 cell assay |
|---|---|
| YU505-A01 | > 99% |
| YU505-A02 | > 99% |
| YU505-E01 | > 98% |
| YU505-F05 | > 95% |

### Example 5: SEC-HLPC of specific Ab

Size-exclusion HPLC was performed with the four antibodies YU505-A01, YU505-A02, YU505-E01 and YU505-F05.
YU505-A01 and YU505-E01 showed no, YU505-A02 and YU505-F05 showed only a very small agglutination-peak (the peaks at time-points > 10 are contaminations of the column), confirming the usability of the antibodies for therapeutic application.
See also Figure 7.

### Example 6: Specificity ELISA

In a specificity ELISA the "stickiness" of some antibodies was compared with Avelumab-control.
Mean S/N stickiness (test antibodies vs. Avelumab) was below 1 for YU505-A01 (about 0.5), YU505-A02 (about 0.6) and YU505-E01 (about 0.8).
See table 9 and Figure 6.

## Table 9: Results of Specificity ELISA

| | YU505-F05 | YU505-E01 | YU505-A01 | YU505-A02 | Trastuzumab | Trastuzumab (silenced Fc) | Palivizumab | Palivizumab (silenced Fc) | IVIGs | Avelumab | 2% BSA-T |
|---|---|---|---|---|---|---|---|---|---|---|---|
| DNA | 0.3 | 0.3 | 0.2 | 0.3 | 0.4 | 0.6 | 0.3 | 0.5 | 0.6 | 1.0 | 0.1 |
| LPS | 1.3 | 1.1 | 0.9 | 0.8 | 1.5 | 1.2 | 1.3 | 1.2 | 3.9 | 1.0 | 0.8 |
| Lysozyme | 1.1 | 1.4 | 0.7 | 0.9 | 1.0 | 0.6 | 0.8 | 0.9 | 0.7 | 1.0 | 0.3 |
| Cell lysate | 1.7 | 0.4 | 0.2 | 0.2 | 0.3 | 0.2 | 0.3 | 0.3 | 0.3 | 1.0 | 0.1 |
| Mean stickyness | 1.1 | 0.8 | 0.5 | 0.6 | 0.8 | 0.7 | 0.7 | 0.7 | 1.4 | 1.0 | 0.3 |

| | YU505-F05 | YU505-E01 | YU505-A01 | YU505-A02 | Trastuzumab | Trastuzumab (silenced Fc) | Palivizumab | Palivizumab (silenced Fc) | IVIGs | Avelumab | 2% BSA-T |
|---|---|---|---|---|---|---|---|---|---|---|---|
| Stdev. stickyness | 0.6 | 0.5 | 0.4 | 0.4 | 0.5 | 0.4 | 0.5 | 0.4 | 1.7 | 0.0 | .03 |

Please note: Values >1.0 indicate increased "stickyness" compared to Avelumab

### Example 7: Melting Temperature Analysis

Antibodies were analyzed by nanoDSF which uses tryptophan or tyrosin fluorescence to monitor protein unfolding in terms of effect on the onset melting temperature ($T_m$ onset), the melting temperature ($T_m$) and aggregation temperature ($T_{agg}$) of the target protein.

10 Antibodies were tested, the target assay concentration was 0.15 mg/ml, the assay buffer was 1x PBS pH 7.4, assay samples were prepared by diluting the target protein from its stock to the given concentration in assay buffer, all samples were stored at -80°C prior to the experiment phase.

The experiments were performed on a Prometheus NT.48™ device equipped with additional back reflection options for detection of target protein aggregation. The technical parameters were as follows: The LED-sensitivity was set at 50% (the capillary scan indicated that 50% LED intensity resulted in sufficient intrinsic fluorescence of the targets), the temperature range was 20-95°C, the heating speed 1°C/min. The capillary type was a "high sensitivity"-type. The ratio 350/330 nm was used for protein unfolding ($T_m$) and onset of protein unfolding ($T_m$ onset). Back scattering for sample aggregation ($T_{agg}$).

All samples showed comparable fluorescence signals. No protein aggregation was observed at the starting temperature of 20°C, indicated by scattering signals (crosses) around 80 mAU.

Measurements were done in duplicates and the mean as well as the error deviation was calculated. Data were analyzed using the PR.ThermalControl™ v2.0.4 software from Nanotemper Technologies™.

The results of the analysis are summarized in Table 10.

## Table 10: Summary of results of $T_m$ onset, $T_{m1}$, $T_{m2}$, and $T_{agg}$ for the tested antibodies:

| Sample | $T_m$ onset | Tm1 | Tm2 | Tagg |
|---|---|---|---|---|
| YU505-A01 | 61.6 ± 0.1°C | 68.8 ± 0.1°C | 81.9 ± 0.1°C | 71.3 ± 0.1°C |
| YU505-A02 | 60.1 ± 0.2°C | 70.0 ± 0.2°C | 79.7 ± 0.1°C | 78.6 ± 0.2°C |
| YU505-E01 | 57.1 ± 0.3°C | 60.3 ± 0.0°C | 68.2 ± 0.1°C | 68.4 ± 0.1°C |
| YU505-F05 | 57.3 ± 0.3°C | 64.5 ± 0.0°C | 81.6 ± 0.1°C | 69.5 ± 0.3°C |
| YU534-C12 | 65.3 ± 0.8°C | 69.5 ± 0.0°C | 81.4 ± 0.7°C | 71.2 ± 0.0°C |
| YU534-D09 | 62.4 ± 0.1°C | 68.9 ± 0.0°C | 81.4 ± 0.1°C | 69.9 ± 0.0°C |
| YU535-A02 | 62.2 ± 0.1°C | 70.8 ± 0.2°C | 81.9 ± 0.2°C | 75.9 ± 0.0°C |
| YU536-D04 | 62.4 ± 0.0°C | 69.3 ± 0.1°C | 80.9 ± 0.0°C | 81.2 ± 0.3°C |
| YU537-H08 | 56.9 ± 0.2°C | 62.1 ± 0.2°C | 68.0 ± 0.2°C | 70.1 ± 0.1°C |
| YU537-H11 | 63.7 ± 0.0°C | 70.8 ± 0.2°C | --- | 71.7 ± 0.2°C |

### Example 8: Further characteristics of the antibodies

Antibodies were further characterized regarding ACE2:RBD-inhibition, isoelectric point (pI) of human IgG, hydrophobicity and charge. The results are outlined in table 11.

### Table 11: Further characteristics of the antibodies

| Antibody | ACE2:RB D inhibi-tion with 5 μg/mL IgG [%] | pI human IgG | Germina-lity Index VH [%] | Germina-lity Index VL [%] | Total hydro-phobicity score | CDR hydro-phobicity score | CDR charge total |
|---|---|---|---|---|---|---|---|
| YU535-C09 | | 8.11 | 91.9 | 96.9 | 4.09 | 1.28 | -1.03 |
| YU537-B05 | | 8.3 | 99.1 | 100 | 4 | 1.94 | -0.49 |
| YU534-H01 | | 8.21 | 99.1 | 99 | 3.26 | 1.23 | -0.6 |
| YU534-F11 | | 8.01 | 96.3 | 97 | 3.7 | 1.6 | 0.15 |
| YU536-C01 | | 8.11 | 98.2 | 99 | 3.89 | 1.23 | -1.36 |
| YU535-A06 | | 8.11 | 97.2 | 100 | 2.73 | 0.45 | -0.63 |
| YU535-D12 | | 8.21 | 97.2 | 99 | 3.22 | 0.58 | -0.49 |
| YU534-C09 | | 8.11 | 84.4 | 100 | 5.08 | 2.09 | -0.83 |

(continued)

| Antibody | ACE2:RB D inhibi-tion with 5 µg/mL IgG [%] | pI human IgG | Germina-lity Index VH [%] | Germina-lity Index VL [%] | Total hydro-phobicity score | CDR hydro-phobicity score | CDR charge total |
|---|---|---|---|---|---|---|---|
| YU534-C12 | | 8.21 | 99.1 | 96 | 4.36 | 1.84 | 0.3 |
| YU535-A02 | | 8.3 | 99.1 | 98 | 2.82 | 0.46 | -0.7 |
| YU535-E07 | | 8.3 | 97.2 | 99 | 2.96 | 0.79 | -0.6 |
| YU536-E12 | | 7.74 | 97.2 | 100 | 3.9 | 0.47 | -1.45 |
| YU537-E03 | | 7.88 | 96.3 | 99 | 3.9 | 0.75 | -0.89 |
| YU534-D06 | | 7.88 | 97.2 | 100 | 3.56 | 1.47 | -1.71 |
| YU536-E07 | | 8.11 | 98.1 | 100 | 4.31 | 1.17 | -0.1 |
| YU534-B03 | | 7.74 | 97.2 | 99 | 4.3 | 0.87 | -1.24 |
| YU534-D10 | | 8 | 98.1 | 99 | 4.52 | 1.3 | -0.14 |
| YU536-D04 | | 8.39 | 95.4 | 96.9 | 4.37 | 1.45 | -0.25 |
| YU537-B08 | | 8.11 | 97.2 | 97 | 4.43 | 2.05 | -0.47 |
| YU537-H11 | | 8.11 | 96.3 | 99 | 4.64 | 1.29 | -0.19 |
| YU534-A11 | | 8.21 | 99.1 | 100 | 4.38 | 1.71 | -0.86 |
| YU534-G06 | | 8.11 | 94.5 | 95 | 3.83 | 1.52 | -0.52 |
| YU534-H02 | | 7.58 | 93.6 | 96 | 3.38 | 1.55 | 0.53 |
| YU534-F06 | | 8.01 | 93.6 | 98 | 4.3 | 2.2 | 0.07 |
| YU534-C05 | | 8.31 | 93.6 | 93 | 4.54 | 1.53 | -0.09 |
| YU534-D09 | | 8.11 | 95.4 | 99 | 3.65 | 1.58 | 0.01 |
| YU535-C06 | | 8.21 | 99.1 | 99 | 2.57 | 0.49 | -1.18 |
| YU535-A09 | | 7.88 | 100 | 95.9 | 3.27 | 0.52 | -1.7 |
| YU535-G09 | | 8 | 100 | 100 | 3.17 | 0.48 | -0.72 |

(continued)

| Antibody | ACE2:RB D inhibi-tion with 5 µg/mL IgG [%] | pI human IgG | Germina-lity Index VH [%] | Germina-lity Index VL [%] | Total hydro-phobicity score | CDR hydro-phobicity score | CDR charge total |
|---|---|---|---|---|---|---|---|
| YU536-H01 | | 8 | 100 | 99 | 3.31 | 0.76 | -0.54 |
| YU536-H05 | | 7.87 | 99.1 | 98 | 2.93 | 0.75 | -0.7 |
| YU535-G02 | | 8 | 99.1 | 91.8 | 2.98 | 0.45 | -1.05 |
| YU535-G11 | | 8 | 100 | 96.9 | 3.43 | 0.75 | -0.87 |
| YU534-B11 | | 7.88 | 98.2 | 100 | 4.25 | 0.77 | -1.27 |
| YU534-C10 | | 8 | 97.2 | 100 | 4.45 | 0.95 | -1.81 |
| YU535-D10 | | 8.11 | 98.2 | 98 | 3 | 0.77 | -1.2 |
| YU535-B02 | | 7.87 | 99.1 | 91.8 | 2.41 | 0.59 | -1.56 |
| YU534-D07 | | 8 | 100 | 99 | 3.09 | 0.6 | -2.17 |
| YU537-H08 | | 8.01 | 98.2 | 97.9 | - | - | - |
| YU534-E01 | | 7.88 | 93.6 | 93.9 | - | - | - |
| YU534-C02 | | 7.88 | 96.3 | 91 | 4.69 | 1.55 | -2.92 |
| YU534-C06 | | 7.74 | 96.3 | 93 | 4.38 | 1.02 | -1.52 |
| YU536-C12 | | 7.75 | 96.3 | 94 | 3.92 | 1.45 | -1.58 |
| YU534-G10 | | 8 | 95.4 | 98 | 3.91 | 1.46 | -1.22 |
| YU505-A01 | 102.2 | 8.11 | 100 | 98 | 3.41 | 0.5 | -0.58 |
| YU505-A02 | 43.8 | 7.75 | 99.1 | 100 | 6.02 | 3.14 | -0.63 |
| YU505-A05 | 52.7 | 8.11 | 100 | 92 | 3.84 | 1.76 | 0.09 |
| YU505-B03 | 78.9 | 8.56 | 98.2 | 93.9 | 3.13 | 0.53 | 0.82 |
| YU505-B04 | 76.6 | 8.01 | 100 | 99 | 4.22 | 0.88 | -0.32 |
| YU505-B06 | 58.5 | 8.12 | 98.2 | 100 | 4.29 | 0.62 | -1.28 |

(continued)

| Antibody | ACE2:RB D inhibi-tion with 5 μg/mL IgG [%] | pI human IgG | Germina-lity Index VH [%] | Germina-lity Index VL [%] | Total hydro-phobicity score | CDR hydro-phobicity score | CDR charge total |
|---|---|---|---|---|---|---|---|
| YU505-D01 | 89.0 | 8.39 | 100 | 97 | 3.12 | 0.35 | -1.49 |
| YU505-D02 | 49.4 | 8.12 | 100 | 99 | 4.62 | 1.16 | -0.55 |
| YU505-E01 | 24.0 | 7.88 | 93.6 | 97 | 5.18 | 1.86 | -0.85 |
| YU505-E02 | 58.8 | 8.21 | 100 | 97 | 6.46 | 3.11 | 0.24 |
| YU505-E04 | 28.0 | 7.74 | 100 | 96 | 4.57 | 1.72 | -1.22 |
| YU505-F03 | 42.2 | 8.3 | 96.3 | 96 | 3.14 | 0.48 | -1.13 |
| YU505-F04 | 79.1 | 7.74 | 100 | 92 | 4.46 | 1.37 | -1.15 |
| YU505-F05 | 26.7 | 8.31 | 93.6 | 95.9 | 4.5 | 0.44 | -0.48 |
| YU519-A01 | 74.1 | 7.88 | 98.2 | 92.9 | 5.22 | 2.65 | -1.97 |
| YU519-A09 | 35.6 | 7.88 | 97.2 | 94.8 | 4.63 | 1.17 | -2.46 |
| YU519-B05 | 128.4 | 8.21 | 100 | 99 | 5.08 | 2.71 | 0.53 |
| YU519-C01 | 140.7 | 8.48 | 98.2 | 98 | 3.9 | 1.11 | -0.57 |
| YU519-E03 | 148.9 | 8.11 | 100 | 98 | 4.23 | 1.82 | 0.63 |
| YU519-E12 | 104.1 | 8 | 100 | 94.9 | 4.84 | 2.21 | 0.08 |

**Example 9: IC$_{50}$-measurements in Vero E6 Cells**

Screening and titration of some therapeutic monoclonal antibodies for SARS-CoV-2 neutralization in cell culture VeroE6 cells (ATCC CRL-1586) were seeded at a density of 6*10$^4$/well onto cell culture 96-well plates (Nunc, Cat.#167008). Two days later, cells reached 100% confluence. For neutralization screening, antibodies (1 μg/ml final concentration) were mixed with the virus inoculum (250 pfu/well), using strain SARS-CoV-2/Münster/FI 110320/1/2020, in 100 μl full VeroE6 culture medium (DMEM, 10% FCS, 2 mM glutamine, penicillin, streptomycin) in technical quadruplicates or six fold replicates and incubated for 1 hour at 37°C.

Then, cells were overlaid with the antibody/virus mix and phase contrast images were taken automatically using a Sartorius IncuCyte S3 (10x objective, two hours image intervals, 4 images per well) housed in a HeraCell 150i incubator (37°C, 100% humidity, 5% CO2).

Image data was quantified with the IncuCyte S3 GUI tools measuring the decrease of confluence concomitant with the cytopathic effect of the virus in relation to uninfected controls and controls without antibody and analyzed with GraphPad Prism 8.

For antibody titration, serial dilutions of antibodies from 1 μg/ml to 0.3 ng/ml were incubated with 25 pfu/well for one hour at 37°C and then added to confluent VeroE6 cells on 96-well cell culture plates for one hour in the incubator

(100 μl/well).

Finally, the inoculum was removed and cells were overlaid with 100 μl MEM containing 1.5% methyl-cellulose.

Three days post infection, resulting plaques per well were counted from phase contrast images obtained with a Sartorius IncuCyte S3.

IC50 was determined by fitting a sigmoidal function (four parameter logistic growth) using GraphPad Prism 8™.

Further results are listed in Figure 17.

**Example 10: Features of the most preferred antibodies**

Features of the most preferred antibodies are depicted in tables 12 to 29.

**Table 12.**

| mAb | Origin | Germline VH | Germline VL | Virus Neutralization at 1 μg/mL | |
|---|---|---|---|---|---|
| | | | | IgG [mean %] | scFv-Fc [median %] |
| STE90-C11 | immune scFv library | IGHV3-66*04 | IGKV1-9*01 | n.d. | 100 |
| YU536-D04 | immune scFv library | IGHV3-66*02 | IGKV1-9*01 | 99 | n.d. |
| YU537-H11 | immune scFv library | IGHV3-53*01 | IGLV1-51*01 | 93 | n.d. |

**Table 13.**

| mAb | Virus neutralization (96-well titration) | Virus neutralization (24-well titration) | cell based ACE2:10 nM RBD inhibition (FACS) |
|---|---|---|---|
| | TCID50 (IgG) [nM] | TCID50 (IgG) [nM] | scFv-Fc IC50[nM] |
| STE90-C11 | 0.91 | 0.56 | n.d. |
| YU536-D04 | 0.20 | 0.40 | n.d. |
| YU537-H11 | 0.27 | 0.47 | n.d. |

**Table 14.**

| mAb | cell based ACE2:50 nM S1-S2 inhibition (FACS) | | ACE2:RBD Competition ELISA |
|---|---|---|---|
| | IgG IC50[nM] | scFv-Fc IC50[nM] | RBD binding activity at 33 nM IgG (=5 μg/ml) [%] |
| STE90-C11 | 13 | n.d. | n.d. |
| YU536-D04 | n.d. | n.d. | 6 |
| YU537-H11 | n.d. | n.d. | 10 |

**Table 15.**

| mAb | ELISA EC50 (IgG) | | | ELISA EC50 (scFv-Fc) | | |
|---|---|---|---|---|---|---|
| | RBD [nM] | S1-S2 [nM] | S1 [nM] | RBD [nM] | S1-S2 [nM] | S1 [nM] |
| STE90-C11 | 0.3 | 0.5 | 0.3 | n.d. | n.d. | n.d. |
| YU536-D04 | 1.1 | n.d. | n.d. | n.d. | n.d. | n.d. |
| YU537-H11 | 1.4 | n.d. | n.d. | n.d. | n.d. | n.d. |

**Table 16.**

| mAb | Epitope | Yield (Expi293F) IgG [mg/L] | SDS-PAGE | High MW fraction in SEC [%] | | | Melt. Temp [°C] | Agg. Temp [°C] |
|---|---|---|---|---|---|---|---|---|
| | | | | normal | 24 h at pH3 | 24 h at 45°C | | |
| STE90-C11 | n.d. | 59 | ok | 0.0 | n.d. | 0.0 | n.d. | n.d. |
| YU536-D04 | conformational | n.d. | ok | 0.0 | 1.1 | 0.0 | 69 | 81 |
| YU537-H11 | conformational | n.d. | ok | 0.0 | 0.0 | 0.0 | 71 | 72 |

**Table 17.**

| mAb | S/N stickiness to DNA | S/N stickiness to LPS | S/N stickiness to Lysozyme | S/N stickiness to cell lysate |
|---|---|---|---|---|
| STE90-C11 | 1.0 | 1.0 | 1.0 | 0.7 |
| YU536-D04 | 0.3 | 1.2 | 0.6 | 0.3 |
| YU537-H11 | 0.3 | 1.2 | 0.7 | 0.6 |

**Table 18.**

| mAb | CDR of VH | CDR of VL |
|---|---|---|
| STE90-C11 | GLTVSSNY_IYSGGST_ARDVADAFDI | QGISSY_AAS_QQLN-SYPPFT |
| YU536-D04 | SNYMS_VIYSGGSTYYADSVKG_DLVVM-GLDV | RASQGISSYLA_AAST-LQS_QQLNSYPIT |
| YU537-H11 | SNYMS_VIYSGGSTYYADSVKG_GES-GSPYGMDV | SGSSSNIGN-NYVS_DTNKRPS_ATWDS-SLSVGV |

Table 19.

| mAb | AA of VH |
|---|---|
| STE90 -C11 | QVQLVESGGGLVQPGGSLRLSCAASGLTVSSNYMSWWRQAPGK-GLEWVSVIYSGGSTYYADSVKGRFTISRDDSKNTLYLQMNSLRAEDTA-VYYCARDVADAFDIWGQGTMVTVSS |
| YU536 -D04 | QVQLVQSGGGLVQPGGSLRLSCAASGLTVSSNYMSWWRQAPGK-GLEWVSVIYSGGSTYYADSVKGRFIISRDNSKNTLYLQMNSLRAEDTA-VYYCARDLVVMGLDVWGKGTLVTVSS |
| YU537 -H11 | EVQLLESGGGLIQPGGSLRLSCAASGLTVSSNYMSWWRQAPGK-GLEWVSVIYSGGSTYYADSVKGRFTISRDNSKNALYLQMNSLRAEDTAVYYCAR-GESGSPYGMDVWGQGTLVTVSS |

**Table 20.**

| mAb | AA of VL |
|---|---|
| STE90-C11 | DIVMTQSPSFLSASVGDRVTITCRASQGISSYLAWYQQKPGKAPKLLIY*AAST*-LQSGVPSRFSGSGSGTEFTLTISSLQPEDFATYYC<u>QQLNSYPPFT</u>FGPGTKVDIK |
| YU536-D04 | DIQLTQSPSSLSASVGDRVTITCRASQGISSYLAWYQQKPGKAPKLLIYAAST-LQSGVPSRFSGSGSGTDFTLTISSLQPEDFATYYCQQLNSYPITFGQGTKLEIK |
| YU537-H11 | QSVLTQPPSVSAAPGQKVTISCSGSSSNIGNNYVSWYQQLPG-TAPKLLIYDTNKRPSGIPDRFSGSKSGTSATLGITGLQTGDEADYYCATWDS-SLSVGVFGGGTKLTVL |

**Table 21.**

| mAb | Pairing a-bundamce | pI human IgG | Germinality Index VH [%] | Germinality Index VL [%] | Unusual residues VH |
|---|---|---|---|---|---|
| STE90-C11 | 0.02 | 8 | 97.2 | 98 | 0 |
| YU536-D04 | 0.02 | 8.39 | 95.4 | 96.9 | 0 |
| YU537-H11 | 0.02 | 8.11 | 96.3 | 99 | 0 |

**Table 22.**

| mAb | Unusual residues VL | Total CDR Length | Total hydrophobicity score | CDR Hydrophobicity score |
|---|---|---|---|---|
| STE90-C11 | 0 | 57 | 4.2 | 0.92 |
| YU536-D04 | 0 | 57 | 4.37 | 1.45 |
| YU537-H11 | 0 | 63 | 4.64 | 1.29 |

**Table 23.**

| mAb | scFv charge | CDR charge total | Cysteines/Glycosylation Flags | Other PTM flags |
|---|---|---|---|---|
| STE90-C11 | -1.04 | -1.14 | 0 | 0 |
| YU536-D04 | 1.22 | -0.25 | 0 | 1 |
| YU537-H11 | 0.82 | -0.19 | 0 | 0 |

**Table 24.**

| mAb | Amidation site | Asparagine deamidation site | Aspartate isomerization site | Cysteine in CDR |
|---|---|---|---|---|
| STE90-C11 | 0 | 1 | 1 | 0 |
| YU536-D04 | 0 | 1 | 1 | 0 |
| YU537-H11 | 0 | 0 | 3 | 0 |

**Table 25.**

| mAb | Fragmentation site | Integrin binding site | Lysine glycation site | Methionine in CDR | N-glycosylation site |
|---|---|---|---|---|---|
| STE90-C11 | 0 | 0 | 0 | 1 | 0 |
| YU536-D04 | 0 | 1 | 0 | 2 | 0 |

(continued)

| mAb | Fragmentation site | Integrin binding site | Lysine glycation site | Methionine in CDR | N-glycosylation site |
|---|---|---|---|---|---|
| YU537-H11 | 0 | 0 | 0 | 2 | 0 |

**Table 26.**

| mAb | N-terminal glutamate | Tryptophan in CDR |
|---|---|---|
| STE90-C11 | 0 | 0 |
| YU536-D04 | 0 | 0 |
| YU537-H11 | 1 | 1 |

**Table 27.**

| mAb | ID-hIgG1 HS | Name | T cell Epitope Score | Critical Cys, N-Glyco |
|---|---|---|---|---|
| STE90-C11 | TPP-27099 | STE90-C11-hIgG1wt-HS-kappa | 354.2 | 0 |
| YU536-D04 | TPP-27110 | YU536-D04-hIgG1wt-HS-kappa | 649.5 | 0 |
| YU537-H11 | TPP-27096 | YU537-H11-hIgG1wt-HS-lambda | 501.8 | 0 |

**Table 28.**

| mAb | Non-Critical Asp Isomerisation, Deamidation, M-Oxidation (CDRs only M35 excluded) | Predicted Solubility [mg/ml] | Predicted thermostability [deg_C] | Predicted viscosity [cP] |
|---|---|---|---|---|
| STE90-C11 | 2 | 92.186 | 73.86 | 60.239 |
| YU536-D04 | 3 | 89.391 | 76.308 | 26.142 |
| YU537-H11 | 4 | 103.588 | 75.624 | 67.315 |

**Table 29.**

| mAb | | | |
|---|---|---|---|
| STE90-C11 | Kappa, G1 | (IGKV1-9-01-IGKJ3-01) | (IGHV3-66-01-D-IGHJ3-01) |
| YU536-D04 | Kappa, G1 | (IGKV1-39-01-IGKJ2-01) | (IGHV3-66-01-D-IGHJ2-01) |
| YU537-H11 | Lambda, G1 | (IGLV1-51-01-IGLJ2-01) | (IGHV3-53-01-D-IGHJ1-01) |

**Example 11: Binding studies with "escape"-mutations**

A deeper characterization of the best neutralizer was performed in respect to cross-reaction to other coronaviruses, binding specifically to known RBD mutations, biochemical and biophysical properties was conducted. The binding of STE90-C11, YU505-A02, YU537-H11 and YU536-D04 to other coronaviruses SARS-CoV-1, MERS-CoV, HCov-HKU1, HCoV-229E and HCoV-NL63 Spike proteins was tested by titration ELISA. The antibody bound specifically SARS-CoV-2 and did not show any cross reactions to other human-pathogenic coronaviruses. The antibody CR3022 (Tian et al., 2020) was used as control, because of the known cross reactivity to SARS (data not shown).

Because first SARS-CoV-2 mutants in patients are being identified and mutations within the RBD have been characterized to arise under antibody selection pressure (Baum et al., 2020; Shi et al., 2020). S1 subunits harbouring single point mutations in the RBD were produced and binding of STE90-C11, YU505-A02, YU537-H11 and YU536-D04 to those mutants was tested.

A "worst-scenario" mutant containing all the 7 single point mutations, termed S1-7PM, was producible and therefore also tested. All constructs were still functionally binding recombinant ACE2.

The published antibodies anti-SARS-CoV-2 antibody CR3022 which is cross-reactive with SARS-CoV-2, the SARS-CoV-2 antibody CB6 (Shi et al., 2020) and the antibodies REGN10933 and REGN10987 (Hansen et al., 2020) were also titrated against the same panel of S1 mutants. The cumulative results of these experiments are summarized in Figure 30.

The antibodies STE90-C11, YU505-A02, YU537-H11 and YU536-D04 were still binding specifically to all of the investigated RBD mutations with only a slightly reduced binding specifically to the F486V mutation. Surprisingly, the antibodies still retain at least partially their binding activity to the "worst-case" S1-7PM mutant.

In contrast, CB6 showed reduced or strongly reduced binding specifically to four of the seven mutations. REGN10933 lost binding specifically to S1-7PM and showed reduced binding specifically to the F486V mutants. Binding to E484K, G485R seemed also to be at least reduced.

CR3022 showed also loss of binding specifically to the combined seven mutations and to the V367F mutation which is close to the antibody binding site and reduced binding specifically to the E484K and G485R mutation.

**Example 12: Binning study**

Epitope binning was performed in a premix setup. The antibodies were coupled in replicate (2X) on a HC30M chip using EDC/NHS chemistry:

- 5µg/mL in acetate buffer pH 4.5, final volume 250µL

- Amount of antibody needed: 1.25µg

- Immobilization time 10min

Mixture of antibodies (1µM) and antigen (200nM) was prepared in running buffer (HBST), the final volume was 300µL.

- Amount of antibodies needed: 4µg

- Amount of antigen: RBD 40µg, S1-S2 177µg

Epitope binning setup: association 5 min, regeneration Glycine 1.5 2x40s.

**Example 13: BLI-Measurement**

In order to test the affinity of the inventive antibodies to the "escape"-mutants mentioned before, a BLI (bio-layer interferometry)-measurement was done (cf. also Abdiche et al., PLOS ONE, 1 March 2014 | Volume 9 | Issue 3).

BLI is a label-free technology for measuring biomolecular interactions. It is an optical analytical technique that analyzes the interference pattern of white light reflected from two surfaces: a layer of immobilized protein on the biosensor tip, and an internal reference layer. Any change in the number of molecules bound to the biosensor tip causes a shift in the interference pattern that can be measured in real-time.

The binding between a ligand immobilized on the biosensor tip surface and an analyte in solution produces an increase in optical thickness at the biosensor tip, which results in a wavelength shift, $\Delta\lambda$, which is a direct measure of the change in thickness of the biological layer.

The affinity was measured by Bio-Layer Interferometry in three different assays using the Octet qKe (Fortebio/Sartorius GmbH, Göttingen, Germany).

In the first assay, anti-mouse Fc-Capture (AMC) sensors were activated for 10 min in PBS. After that, the sensors were equilibrated in assay buffer (PBS containing 1% BSA and 0.05% Tween 20) for 60 s before RBD-mFc (Sino Biologicals) was loaded onto the sensors at 10 µg/ml for 180 s. After a stable baseline measurement was established (60 s), antigen-loaded sensors were transferred to an 8-point antibody dilution series (500, 158, 50, 15, 5, 1.5, 0.5 and 0 nM). Association of the Fab antibody to the antigen was measured for 300 s. After that, the sensors were transferred into assay buffer were the dissociation was measured for 900 s. Significant binding of the antibody to an unloaded sensor was not detected. For data analysis, the reference measurement (0 nM) was subtracted from the other measurements and data traces ranging from 158 to 1.5 nM were used for modelling of the kinetic data using a 1:1 binding model.

In the second assay, anti-human Fab (FAB2G) sensors were activated for 10 min in PBS. After that, the sensors were equilibrated in assay buffer (PBS containing 1% BSA and 0.05% Tween 20) for 60 s before the IgG antibody was loaded onto the sensors at 2.5 µg/ml for 180 s. After a stable baseline measurement was established (60 s), antibody-loaded sensors were transferred to an 8-point S1-HIS antigen dilution series (500, 158, 50, 15, 5, 1.5, 0.5 and 0 nM). Association of the S1 antigen to the antibody was measured for 300 s. After that, the sensors were transferred into assay buffer were the dissociation was measured for 900 s. Significant binding of the antigen to an unloaded sensor was not detected. For data analysis, the reference measurement (0 nM) was subtracted from the other measurements and data traces ranging from 50 to 5 nM were used for modelling of the kinetic data using a 1:1 binding model in the Data Analysis HT 11.0 software tool.

And in the third assay, Protein A sensors were activated for 10 min in PBS. Before use, the sensors were regenerated for 5 cycles in 10 mM Glycine buffer (pH2.0) followed by neutralization in PBS. Each step was performed for 5 s. After that, the regenerated sensors were equilibrated in assay buffer (PBS containing 1% BSA and 0.05% Tween 20) for 60 s before the IgG antibody was loaded onto the sensors at 2.5 µg/ml for 180 s. After a stable baseline measurement was established (60 s), antibody-loaded sensors were transferred to an 8-point S1-HIS antigen dilution series (500, 158, 50, 15, 5, 1.5, 0.5 and 0 nM). Association of the S1 antigen to the antibody was measured for 300 s. After that, the sensors were transferred into assay buffer were the dissociation was measured for 900 s. Significant binding of the antigen to an unloaded sensor was not detected. For data analysis, the reference measurement (0 nM) was subtracted from the other measurements and data traces ranging from 50 to 0.5 nM were used for modelling of the kinetic data using a 1:1 binding model.

**Example 14: Affinity Study**

STE90-C11, YU537-H11 and YU536-D04 affinities to different targets were measured by Biolayer Interferometrie (BLI) (see example 13).

The binding-affinity of Fab antibodies to "wild-type" RBD-mFc (i.e. RBD without point mutations) immobilized on an anti-Fmc capture surface was as follows:

| Antibody | $K_D$ [nM] | $R^2$ |
|---|---|---|
| STE90-C11 | 8.14 | 0.9917 |
| YU537-H11 | 61.1 | 0.9703 |
| YU536-D04 | 17.5 | 0.9953 |

The binding-affinity to S1-His by immobilized Fab antibodies on a FAB2G- capture surface was as follows:

| Antibody | $K_D$ [nM] | $R^2$ |
|---|---|---|
| STE90-C11 | 1.56 | 0.9969 |
| YU537-H11 | 11.7 | 0.9963 |
| YU536-D04 | 5.49 | 0.9917 |

The binding-affinity to S1-His by immobilized Fab antibodies on a Protein A-surface was as follows:

| Antibody | $K_D$ [nM] | $R^2$ |
|---|---|---|
| STE90-C11 | 6.53 | 0.9891 |
| YU537-H11 | 32.5 | 0.9798 |
| YU536-D04 | 23 | 0.9964 |

**Claims**

1. A humanized or a human therapeutic monoclonal antibody or antigen-binding fragment, or a substantially similar variant thereof that binds specifically to SARS-CoV-2 glycoprotein S with the amino acid sequence of SEQ ID NO: 02 and neutralizes the infectious activity of SARS-CoV-2.

2. The therapeutic antibody or antigen-binding fragment or a substantially similar variant thereof according to claim 1, wherein the neutralization of the infectious activity of SARS-CoV-2 results in confluence score of at least 95% as measured with an infectious SARS-CoV-2 VERO E6 neutralizing assay.

3. The therapeutic antibody or antigen-binding fragment or a substantially similar variant thereof according to any of claims 1 or 2, wherein the antibody or antigen-binding fragment binds specifically to an epitope within the receptor binding domain (RBD) of the S1 domain of SARS-CoV-2 glycoprotein (SEQ ID NO: 06) and binds specifically to at least one of the escape-mutations of the S1 domain of SARS-CoV-2 glycoprotein selected from the list consisting of S1-V367F, S1-N439K, S1-G476S, S1-V483A, S1-E484K, S1-G485R, S1-F486V, S1-7PM-mutant (SEQ ID NO: 1730), and any combination thereof.

4. The therapeutic antibody or antigen-binding fragment or a substantially similar variant thereof according to claim 3, wherein said antibody or antigen-binding fragment thereof binds specifically both to an epitope comprising the amino acids 400-FVIR-GDEVRQIAPQTGKIADDYN-422 within the RBD (SEQ ID NO: 06) as well as to the S1-7PM-mutant (SEQ ID NO: 1730).

5. The therapeutic antibody or antigen-binding fragment or a substantially similar variant thereof according to any of claims 1 or 2, wherein said antibody or antigen-binding fragment thereof competes for binding to an epitope within the RBD (SEQ ID NO: 06) with an antibody comprising a heavy chain variable region of the amino acid sequence in SEQ ID NO: 1305; and a light chain variable region of the amino acid sequence in SEQ ID NO: 1309 or with an antibody comprising a heavy chain variable region of the amino acid sequence in SEQ ID NO: 926 and a light chain variable region of the amino acid sequence in SEQ ID NO: 928; and wherein the contamination ratio is less than 0.2.

6. The therapeutic antibody or antigen-binding fragment or a substantially similar variant thereof according to any of claims 1 or 2, wherein said antibody or antigen-binding fragment thereof competes for binding to the epitope 165-AKRVNCYFPLQSYGFQ-180 within the S1-7PM-mutant (SEQ ID NO: 1730) with an antibody comprising a heavy chain variable region of the amino acid sequence in SEQ ID NO: 1305; and a light chain variable region of the amino acid sequence in SEQ ID NO: 1309 or with an antibody comprising a heavy chain variable region of the amino acid sequence in SEQ ID NO: 926 and a light chain variable region of the amino acid sequence in SEQ ID NO: 928.

7. The therapeutic antibody or antigen-binding fragment or a substantially similar variant thereof according to any of the previous claims, comprising

   a heavy chain CDR1 (H-CDR1) domain with an amino acid sequence selected from the group consisting of SEQ ID NO: 1306, 910, 350, and 390;
   a heavy chain CDR2 (H-CDR2) domain with an amino acid sequence selected from the group consisting of SEQ ID NO: 1307, 912, 352, and 392;
   a heavy chain CDR3 (H-CDR3) domain with an amino acid sequence selected from the group consisting of SEQ ID NO: 1308, 914, 354, and 394;
   and
   a light chain CDR1 (L-CDR1) domain with an amino acid sequence selected from the group consisting of SEQ ID NO: 1310, 916, 356, and 396;
   a light chain CDR2 (L-CDR2) domain with an amino acid sequence selected from the group consisting of SEQ ID NO: 1311, 918, 358, and 398;
   a light chain CDR3 (L-CDR3) domain with an amino acid sequence selected from the group consisting of SEQ ID NO: 1312, 920, 360, and 400.

8. The therapeutic antibody or antigen-binding fragment or a substantially similar variant thereof according to any one of the previous claims, selected from the group of antibodies comprising a heavy chain variable region of the amino acid sequence in SEQ ID NO: 1305 and a light chain variable region of the amino acid sequence in SEQ ID NO: 1309; a heavy chain variable region of the amino acid sequence in SEQ ID NO: 926 and a light chain variable region of the amino acid sequence in SEQ ID NO: 928; a heavy chain variable region of the amino acid sequence in SEQ ID NO: 366 and a light chain variable region of the amino acid sequence in SEQ ID NO: 368; and/or a heavy chain variable region of the amino acid sequence in SEQ ID NO: 406 and a light chain variable region of the amino acid sequence in SEQ ID NO: 408; and/or any combinations thereof.

9. The therapeutic antibody or antigen-binding fragment or a substantially similar variant thereof according to any one of the previous claims, comprising a heavy chain variable region of the amino acid sequence in SEQ ID NO: 1305 and a light chain variable region of the amino acid sequence in SEQ ID NO: 1309.

10. The therapeutic antibody or antigen-binding fragment or a substantially similar variant thereof according to any of the previous claims, **characterized by** one or more of the following:

- capable of binding specifically to the RBD of SARS-CoV-2 Glycoprotein S with an affinity ($K_D$) of 6.5 x $10^{-8}$ M or less, preferably 10 x $10^{-9}$ M or less,
- capable of reducing or preventing an antibody-dependent enhancement (ADE) reaction in a patient,
- capable of preventing, reducing or healing a viral infection with SARS-CoV-2,
- capable of reducing or preventing the cytokine storm syndrome (CSS) in a patient,
- capable of reducing the load of viral particles in the sputum of a patient by at least 50% for at least 48 hours after administration of the therapeutic antibody or antigen-binding fragment thereof,
- having a specific binding specifically to the S1-7PM-mutant (SEQ ID. 1730) with a $K_D$ of 10 x $10^{-9}$ M or less,
- having an $IC_{50}$ of less than 0.036 $\mu$g/ml, preferably of 0.026 $\mu$g/ml or less.

11. An isolated nucleic acid molecule encoding the therapeutic antibody or antigen-binding fragment or a substantially similar variant thereof according to any one of the previous claims.

12. An expression vector comprising the nucleic acid molecule according to claim 11.

13. A method of producing the therapeutic antibody or antigen-binding fragment or a substantially similar variant thereof according to any one of the previous claims comprising the steps of

introducing the expression vector according to claim 12 into an isolated host cell,
growing the cell under conditions permitting production of the antibody or antigen-binding fragment thereof,
recovering the antibody or fragment so produced.

14. A pharmaceutical composition comprising the therapeutic antibody or antigen-binding fragment or a substantially similar variant thereof according to any one of claims 1 to 10, and a pharmaceutically acceptable carrier.

15. A therapeutic antibody or antigen-binding fragment according to any one of claims 1 to 10 for the use in the prevention and/or treatment of a SARS-CoV-2-related disease or condition in a patient.

**Figures**

**Figure 1a:**

**Figure 1b:**

**Figure 2:**

Figure 3:

| Antibody | Germline VH | Germline VL | CDR of VH | CDR of VL |
|---|---|---|---|---|
| YU535-C09 | IGHV3-15*04 | IGKV1-39*01 | DAWMT_RINSNTDHGTTDYAAPVKG_SYYDYWSAFLY | RASQSISSYLN_AASSLQS_QQAYNIPYT |
| YU537-B05 | IGHV1-2*02 | IGLV2-14*01 | GYYMH_WINPISGGTNYAQKFQG_VPYYYDSSGLWFDP | TGTSSDVGGYNYVS_EVSNRPS_SSYTSSSTWV |
| YU534-H01 | IGHV1-2*02 | IGLV2-14*01 | GYYMH_WINPNSGGTNYAQKFQG_APMYYYDSSGYFDY | TGTSSDVGGYNYVS_EVSNRPS_SSYTSSSTYV |
| YU534-F11 | IGHV1-2*04 | IGLV2-14*01 | GYYMH_WINPNSGGTNYAQKFQG_GPQLNMITFGGVGYAFDI | TGTSSDVGGYNYVS_DVSNRPS_SSYTSSSTVV |
| YU536-C01 | IGHV3-23*01 | IGKV1-5*01 | NYAMS_AFSGSGGSTYYADSVKG_GARGGYDYGDY | RASQSISSWLA_DASSLES_QQYNSYSPT |
| YU535-A06 | IGHV3-23*04 | IGKV1-5*01 | SYAMS_AIGGSGGSTYYADSVKG_GPRSSGDYFDY | RASQSISSWLA_DASSLES_QQYNSYWGT |
| YU535-D12 | IGHV3-23*04 | IGKV1-5*01 | RYAMS_AISGSGGSTYYADSVKG_GPRSGWDYFDY | RASQSISSWLA_DASSLES_QQYNSYPMYT |
| YU534-C09 | IGHV1-69*09 | IGLV1-36*01 | NYAVS_RIIPNLGLTKYAQNFQG_GLGAYPDY | SGSSSNIGNNAVN_YDDLLPS_AAWDDSLSGPV |
| YU534-C12 | IGHV1-46*01 | IGLV2-14*01 | NYYMH_IINPSGGTSYAQKFQG_DLYAIFGVVID | AGTSSDVGGYNYVS_DVTNRPS_SSYTSSSTWV |
| YU535-A02 | IGHV1-3*01 | IGKV1-5*01 | SNAMH_WINAGNGNTKYSQKFQG_GRDGYNVDYFEY | RASQSISSWLA_DASSLES_QQYNSYPWT |
| YU535-E07 | IGHV1-3*01 | IGKV1-5*01 | SNVMH_WINAGNGNTKYSQKFQG_GRDGYNVDYFDY | RASQSISSWLA_DASSLES_QQYNSYPWT |
| YU536-E12 | IGHV3-53*01 | IGLV1-40*01 | SNYMS_LIYSGGSTYYADSVKG_ALEVNTAGDYFDY | TGSSSNIGAGYDVH_GNSNRPS_QSYDSSLSGWV |
| YU537-E03 | IGHV3-53*01 | IGLV1-40*01 | SNYMS_LIYSGGSTYYADSVKG_ALEVNTAGDYFDY | TGSSSNIGAGYDVH_GNSNRPS_QSYDSSLSGYV |
| YU534-D06 | IGHV3-66*02 | IGLV2-14*01 | SNYMS_VIYSGGSTFYADSVKG_DGYYDILTGYYSENSFDI | TGTSSDVGGYNYVS_EVSNRPS_SSYTSSSTYVV |
| YU536-E07 | IGHV3-66*02 | IGKV1-9*01 | SNYMS_VIYSGGSTFYADSVKG_DLGPYGMDV | RASQGISSYLA_AASTLQS_QQLNSYPPYT |
| YU534-B03 | IGHV3-66*02 | IGLV1-40*01 | SNYMS_VIYSGGSTFYADSVKG_DLGPYGMDV | TGSSSNIGAGYDVH_GSSNRPS_AAWDDSLSVDV |
| YU534-D10 | IGHV3-66*02 | IGLV1-40*01 | SNYMS_VIYSGGSTYYADSVKG_DLVNWGMDY | TGSSSNIGAGYDVH_GNSNRPS_QSYDSSLSGYV |
| YU536-D04 | IGHV3-66*02 | IGKV1-9*01 | SNYMS_VIYSGGSTYYADSVKG_DLVVMGLDV | RASQGISSYLA_AASTLQS_QQLNSYPIT |
| YU537-B08 | IGHV3-53*01 | IGLV2-14*01 | SNYMS_VIYSGGSTYYADSVKG_GEIQPYYYYGMDV | TGTSGDVGGYNYVS_DVSNRPS_SSYTSSTPVV |
| YU537-H11 | IGHV3-53*01 | IGLV1-51*01 | SNYMS_VIYSGGSTYYADSVKG_GESGSPYGMDV | SGSSSNIGNNYVS_DTNKRPS_ATWDSSLSVGV |
| YU534-A11 | IGHV3-66*02 | IGLV1-36*01 | SNYMS_VIYSGGSTYYADSVKG_GKSITMVRGVPTGVPPYMDV | SGSSSNIGNNAVN_YDDLLPS_AAWDDSLNGVV |
| YU534-G06 | IGHV1-69*14 | IGLV2-14*01 | SYAIT_GIIPIFGTPNYAQKFQD_DKGFGEGKGYHYYGLDV | TGTSSDVGSYNFVS_EVNNRPS_SSYTSSSVV |
| YU534-H02 | IGHV1-69*14 | IGLV2-14*01 | SYALT_GIIPIFGTPNYAQKFQD_DKGFGEGKGYHFYGLDV | TGTSSDVGGYNFVS_EVSNRPS_QSYTSSGSLI |
| YU534-F06 | IGHV1-69*14 | IGLV2-23*02 | SYAIT_GIIPIFGTPNYAQKFQD_DKGFGEGKGYHYYGLDV | TGTSSDVGSYNLVS_EVSKRPS_SSYAGSNNVV |
| YU534-C05 | IGHV1-2*02 | IGLV1-40*01 | SYAIS_WINPNSGGTNYAQKFQG_GPLPYNWNYEGHWFDP | SGSSSNIGNNYVS_GNSNRPS_QSYDSSLSGSV |
| YU534-D09 | IGHV1-69*14 | IGLV2-14*01 | SYAIT_GIIPIFGTPNYAQKFQD_DKGLGEGKGYHYYGLDV | TGTSSDVGGYNYVS_EVSNRPS_TSYTSNSNLA |
| YU535-C06 | IGHV1-3*01 | IGKV1-5*01 | SYAMH_WINAGNGNTKYSQKFQG_SFDSGSYYNVDAFDI | RASQSISSWLA_DASSLES_QQYNSYSWT |
| YU535-A09 | IGHV3-23*01 | IGKV1-5*01 | SYAMS_AISGSGGSTYYADSVKG_GPRGSADYVDY | RASHSISSWLA_DASSLES_QQYNTYSGT |
| YU535-G09 | IGHV3-23*01 | IGKV1-5*01 | SYAMS_AISGSGGSTYYADSVKG_GPRGSADYVDY | RASQSISSWLA_DASSLES_QQYNSYWT |
| YU536-H01 | IGHV3-23*01 | IGKV1-5*01 | SYAMS_AISGSGGSTYYADSVKG_GVRYNSDYFDY | RASQSISSWLA_DASSLES_QQYNSVLGVT |
| YU536-H05 | IGHV3-23*01 | IGKV1-5*01 | SYAMS_AISGSGGSTYYADSVKG_GVRYSSDYFEY | RASQSISSWLA_DASSLES_QQYETYSQT |
| YU535-G02 | IGHV3-23*04 | IGKV1-5*01 | SYAMS_AISGSGGSTYYADSVKG_GPRSGTDYFDY | RASESIRSWLA_DASNLES_QQYYSYPLT |
| YU535-G11 | IGHV3-23*01 | IGKV1D-13*01 | SYAMS_AISGSGGSTYYADSVKG_GPRSGYDYGDY | RASQGISSWLA_DASSLES_QQFNSYPLT |
| YU534-B11 | IGHV3-23*04 | IGLV1-40*01 | SYAMS_AISGSGGSTYYADSVKG_SVGYYDSSGYYHFGDYFDY | TGSSSNIGAGYDVH_GNSNRPS_QSYDSSLSGSV |
| YU534-C10 | IGHV3-23*04 | IGLV1-40*01 | SYAMS_AISGSGGSTYYADSVKG_SVGYYDSSGYYHFGDYFDY | TGSSSNIGAGYDVH_GNSNRPS_QSYDSSLSGVV |
| YU535-D10 | IGHV5-51*01 | IGKV1-39*01 | SYWIG_IIYPGDSDTRYSPSFQG_QNYYDSSGYYYFDY | RASQSISSYLN_AASSLQS_QQSYSTHSWT |
| YU535-B02 | IGHV5-51*01 | IGKV1S2*01 | SYWIG_IIYPGDSDTRYSPSFQG_SRSSDYFDY | RASQGISSWLA_DASNLET_QQYDNLPVT |
| YU534-D07 | IGHV5-51*01 | IGLV1-44*01 | SYWIG_IIYPGDSDTRYSPSFQG_YNSGWLDY | SGSSSNIGSNPVN_SNNQRPS_AAWDDSLNGNVL |
| YU537-H08 | IGHV1-46*01 | IGLV3-21*02 | SYYMH_IINPSGGSTSYAQKFQG_ASRDYYDSSGYLRRMGGDY | GGNNIGSKSVH_DDSDRPS_QVWDSSTAV |
| YU534-E01 | IGHV3-23*04 | IGLV1-51*02 | TYAMG_TISGTGGITYYADSVKG_VGDNYYDRSGNYHMDYFDY | SGSSSNIGNNYVS_EDNERPS_GTWDSSLSVML |
| YU534-C02 | IGHV3-23*01 | IGLV1-51*02 | TYAMS_TISGTGGITYYADSVKG_VGDNYYDRSGNYHMDYFDY | TGSSSNIGAAYDVH_ENNKRPS_GTWDGSLSV |
| YU534-C06 | IGHV3-23*01 | IGLV1-40*01 | TYAMS_TISGTGGITYYADSVKG_VGDNYYDRSGNYHMDYFDY | TGSTSNIGAGYDVQ_EDNNRPS_QSSVSSLSAVV |
| YU536-C12 | IGHV3-23*01 | IGLV2-14*01 | TYAMS_TISGTGGITYYADSVKG_VGDNYYDRSGNYHMDYFDY | TGTSSDLGAYDYVS_DVNNRPS_NSYTSSSTWV |
| YU534-G10 | IGHV3-23*04 | IGLV2-14*02 | TYAMS_TISGTGGITYYADSVKG_VGDNYYDRSGNYHMDYFDY | TGTSSDVGSYNLVS_EGSQRPS_SSYTSSSTVV |

**Figure 4a:**

YU505-A01: VH

QVQLVQSGAEVKKPGASVKVSCKASGYTFTSYYMHWVRQAPGQGLE
WMGIINPSGGSTSYAQKFQGRVTMTRDTSTSTVYMELSSLRSEDTAVYY
CARDRHYYDSSGSSQATFLFDYWGQGTLVTVSS

YU505-A01: VL
QSVLTQPPSVSGAPGQRVTISCTGSSSNIGAGYDVHWYQQLPGTAPKLLI
YGNSNRPSGVPDRFSGSKSGTSASLAITGLQAEDEADYYCQSYDSSLNVF
GTGTKLTVL

Figure 4b:


YU505-A02: VH

QVQLVESGAEVKKPGSSVKVSCKASGGTFSSYAISWVRQAPGQGLEWM
GGIIPIFGTANYAQKFQGRVTITADESTSTAYMELSSLRSEDTAVYYCARQI
WAYDSSGYPVPAEYFQHWGQGTLVTVSS


YU505-A02: VL

QSVLTQPPSVSGAPGQRVTISCTGSSSNIGAGYDVHWYQQLPGTAPKLLI
YGNSNRPSGVPDRFSGSKSGTSASLAITGLQAEDEADYYCQSYDSSLSGV
VFGGGTKLTVL

**Figure 4c:**

YU505-E01: VH

QVQLVQSGAEVKKPGSSVKVSCKASGGTFSSYSIVWVRQAPGQGLEW
MGGIIPIFGTTNYAQKFQGRVTITADESTSTAFMELSNLKSEDTAVYFCAR
SLGATRDYWGQGTLVTVSS

YU505-E01: VL

SYVLTQPPSVSEAPRQRVTISCSGSSSNIGNNAVNWYQQLPGKAPKLLIY
YDDLLPSGVSDRFSGSKSGTSASLAISGLQSEDEADYYCAAWDDSLNGPV
FGGGTKVTVL

**Figure 4d:**

YU505-F05: VH
EVQLLESGGGVVQPGRSLRLSCAASGFTFSSYGMHWVRQAPGKGLEW
VAVMSYDGSKKYYADSVKGRFTISRDNSKNTLYLQMNSLRAEDTAVYYC
VTQGMDVWGQGTLVTVSS

YU505-F05: VL
QSVLTQPPSVSVAPGKTARITCGGNNIGSKSVHWYQQKPGQAPVLVIYYI
SDRPSGIPERFSGSNSGNTATLTISRVEAGDEAAYYCHVWDSSSDHVVFG
GGTKLTVL

**Figure 5a:**

YU505-A01: DNA-sequence of scFv

CAGGTGCAGCTGGTGCAGTCTGGGGCTGAGGTGAAGAAGCCTGGGGCCT
CAGTGAAGGTTTCCTGCAAGGCATCTGGATACACCTTCACCAGCTACTATAT
GCACTGGGTGCGACAGGCCCCTGGACAAGGGCTTGAGTGGATGGGAATA
ATCAACCCTAGTGGTGGTAGCACAAGCTACGCACAGAAGTTCCAGGGCAG
AGTCACCATGACCAGGGACACGTCCACGAGCACAGTCTACATGGAGCTGA
GCAGCCTGAGATCTGAGGACACGGCCGTGTATTACTGTGCTAGAGACCGCC
ATTACTATGATAGTAGTGGTTCTTCCCAGGCCACCTTTCTCTTTGACTACTGG
GGCCAGGGAACCCTGGTCACCGTGTCCTCACAGTCTGTGTTGACGCAGCC
GCCCTCAGTGTCTGGGGCCCCAGGGCAGAGGGTCACCATCTCCTGCACTG
GGAGCAGCTCCAACATCGGGGCAGGTTATGATGTACACTGGTACCAGCAG
CTTCCAGGAACAGCCCCCAAACTCCTCATCTATGGTAACAGCAATCGGCCCT
CAGGGGTCCCTGACCGATTCTCTGGCTCCAAGTCTGGCACCTCAGCCTCCC
TGGCCATCACTGGGCTCCAGGCTGAGGATGAGGCTGATTATTACTGCCAGT
CCTATGACAGCAGCCTGAATGTCTTCGGAACTGGGACCAAGCTGACCGTCC
TA

**Figure 5b:**

YU505-A02: DNA-sequence of scFv

CAGGTGCAGCTGGTGGAGTCTGGGGCTGAGGTGAAGAAGCCTGGGTCCT
CGGTGAAGGTCTCCTGCAAGGCTTCTGGAGGCACCTTCAGCAGCTATGCTA
TCAGCTGGGTGCGACAGGCCCCTGGACAAGGGCTTGAGTGGATGGGAGG
GATCATCCCTATCTTTGGTACAGCAAACTACGCACAGAAGTTCCAGGGCAG
AGTCACGATTACCGCGGACGAATCCACGAGCACAGCCTACATGGAGCTGA
GCAGCCTGAGATCTGAGGACACGGCCGTGTATTACTGTGCACGTCAAATCT
GGGCTTATGATAGTAGTGGTTACCCCGTCCCGGCCGAATACTTCCAGCACTG
GGGCCAGGGCACCCTGGTCACCGTGTCCTCACAGTCTGTGTTGACGCAGC
CGCCCTCGGTGTCTGGGGCCCCAGGGCAGAGGGTCACCATCTCCTGCACT
GGGAGCAGCTCCAACATCGGGGCAGGTTATGATGTACACTGGTACCAGCA
GCTTCCAGGAACAGCCCCCAAACTCCTCATCTATGGTAACAGCAATCGGCC
CTCAGGGGTCCCTGACCGATTCTCTGGCTCCAAGTCTGGCACCTCAGCCTC
CCTGGCCATCACTGGGCTCCAGGCTGAGGATGAGGCTGATTATTACTGCCA
GTCCTATGACAGCAGCCTGAGTGGTGTGGTATTCGGCGGAGGGACCAAGC
TGACCGTCCTA

**Figure 5c:**

YU505-E01: DNA-sequence of scFv

CAGGTTCAGCTGGTGCAGTCTGGAGCTGAGGTGAAGAAGCCTGGGTCCTC
GGTGAAGGTCTCCTGCAAGGCTTCTGGAGGCACCTTCAGCAGTTATAGTAT
TGTCTGGGTGCGACAGGCCCCTGGACAAGGACTTGAGTGGATGGGAGGG
ATCATCCCTATTTTTGGTACTACAAACTATGCACAGAAGTTCCAGGGCAGAG
TCACGATTACCGCGGACGAATCCACAAGCACAGCCTTCATGGAGCTGAGCA
ACCTAAAATCTGAGGACACGGCCGTCTATTTCTGTGCGAGATCTCTGGGAG
CCACTCGTGACTACTGGGGCCAGGGAACCCTGGTCACCGTGTCCTCATCCT
ATGTGCTGACTCAGCCACCCTCGGTGTCTGAAGCCCCCAGGCAGAGGGTC
ACCATCTCCTGTTCTGGAAGCAGCTCCAACATCGGAAATAATGCTGTAAACT
GGTACCAGCAGCTCCCAGGAAAGGCTCCCAAACTCCTCATCTATTATGATGA
TCTGCTGCCCTCAGGGGTCTCTGACCGATTCTCTGGCTCCAAGTCTGGCAC
CTCAGCCTCCCTGGCCATCAGTGGGCTCCAGTCTGAGGATGAGGCTGATTA
TTACTGTGCAGCATGGGATGACAGCCTGAATGGTCCGGTGTTCGGCGGAG
GGACCAAGGTCACCGTCCTA

**Figure 5d:**

YU505-F05: DNA-sequence of scFv

GAGGTGCAGCTGTTGGAGTCTGGGGGAGGCGTGGTCCAGCCTGGGAGGT
CCCTGAGACTCTCCTGTGCAGCCTCTGGATTCACCTTCAGTAGCTATGGCAT
GCACTGGGTCCGCCAGGCTCCAGGCAAGGGGCTGGAGTGGGTGGCAGTT
ATGTCATATGATGGAAGTAAAAAATACTATGCAGACTCCGTGAAGGGCCGAT
TCACCATCTCCAGAGACAATTCCAAGAACACGCTGTATCTGCAAATGAACA
GCCTGAGAGCTGAGGACACGGCTGTGTATTACTGTGTCACTCAGGGTATGG
ACGTCTGGGGCCAAGGGACCCTGGTCACCGTGTCCTCACAGTCTGTGCTG
ACTCAGCCACCCTCAGTGTCAGTGGCCCCAGGAAAGACGGCCAGGATTAC
CTGTGGGGGAAACAACATTGGAAGTAAAAGTGTGCACTGGTACCAGCAGA
AGCCAGGCCAGGCCCCTGTGTTGGTCATCTATTATATTAGCGACCGGCCCTC
AGGGATCCCTGAGCGATTCTCTGGCTCCAACTCTGGGAACACGGCCACCCT
GACCATCAGCAGGGTCGAGGCCGGGGATGAGGCCGCCTATTACTGTCACG
TGTGGGATAGTAGTAGTGATCATGTGGTATTCGGCGGAGGGACCAAGCTGA
CCGTCCTA

**Figure 6**

Mean S/N stickiness (test antibodies vs Avelumab)

**Figure 7a**

**Figure 7b:**

**Figure 7c:**

Figure 7d:

Figure 8:

Figure 9:

SEC-HPLC analysis

**Figure 10:**

Specificity assay

**Figure 11:**

**Figure 12:**

ACE2:RBD blocking assay at 5 µg/ml IgG

**Figure 13:**

**Figure 14:**

Heat stability assay

**Figure 15:**

Multimerization analysis with/without stress conditions

Figure 16:

**Figure 17:**

| IC50 (µg/ml) | $R^2$ | Ab-Name |
|---|---|---|
| 0.02539 | 0.857 | YU537-H11 |
| 0.03243 | 0.606 | YU537-E03 |
| 0.03599 | 0.885 | YU534-C09 |
| 0.03672 | 0.855 | YU536-D04 |
| 0.04848 | 0.794 | YU535-A02 |
| 0.1195 | 0.530 | YU505-F05 |
| 0.1296 | 1.075 | YU534-D09 |
| 0.1304 | 0.658 | YU535-C06 |
| 0.5112 | 1.122 | YU534-C12 |
| 2.052 | 0.806 | YU505-E01 |

**Figure 18**

**Figure 19A**

Figure 19B

EP 3 919 126 A1

**Figure 19C**

**Figure 20**

**Figure 21:**

**Figure 22:**

Figure 23:

Figure 24:

**Figure 25:**

Figure 26:

| Antibody name | VH | VL | EC50 ELISA [nM] | | | Flow cytometry spike binding inhibition assay | | | |
|---|---|---|---|---|---|---|---|---|---|
| | | | RBD | S1 | S1-S2 | IC 50 [nM] with 50 nM spike | Molar ratio antibody arm: spike | IC 50 [nM] with 10 nM RBD | Molar ratio antibody arm: RBD |
| STE70-1E12 | IGHV1-2 | IGLV6-57 | 1.1 | 1.5 | 13.3 | 180 | 7.2 | 3.2 | 0.64 |
| STE72-1B6 | IGHV3-23 | IGKV1-12 | 1.6 | 2.2 | 4.4 | 240 | 9.6 | 4.8 | 0.96 |
| STE72-1G5 | IGHV1-69 | IGKV3-20 | 8.9 | 10.7 | 16.3 | n.d. | n.d. | n.d. | n.d. |
| STE72-4C10 | IGHV3-30 | IGKV1D-39 | 1.7 | 3.2 | 7.4 | 117 | 4.8 | 3.5 | 0.7 |
| STE72-4E12 | IGHV1-46 | IGKV3-15 | 4.7 | 10.4 | 11.6 | n.d. | n.d. | 3.0 | 0.6 |
| STE72-8A2 | IGHV1-18 | IGKV1D-33 | 1.5 | 2.3 | 2.6 | 35 | 1.4 | 1.5 | 0.3 |
| STE72-8A6 | IGHV1-18 | IGKV1-5 | 1.5 | 2.9 | 3.7 | 102 | 4.0 | 2.8 | 0.56 |
| STE72-8E1 | IGHV4-61 | IGKV1-5 | 1.4 | 2.4 | 2 | 20 | 0.8 | 5.6 | 1.1 |
| STE72-2G4 | IGHV1-2 | IGLV2-8 | 0.7 | 0.9 | 1 | 37 | 1.4 | 3.7 | 0.74 |

| STE73-2B2 | IGHV1-2 | IGLV6-57 | 0.5 | 0.8 | 1 | 63 | 2.6 | 1.7 | 0.4 |
|---|---|---|---|---|---|---|---|---|---|
| STE73-2C2 | IGHV3-66 | IGLV6-57 | 9.6 | 17.9 | 24.5 | 59 | 2.4 | 3.0 | 0.6 |
| STE73-2E9 | IGHV1-18 | IGLV1-36 | 0.7 | 0.6 | 0.5 | 20 | 0.8 | 3.4 | 0.68 |
| STE73-2G8 | IGHV3-66 | IGLV3-19 | 0.7 | 1 | 1.4 | 23 | 1.0 | 2.8 | 0.56 |
| STE73-6B10 | IGHV1-2 | IGLV2-11 | 17.3 | 15.3 | 63.4 | 612 | 24 | 73 | 14.6 |
| STE73-6C1 | IGHV3-30 | IGLV1-40 | 2 | 2.8 | 5.5 | 97 | 3.8 | 4.1 | 0.81 |
| STE73-6C8 | IGHV1-69 | IGLV6-57 | 3.3 | 6 | 17 | 332 | 13.2 | 5.4 | 1.08 |
| STE73-9G3 | IGHV3-23 | IGLV1-40 | 1.4 | 1.6 | 2.8 | 40 | 1.6 | 3.4 | 0.6 |

**Figure 27:**

**Figure 28:**

| IC$_{50}$ (µg/ml) | R$^2$ | Ab |
|---|---|---|
| 0,03663 | 0,9001 | STE94-H2 (scFv-Fc) |
| 0,07019 | 0,5856 | STE73-2E9 (IgG) |
| 0,09516 | 0,71 | STE90-C11 (scFv-Fc) |
| 0,8039 | 0,8663 | STE91-B1-C4 (scFv-Fc) |

**Figure 29:**

Figure 30a:

**Figure 30b:**

Figure 31:

**S1 mutants binding to ACE**

Legend: S1-HIS, V367F, N439K, G476S, V483A, E484K, G485R, F486V

**S1-7PM binding to ACE**

Legend: S1-HIS, S1-7PM

Figure 32:

**Figure 33:**

## Com 1 (BIN 1):

- YU505-E01
- REGN10933
- YU536-D04
- STE90-C11
- YU536-D04
- YU537-H11
- YU505-A02
- YU537-H08

## COM2 (BIN 2):

- YU534-D09
- YU505-F05

## COM3 (BIN 3):

- YU535-A02
- REGN10987

**Figure 34**

| S1-7PM | KD (M) | KD Error | Full R^2 |
|---|---|---|---|
| STE90-C11 | 4.27E-08 | 1.28E-09 | 0.95 |
| REGN10933 | - | - | - |
| REGN10987 | 4.91E-08 | 1.33E-09 | 0.97 |
| YU536-D04 | 3.22E-08 | 2.72E-09 | 0.85 |
| YU537-H11 | 2.10E-07 | 7.69E-09 | 0.97 |
| YU505-A02 | 5.57E-09 | 5.38E-10 | 0.85 |
| YU505-F05 | 7.13E-08 | 2.15E-09 | 0.96 |
| YU535-A02 | 9.10E-08 | 3.63E-09 | 0.95 |

| S1-HIS V367F | KD (M) | KD Error | Full R^2 |
|---|---|---|---|
| STE90-C11 | 1.66E-08 | 3.15E-10 | 0.99 |
| REGN10933 | 3.65E-09 | 2.75E-11 | 1.00 |
| REGN10987 | 7.19E-09 | 9.82E-11 | 0.99 |
| YU536-D04 | 1.49E-08 | 1.80E-10 | 1.00 |
| YU537-H11 | 1.54E-08 | 3.65E-10 | 0.98 |
| YU505-A02 | 2.67E-09 | 1.84E-10 | 0.86 |
| YU505-F05 | 9.91E-09 | 1.12E-10 | 1.00 |
| YU535-A02 | 7.21E-09 | 1.54E-10 | 0.97 |

| S1-HIS N439K | KD (M) | KD Error | Full R^2 |
|---|---|---|---|
| STE90-C11 | 9.90E-09 | 2.40E-10 | 0.96 |
| REGN10933 | 3.92E-09 | 3.48E-11 | 1.00 |
| REGN10987 | 9.68E-08 | 2.76E-09 | 0.97 |
| YU536-D04 | 4.66E-08 | 5.18E-10 | 0.99 |
| YU537-H11 | 2.43E-08 | 7.67E-10 | 0.98 |
| YU505-A02 | 1.95E-07 | 1.45E-08 | 0.91 |
| YU505-F05 | 5.00E-09 | 9.88E-11 | 1.00 |
| YU535-A02 | 3.31E-08 | 5.10E-10 | 0.99 |

| S1-HIS G476S | KD (M) | KD Error | Full R^2 |
|---|---|---|---|
| STE90-C11 | 5.89E-09 | 1.96E-10 | 0.95 |
| REGN10933 | 3.85E-09 | 4.57E-10 | 1.00 |
| REGN10987 | 7.51E-09 | 1.12E-10 | 0.99 |
| YU536-D04 | 1.64E-08 | 2.50E-10 | 0.99 |
| YU537-H11 | 2.82E-08 | 3.57E-10 | 0.99 |
| YU505-A02 | 4.03E-07 | 2.51E-08 | 0.96 |
| YU505-F05 | 4.94E-09 | 9.96E-11 | 1.00 |
| YU535-A02 | 6.50E-09 | 1.76E-10 | 0.97 |

| S1-HIS V483A | KD (M) | KD Error | Full R^2 |
|---|---|---|---|
| STE90-C11 | 1.86E-08 | 3.44E-10 | 0.98 |
| REGN10933 | 7.26E-09 | 3.98E-11 | 1.00 |
| REGN10987 | 7.61E-09 | 1.13E-10 | 0.99 |
| YU536-D04 | 4.78E-08 | 3.82E-10 | 1.00 |
| YU537-H11 | 9.87E-08 | 1.61E-09 | 0.99 |
| YU505-A02 | 5.51E-07 | 4.33E-08 | 0.96 |
| YU505-F05 | 3.77E-08 | 4.40E-10 | 0.99 |
| YU535-A02 | 1.88E-08 | 3.28E-10 | 0.98 |

| S1-HIS E484K | KD (M) | KD Error | Full R^2 |
|---|---|---|---|
| STE90-C11 | 7.85E-09 | 1.55E-10 | 0.98 |
| REGN10933 | 9.26E-08 | 1.29E-09 | 1.00 |
| REGN10987 | 5.26E-09 | 7.38E-11 | 0.99 |
| YU536-D04 | 4.39E-08 | 5.25E-10 | 0.99 |
| YU537-H11 | 5.74E-08 | 8.20E-10 | 0.99 |
| YU505-A02 | 4.95E-07 | 1.96E-08 | 0.99 |
| YU505-F05 | 5.27E-09 | 8.77E-11 | 1.00 |
| YU535-A02 | 1.39E-07 | 4.40E-09 | 0.99 |

| S1-HIS G485R | KD (M) | KD Error | Full R^2 |
|---|---|---|---|
| STE90-C11 | 1.73E-08 | 4.26E-10 | 0.97 |
| REGN10933 | 3.07E-08 | 5.91E-10 | 0.99 |
| REGN10987 | 7.97E-09 | 9.80E-11 | 0.99 |
| YU536-D04 | 3.45E-08 | 5.68E-10 | 0.99 |
| YU537-H11 | 6.43E-08 | 1.53E-09 | 0.98 |
| YU505-A02 | 2.13E-07 | 1.75E-08 | 0.98 |
| YU505-F05 | 1.11E-08 | 8.94E-11 | 1.00 |
| YU535-A02 | 1.07E-08 | 1.69E-10 | 0.99 |

Europäisches
Patentamt

European
Patent Office

Office européen
des brevets

# EUROPEAN SEARCH REPORT

Application Number

EP 20 21 0803

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHI RUI ET AL: "A human neutralizing antibody targets the receptor-binding site of SARS-CoV-2", NATURE, MACMILLAN JOURNALS LTD., ETC, LONDON, vol. 584, no. 7819, 26 May 2020 (2020-05-26), pages 120-124, XP037211681, ISSN: 0028-0836, DOI: 10.1038/S41586-020-2381-Y [retrieved on 2020-05-26] | 1-3,5-8, 10-15 | INV. A61P31/14 C07K16/10 |
| A | * figures 1-4; table E4 * | 4,9 | |
| X | Wu Yan T AL: "A noncompeting pair of human neutralizing antibodies block COVID-19 virus binding to its receptor ACE2", Science (American Association for the Advancement of Science), 13 May 2020 (2020-05-13), pages 1274-1278, XP055758869, United States DOI: 10.1126/science.abc2241 Retrieved from the Internet: URL:https://science.sciencemag.org/content/sci/368/6496/1274.full.pdf * figures 1-3 * | 1-15 | |
| | -/-- | | |

TECHNICAL FIELDS SEARCHED (IPC)

A61P
C07K

The present search report has been drawn up for all claims

| Place of search | Date of completion of the search | Examiner |
|---|---|---|
| Munich | 28 April 2021 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 3

Europäisches
Patentamt

European
Patent Office

Office européen
des brevets

## EUROPEAN SEARCH REPORT

Application Number

EP 20 21 0803

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & Wu Yan ET AL: "Supplementary Material - A noncompeting pair of human neutralizing antibodies block COVID-19 virus binding to its receptor ACE2", Science, vol. 368, no. 6496, 13 May 2020 (2020-05-13), pages 1274-1278, XP055799109, US ISSN: 0036-8075, DOI: 10.1126/science.abc2241 Retrieved from the Internet: URL:https://science.sciencemag.org/highwir e/filestream/744452/field_highwire_adjunct _files/1/abc2241_Wu_SM.pdf> * tables S3,S4 *<br>----- | | |
| X | Seth J. Zost ET AL: "Potently neutralizing human antibodies that block SARS-CoV-2 receptor binding and protect animals", bioRxiv, 22 May 2020 (2020-05-22), XP055735422, DOI: 10.1101/2020.05.22.111005 Retrieved from the Internet: URL:https://www.biorxiv.org/content/10.110 1/2020.05.22.111005v1.full.pdf * figure E4a; table E1 *<br>----- | 1-15 | TECHNICAL FIELDS SEARCHED (IPC) |
| | -/-- | | |

The present search report has been drawn up for all claims

| Place of search | Date of completion of the search | Examiner |
|---|---|---|
| Munich | 28 April 2021 | Cilensek, Zoran |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 3

Europäisches
Patentamt

European
Patent Office

Office européen
des brevets

## EUROPEAN SEARCH REPORT

Application Number

EP 20 21 0803

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | JOHANNA HANSEN ET AL: "Studies in humanized mice and convalescent humans yield a SARS-CoV-2 antibody cocktail", SCIENCE, 15 June 2020 (2020-06-15), page eabd0827, XP055707770, US ISSN: 0036-8075, DOI: 10.1126/science.abd0827 * figures 2-4 * | 1-15 | |
| A,P | ALINA BAUM ET AL: "Antibody cocktail to SARS-CoV-2 spike protein prevents rapid mutational escape seen with individual antibodies", SCIENCE, 15 June 2020 (2020-06-15), page eabd0831, XP055707765, US ISSN: 0036-8075, DOI: 10.1126/science.abd0831 * the whole document * | 1-15 | |
| T | Bertoglio Federico ET AL: "A SARS-CoV-2 neutralizing antibody selected from COVID-19 patients by phage display is binding to the ACE2-RBD interface and is tolerant to known RBD mutations", bioRxiv, 3 December 2020 (2020-12-03), 3 December 2020 (2020-12-03), XP055799031, DOI: 10.1101/2020.12.03.409318 Retrieved from the Internet: URL:https://www.biorxiv.org/content/10.1101/2020.12.03.409318v1.full.pdf [retrieved on 2021-04-27] * the whole document * | 1-15 | |

TECHNICAL FIELDS SEARCHED (IPC)

The present search report has been drawn up for all claims

| Place of search | Date of completion of the search | Examiner |
|---|---|---|
| Munich | 28 April 2021 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KABAT et al.** Sequences of Proteins of Immunological Interest. Public Health Service, National Institutes of Health, 1991
- **BROCHET, X. et al.** *Nucl. Acids Res.,* 2008, vol. 36, W503-508
- **GIU-DICELLI, V. ; BROCHET, X. ; LEFRANC, M.-P.** *Cold Spring Harb Protoc.,* 01 June 2011, (6
- **GONNET et al.** *Science,* 1992, vol. 256, 1443-45
- **ALTSCHUL et al.** *J. Mol. Biol.,* 1990, vol. 215, 403-410
- *Nucleic Acids Res.,* 1997, vol. 25, 3389-402
- **ARMOUR, KL ; CLARK, MR ; HADLEY, AG ; WILLIAMSON, LM.** Recombinant human IgG molecules lacking Fcγ receptor I binding and monocyte triggering activities Eur. *J. Immunol.,* 1999, vol. 29, 2613-2624
- **KABAT, EA ; WU, TT ; PERRY, HM ; GOTTESMAN, KS ; FOELLER, C.** Sequences of proteins of immunological interest. US Department of Health and Human Services, NIH, 1991

- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies, A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 77-82
- **LLOYD.** *The Art, Science and Technology of Pharmaceutical Compounding,* 1999
- **YI et al.** *Cell. & Mol. Imm.,* 15 May 2020
- Remington's Pharmaceutical Sciences. Mack Publishing Company
- **ABDICHE et al.** *PLOS ONE,* 01 March 2014, vol. 9 (3